# EUROPEAN PATENT APPLICATION

(11) **EP 3 907 509 A2**
(43) Date of publication of application: **10.11.2021**
(21) Application number: 21171277.3
(22) Date of filing: 10.05.2017
(51) Int. Cl.: G01N 33/574

(54) **DIAGNOSIS OF EARLY STAGE PANCREATIC CANCER**

(30) Priority: 10.05.2016 GB 201608192
(62) Divisional of application: 17725897.7
(71) Applicant: Immunovia AB, 223 81 Lund (SE)
(72) Inventor: BORREBAECK,, Carl, 223 63 Lund (SE); GERDTSSON,, Anna Sandström, 374 40 Karlshamn (SE); WINGREN,, Christer, 247 32 Södra Sandby (SE)
(74) Representative: Potter Clarkson

(57) **Abstract**

The present invention provides a method for diagnosing or determining a pancreatic cancer-associated disease state comprising or consisting of the steps of: (a) providing a sample from an individual to be tested; and (b) determining a biomarker signature of the test sample by measuring the presence and/or amount in the test sample of one or more biomarker selected from the group defined in Table A; wherein the presence and/or amount in the test sample of the one or more biomarker selected from the group defined in Table A is indicative of the pancreatic cancer-associated disease in the individual; uses and methods of determining a pancreatic cancer-associated disease state, and methods of treating pancreatic cancer, together with arrays and kits for use in the same.

## Description

### Field of Invention

The present invention provides methods for determining a pancreatic cancer-associated disease state (early pancreatic cancer presence, pancreatic cancer stage and/or pancreatic cancer presence), as well as arrays and kits for use in such methods.

### Background

Pancreatic ductal adenocarcinoma (PDAC) is one of the deadliest cancers with a 5-year survival rate of 3-4%. A key driver behind this poor prognosis is the current inability to diagnose patients at an early stage. Data supports that it takes more than five years from tumor initiation until the acquisition of metastatic ability (Yachida et al., 2010), which clearly demonstrates a window of opportunity for early detection if accurate markers were available. At the time of diagnosis, patients have often developed late-stage disease, and only approximately 15% of the patients have resectable tumors (Conlon et al., 1996; Sohn et al., 2000). The 5-year survival of these patients, displaying large resected tumors, is only 10-20% (Conlon et al., 1996; Sohn et al., 2000). However, the 5-year survival increases to 30-60% if tumors ≤20 mm (Stage I-II) can be resected (Furukawa et al., 1996; Shimizu et al., 2005). The late diagnosis (Stage III-IV) is due to unspecific clinical symptoms in combination with the lack of markers for early diagnosis, something which is addressed in this study. Interestingly, studies suggest that pancreatic tumors could be resectable as early as six months prior to clinical diagnosis at an asymptomatic stage (Gangi et al., 2004; Pelaez-Luna et al., 2007).

The so far most evaluated marker for PDAC, CA19-9, suffers from poor specificity, with elevated levels in several other indications, as well as a complete absence in patients that are genotypically Lewis a-b- (5% of the population). Consequently, the use of CA19-9 for pancreatic cancer screening is not recommended (Locker et al., 2006). Today, no other single biomarker has been shown to accurately diagnose PDAC, although recent discovery studies have demonstrated that both exosomes and nucleosomes contain information associated with pancreatic cancer (Bauden et al., 2015; Melo et al., 2015). However, there is a need to provide biomarkers and biomarker panels with improved sensitivity and specificity.

### Summary of the Invention

Accordingly, a first aspect of the invention provides a method for diagnosing or determining a pancreatic cancer-associated disease state comprising or consisting of the steps of:
(a) providing a sample from an individual to be tested; and
(b) determining a biomarker signature of the test sample by measuring the presence and/or amount in the test sample of one or more biomarker selected from the group defined in Table A;
wherein the presence and/or amount in the test sample of the one or more biomarker selected from the group defined in Table A is indicative of the pancreatic cancer-associated disease in the individual.

Thus, in one embodiment, the method comprises determining a biomarker signature of the test sample, which enables a diagnosis to be reached in respect of the individual from which the sample is obtained.

By "pancreatic cancer-associated disease state" we include pancreatic cancer presence *per se,* pancreatic cancer stage and/or presence of related lesions such as intraductal papillary mucinous neoplasms (see below). In particular, we include the presence and/or stage of pancreatic ductal adenocarcinoma (PDAC).

In specific embodiments, the methods of the invention permit:
(i) diagnosis and/or staging of early pancreatic cancer; and/or
(ii) diagnosis and/or staging of pancreatic cancer (*i.e.* early or late).

By "biomarker" we mean a naturally-occurring biological molecule, or component or fragment thereof, the measurement of which can provide information useful in the diagnosis and/or prognosis of pancreatic cancer. Thus, in the context of Table A, the biomarker may be the naturally-occurring protein, or a polypeptide fragment or carbohydrate moiety thereof (or, in the case of Lewis x and sialyl Lewis x, a carbohydrate moiety *per se*). Alternatively, the biomarker may be a nucleic acid molecule, such as a mRNA, cDNA or circulating tumour DNA molecule, which encodes the protein or part thereof.

By "diagnosis" we mean or include determining the presence or absence of a disease state in an individual (e.g., determining whether an individual is or is not suffering from early pancreatic cancer or pancreatic cancer (early or late)).

By "staging" we mean or include determining the stage of a pancreatic cancer, for example, determining whether the pancreatic cancer is stage I, stage II, stage III or stage IV (e.g., stage I, stage II, stage I-II, stage III-IV or stage I-IV).

By "early pancreatic cancer" we include or mean pancreatic cancer comprising or consisting of stage I and/or stage II pancreatic cancer.

Alternatively or additionally, by "early pancreatic cancer" we include or mean stage I and/or stage II pancreatic cancer as determined by the American Joint Committee on Cancer (AJCC) TNM system (e.g., see: http://www.cancer.org/cancer/pancreaticcancer/detailedguide/pancreatic-cancer-staging and AJCC Cancer Staging Manual (7th ed.), 2011, Edge et al., Springer which are incorporated by reference herein.

The TNM cancer staging system is based on 3 key pieces of information:
▪ T describes the size of the main (primary) **tumour** and whether it has grown outside the pancreas and into nearby organs.
▪ N describes the spread to nearby (regional) lymph **nodes.**
▪ M indicates whether the cancer has **metastasized** (spread) to other organs of the body. (The most common sites of pancreatic cancer spread are the liver, lungs, and the peritoneum - the space around the digestive organs.)
Numbers or letters appear after T, N, and M to provide more details about each of these factors.

### T categories

**TX:** The main tumour cannot be assessed.
**T0:** No evidence of a primary tumour.
**Tis:** Carcinoma in situ (the tumour is confined to the top layers of pancreatic duct cells). (Very few pancreatic tumours are found at this stage.)
**T1:** The cancer is still within the pancreas and is 2 centimetres (cm) (about ¾ inch) or less across.
**T2:** The cancer is still within the pancreas but is larger than 2 cm across.
**T3:** The cancer has grown outside the pancreas into nearby surrounding tissues but not into major blood vessels or nerves.
**T4:** The cancer has grown beyond the pancreas into nearby large blood vessels or nerves.

### N categories

**NX:** Nearby (regional) lymph nodes cannot be assessed.
**N0:** The cancer has not spread to nearby lymph nodes.
**N1:** The cancer has spread to nearby lymph nodes.

### M categories

**M0:** The cancer has not spread to distant lymph nodes (other than those near the pancreas) or to distant organs such as the liver, lungs, brain, etc.
**M1:** The cancer has spread to distant lymph nodes or to distant organs.

### Stage grouping for pancreatic cancer

Once the T, N, and M categories have been determined, this information is combined to assign an overall stage of 0, I, II, III, or IV (sometimes followed by a letter). This process is called *stage grouping.*
**Stage 0 (Tis, N0, M0):** The tumor is confined to the top layers of pancreatic duct cells and has not invaded deeper tissues. It has not spread outside of the pancreas. These tumors are sometimes referred to as *pancreatic carcinoma in situ* or *pancreatic intraepithelial neoplasia III* (Panln III).
**Stage IA (T1, N0, M0):** The tumor is confined to the pancreas and is 2 cm across or smaller (T1). It has not spread to nearby lymph nodes (N0) or distant sites (M0).
**Stage IB (T2, N0, M0):** The tumor is confined to the pancreas and is larger than 2 cm across (T2). It has not spread to nearby lymph nodes (N0) or distant sites (M0).
**Stage IIA (T3, N0, M0):** The tumor is growing outside the pancreas but not into major blood vessels or nerves (T3). It has not spread to nearby lymph nodes (N0) or distant sites (M0).
**Stage IIB (T1-3, N1, M0):** The tumor is either confined to the pancreas or growing outside the pancreas but not into major blood vessels or nerves (T1-T3). It has spread to nearby lymph nodes (N1) but not to distant sites (M0).
**Stage III (T4, Any N, M0):** The tumor is growing outside the pancreas into nearby major blood vessels or nerves (T4). It may or may not have spread to nearby lymph nodes (Any N). It has not spread to distant sites (M0).
**Stage IV (Any T, Any N, M1):** The cancer has spread to distant sites (M1).

Alternatively or additionally, by "early pancreatic cancer" we include or mean asymptomatic pancreatic cancer. Common presenting symptoms of pancreatic cancers include jaundice (for tumours of the pancreas head), abdominal pain, weight loss, steatorrhoea, and new-onset diabetes. For example, the pancreatic cancer may present at least 1 week before symptoms (e.g., common symptoms) are observed or observable, for example, ≥2 weeks, ≥3 weeks, ≥4 weeks, ≥5 weeks, ≥6 weeks, ≥7 weeks, ≥8 weeks, ≥3 months, ≥4 months, ≥5 months, ≥6 months, ≥7 months, ≥8 months, ≥9 months, ≥10 months, ≥11 months, ≥12 months, ≥18 months, ≥2 years, ≥3 years, ≥4 years, or ≥5 years, before symptoms are observed or observable.

Alternatively and/or additionally, by "early pancreatic cancer" we include pancreatic cancers that are of insufficient size and/or developmental stage to be diagnosed by conventional clinical methods.

Alternatively or additionally, by "early pancreatic cancer" we include or mean pancreatic cancers present at least 1 week before the pancreatic cancer is diagnosed or diagnosable by conventional clinical methods, for example, ≥2 weeks, ≥3 weeks, ≥4 weeks, ≥5 weeks, ≥6 weeks, ≥7 weeks, ≥8 weeks, ≥3 months, ≥4 months, ≥5 months, ≥6 months, ≥7 months, ≥8 months, ≥9 months, ≥10 months, ≥11 months, ≥12 months, ≥18 months, ≥2 years, ≥3 years, ≥4 years, or ≥5 years, before the pancreatic cancer is diagnosed or diagnosable by convention clinical methods.

The contemporary best practice for clinical pancreatic cancer diagnosis will be well known to the person of skill in the art, however, for a detailed review see Ducreux et al., 2015, 'Cancer of the pancreas: ESMO Clinical Practice Guidelines for diagnosis, treatment and follow-up' Annals of Oncology, 26 (Supplement 5): v56-v68 which is incorporated by reference herein and con be obtained here:
https://annonc.oxfordjournals.org/content/26/suppl_5/v56.full.pdf+html.

Alternatively or additionally, by "conventional clinical diagnoses" (and the like [e.g., "diagnosed by conventional clinical methods"]) we include CT scan, ultrasound, endoscopic ultrasound, biopsy (histopathology) and/or physical examination (e.g., of the abdomen and, possibly, local lymph nodes). In one embodiment by "conventional clinical diagnoses" (and the like) we include the pancreatic cancer diagnosis procedures set out in Ducreux et al., 2015, 'Cancer of the pancreas: ESMO Clinical Practice Guidelines for diagnosis, treatment and follow-up' Annals of Oncology, 26 (Supplement 5): v56-v68, which is incorporated by reference herein.

Alternatively or additionally by "conventional clinical diagnoses" (and the like) may include or exclude the use of molecular biomarkers present in bodily fluids (such as blood, serum, interstitial fluid, lymph, urine, mucus, saliva, sputum, sweat) and or tissues.

Alternatively or additionally, by "early pancreatic cancer" we include or mean resectable pancreatic cancer.

By "resectable pancreatic cancer" we include or mean that the pancreatic cancer comprises or consists of tumours that are (and/or are considered) capable of being removed by surgery (i.e., are resectable).

Alternatively or additionally we include pancreatic cancers that are limited to the pancreas (i.e., do not extend beyond the pancreas and/or have not metastasised).

Alternatively or additionally by "early pancreatic cancer" we include pancreatic cancers comprising tumours of 30 mm or less in all dimensions (i.e., in this embodiment individuals with early pancreatic cancer do not comprise pancreatic cancer tumours of greater than 30 mm in any dimension), for example, equal to or less than 29mm, 28mm, 27mm, 26mm, 25mm, 24mm, 22mm, 21mm, 20mm, 19 mm, 18 mm, 17 mm, 16 mm, 15 mm, 14 mm, 13 mm, 12 mm, 11 mm, 10 mm, 9 mm, 8 mm, 7 mm, 6 mm, 5 mm, 4 mm, 3 mm, 2 mm, 1 mm or equal to or 0.1 mm in all dimensions. Alternatively or additionally, the pancreatic cancer tumours of 30 mm or less in all dimensions are at least 2 mm in one dimension. Alternatively or additionally, the pancreatic cancer tumours of 30 mm or less in all dimensions are at least 2 mm all dimensions.

Alternatively or additionally, by "stage I pancreatic cancer" we include or mean AJCC stage IA and/or IB.

Alternatively or additionally, by "stage II pancreatic cancer" we include or mean AJCC stage IIA and/or IIB

Alternatively or additionally, by "late pancreatic cancer" we include or mean pancreatic cancer comprising or consisting of stage III and/or stage IV pancreatic cancer.

Alternatively or additionally, by "stage III pancreatic cancer" we include or mean AJCC stage III.

Alternatively or additionally, by "stage IV pancreatic cancer" we include or mean AJCC stage IV.

By a pancreatic cancer-associated disease state of "pancreatic cancer" we include pancreatic cancer comprising or consisting of pancreatic cancer of any stage.

By "sample to be tested", "test sample" or "control sample" we include a tissue or fluid sample taken or derived from an individual. Preferably the sample to be tested is provided from a mammal. The mammal may be any domestic or farm animal. Preferably, the mammal is a rat, mouse, guinea pig, cat, dog, horse or a primate. Most preferably, the mammal is human.

Preferably the sample is a cell, tissue or fluid sample (or derivative thereof) comprising or consisting of blood (fractionated or unfractionated), plasma, plasma cells, serum, tissue cells or equally preferred, protein or nucleic acid derived from a cell or tissue sample. Preferably test and control samples are derived from the same species. Preferably test and control samples are matched for age, gender and/or lifestyle.

In an alternative or additional embodiment the tissue sample is pancreatic tissue. In an alternative or additional embodiment, the cell sample is a sample of pancreatic cells.

Alternatively or additionally the pancreatic cancer may be diagnosed using conventional clinical methods known in the art. For example, those methods described in Ducreux et al., 2015, 'Cancer of the pancreas: ESMO Clinical Practice Guidelines for diagnosis, treatment and follow-up' Annals of Oncology, 26 (Supplement 5): v56-v68 and/or Freelove & Walling, 2006, 'Pancreatic Cancer: Diagnosis and Management' American Family Physician, 73(3):485-492 which are incorporated herein by reference

Accordingly, the pancreatic cancer may be diagnosed using one or more method selected from the group consisting of:
computed tomography (preferably dual-phase helical computed tomography); transabdominal ultrasonography; endoscopic ultrasonographyguided fine-needle aspiration; endoscopic retrograde cholangiopancreatography; positron emission tomography; magnetic resonance imaging; physical examination; and biopsy.

Alternatively and/or additionally, the pancreatic cancer may be diagnosed using detection of biomarkers for the diagnosis of pancreatic cancer. For example, the pancreatic cancer may be diagnosed with one or more biomarker or diagnostic method described in the group consisting of: WO 2008/117067 A9; WO 2012/120288 A2; and WO 2015/067969 A2.

Alternatively or additionally, step (b) comprises or consists of measuring the presence and/or amount of 1 or more biomarker listed in Table A, for example at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84 or 85 of the biomarkers listed in Table A.

Alternatively or additionally, step (b) comprises, consists of or excludes measuring the expression of CHP-1. Alternatively or additionally, step (b) comprises, consists of or excludes measuring the expression of MAPKK 2. Alternatively or additionally, step (b) comprises, consists of or excludes measuring the expression of MAPKK 6. Alternatively or additionally, step (b) comprises, consists of or excludes measuring the expression of R-PTP-O. Alternatively or additionally, step (b) comprises, consists of or excludes measuring the expression of UBP7. Alternatively or additionally, step (b) comprises, consists of or excludes measuring the expression of Apo-A1. Alternatively or additionally, step (b) comprises, consists of or excludes measuring the expression of BTK. Alternatively or additionally, step (b) comprises, consists of or excludes measuring the expression of C1q. Alternatively or additionally, step (b) comprises, consists of or excludes measuring the expression of C5. Alternatively or additionally, step (b) comprises, consists of or excludes measuring the expression of CDK-2. Alternatively or additionally, step (b) comprises, consists of or excludes measuring the expression of IgM. Alternatively or additionally, step (b) comprises, consists of or excludes measuring the expression of IL-11. Alternatively or additionally, step (b) comprises, consists of or excludes measuring the expression of IL-12. Alternatively or additionally, step (b) comprises, consists of or excludes measuring the expression of IL-6. Alternatively or additionally, step (b) comprises, consists of or excludes measuring the expression of JAK3. Alternatively or additionally, step (b) comprises, consists of or excludes measuring the expression of MAPK8. Alternatively or additionally, step (b) comprises, consists of or excludes measuring the expression of MCP-1. Alternatively or additionally, step (b) comprises, consists of or excludes measuring the expression of MUC-1. Alternatively or additionally, step (b) comprises, consists of or excludes measuring the expression of Properdin. Alternatively or additionally, step (b) comprises, consists of or excludes measuring the expression of VEGF. Alternatively or additionally, step (b) comprises, consists of or excludes measuring the expression of GRIP-2. Alternatively or additionally, step (b) comprises, consists of or excludes measuring the expression of MAPK9. Alternatively or additionally, step (b) comprises, consists of or excludes measuring the expression of PKB gamma. Alternatively or additionally, step (b) comprises, consists of or excludes measuring the expression of PRD14. Alternatively or additionally, step (b) comprises, consists of or excludes measuring the expression of R-PTP-eta. Alternatively or additionally, step (b) comprises, consists of or excludes measuring the expression of TopBP1. Alternatively or additionally, step (b) comprises, consists of or excludes measuring the expression of C3. Alternatively or additionally, step (b) comprises, consists of or excludes measuring the expression of CD40L. Alternatively or additionally, step (b) comprises, consists of or excludes measuring the expression of EGFR. Alternatively or additionally, step (b) comprises, consists of or excludes measuring the expression of HADH2. Alternatively or additionally, step (b) comprises, consists of or excludes measuring the expression of ICAM-1. Alternatively or additionally, step (b) comprises, consists of or excludes measuring the expression of IL-13. Alternatively or additionally, step (b) comprises, consists of or excludes measuring the expression of IL-18. Alternatively or additionally, step (b) comprises, consists of or excludes measuring the expression of Myomesin-2. Alternatively or additionally, step (b) comprises, consists of or excludes measuring the expression of P85A. Alternatively or additionally, step (b) comprises, consists of or excludes measuring the expression of RANTES. Alternatively or additionally, step (b) comprises, consists of or excludes measuring the expression of TGF-b1. Alternatively or additionally, step (b) comprises, consists of or excludes measuring the expression of IL-4. Alternatively or additionally, step (b) comprises, consists of or excludes measuring the expression of CIMS (13). Alternatively or additionally, step (b) comprises, consists of or excludes measuring the expression of GNAl3. Alternatively or additionally, step (b) comprises, consists of or excludes measuring the expression of HsMAD2. Alternatively or additionally, step (b) comprises, consists of or excludes measuring the expression of hSpindly. Alternatively or additionally, step (b) comprises, consists of or excludes measuring the expression of R-PTP-kappa. Alternatively or additionally, step (b) comprises, consists of or excludes measuring the expression of STAT1. Alternatively or additionally, step (b) comprises, consists of or excludes measuring the expression of ATP-5B. Alternatively or additionally, step (b) comprises, consists of or excludes measuring the expression of C4. Alternatively or additionally, step (b) comprises, consists of or excludes measuring the expression of CHX10. Alternatively or additionally, step (b) comprises, consists of or excludes measuring the expression of Factor B. Alternatively or additionally, step (b) comprises, consists of or excludes measuring the expression of Her2/ErbB-2. Alternatively or additionally, step (b) comprises, consists of or excludes measuring the expression of IL-1b. Alternatively or additionally, step (b) comprises, consists of or excludes measuring the expression of IL-7. Alternatively or additionally, step (b) comprises, consists of or excludes measuring the expression of Lewis X. Alternatively or additionally, step (b) comprises, consists of or excludes measuring the expression of MCP-3. Alternatively or additionally, step (b) comprises, consists of or excludes measuring the expression of Sialyl Lewis x. Alternatively or additionally, step (b) comprises, consists of or excludes measuring the expression of TBC1D9. Alternatively or additionally, step (b) comprises, consists of or excludes measuring the expression of TNFRSF3. Alternatively or additionally, step (b) comprises, consists of or excludes measuring the expression of AGAP-2. Alternatively or additionally, step (b) comprises, consists of or excludes measuring the expression of C1-INH. Alternatively or additionally, step (b) comprises, consists of or excludes measuring the expression of C1s. Alternatively or additionally, step (b) comprises, consists of or excludes measuring the expression of GLP-1 R. Alternatively or additionally, step (b) comprises, consists of or excludes measuring the expression of IL-2. Alternatively or additionally, step (b) comprises, consists of or excludes measuring the expression of KSYK. Alternatively or additionally, step (b) comprises, consists of or excludes measuring the expression of MAPK1. Alternatively or additionally, step (b) comprises, consists of or excludes measuring the expression of TENS4. Alternatively or additionally, step (b) comprises, consists of or excludes measuring the expression of FASN. Alternatively or additionally, step (b) comprises, consists of or excludes measuring the expression of IL-10. Alternatively or additionally, step (b) comprises, consists of or excludes measuring the expression of IL-3. Alternatively or additionally, step (b) comprises, consists of or excludes measuring the expression of IL-8. Alternatively or additionally, step (b) comprises, consists of or excludes measuring the expression of STAP2. Alternatively or additionally, step (b) comprises, consists of or excludes measuring the expression of CT17. Alternatively or additionally, step (b) comprises, consists of or excludes measuring the expression of Digoxin. Alternatively or additionally, step (b) comprises, consists of or excludes measuring the expression of HsHec1. Alternatively or additionally, step (b) comprises, consists of or excludes measuring the expression of PAR-6B. Alternatively or additionally, step (b) comprises, consists of or excludes measuring the expression of PGAM5. Alternatively or additionally, step (b) comprises, consists of or excludes measuring the expression of IFN-γ.

Alternatively or additionally, the pancreatic cancer-associated disease state is early pancreatic cancer.

Alternatively or additionally, the method is for the diagnosis and/or staging of stage I or stage II pancreatic cancer (i.e., diagnosing pancreatic cancer and/or characterising the pancreatic cancer as stage I or stage II without necessarily determining which). Alternatively or additionally, step (b) comprises or consists of measuring the presence and/or amount of 1 or more biomarker listed in:
Table A(I), for example at least 2, 3, 4 or 5 of the biomarkers listed in Table A(I);
Table A(III), for example at least 2, 3, 4, 5 or 6 of the biomarkers listed in Table A(III);
Table A(V), for example at least 2, 3, 4, 5 or 6 of the biomarkers listed in Table A(V); and
Table A(XI), for example at least 2 or 3 of the biomarkers listed in Table A(XI).

Alternatively or additionally, step (b) comprises or consists of measuring the presence and/or amount of 1 or more biomarker listed in:
Table A(II), for example at least 2, 3, 4, 5, 6, 7, 8, 9, 10, 11, 12, 13, 14 15 or 16 of the biomarkers listed in Table A(II);
Table A(IV), for example at least 2, 3, 4, 5, 6, 7, 8, 9, 10, 11 or 12 of the biomarkers listed in Table A(IV);
Table A(VI), for example at least 2, 3, 4, 5, 6, 7, 8, 9, 10, 11, 12 or 13 of the biomarkers listed in Table A(VI); and
Table A(XII), for example at least 2, 3, 4, 5 or 6 of the biomarkers listed in Table A(XII).

Alternatively or additionally, the method is for the diagnosis and/or staging of stage I pancreatic cancer (i.e., diagnosing pancreatic cancer and/or characterising the pancreatic cancer as stage I).

Alternatively or additionally, step (b) comprises or consists of measuring the presence and/or amount of 1 or more biomarker listed in:
Table A(I), for example at least 2, 3, 4 or 5 of the biomarkers listed in Table A(I);
Table A(III), for example at least 2, 3, 4, 5 or 6 of the biomarkers listed in Table A(III);
Table A(VII).

Alternatively or additionally, step (b) comprises or consists of measuring the presence and/or amount of 1 or more biomarker listed in:
Table A(II), for example at least 2, 3, 4, 5, 6, 7, 8, 9, 10, 11, 12, 13, 14, 15 or 16 of the biomarkers listed in Table A(II);
Table A(IV), for example at least 2, 3, 4, 5, 6, 7, 8, 9, 10, 11 or 12 of the biomarkers listed in Table A(IV); and
Table A(VIII), for example at least 2, 3, 4, 5 or 6 of the biomarkers listed in Table A(VIII).

Alternatively or additionally, the method is for the diagnosis and/or staging of stage II pancreatic cancer.

Alternatively or additionally, step (b) comprises or consists of measuring the presence and/or amount of 1 or more biomarker listed in:
Table A(I), for example at least 2, 3, 4 or 5 of the biomarkers listed in Table A(I);
Table A(V), for example at least 2, 3, 4, 5 or 6 of the biomarkers listed in Table A(V);
Table A(IX).

Alternatively or additionally, step (b) comprises or consists of measuring the presence and/or amount of 1 or more biomarker listed in:
Table A(II), for example at least 2, 3, 4, 5, 6, 7, 8, 9, 10, 11, 12, 13, 14, 15 or 16 of the biomarkers listed in Table A(II);
Table A(VI), for example at least 2, 3, 4, 5, 6, 7, 8, 9, 10, 11, 12 or 13 of the biomarkers listed in Table A(VI); and
Table A(X), for example at least 2, 3 or 4 of the biomarkers listed in Table A(X).

Alternatively or additionally, the method is for diagnosing or characterising stage III or IV pancreatic cancer (i.e., diagnosing pancreatic cancer and/or characterising the pancreatic cancer as stage **III** or IV), comprising or consisting of measuring the presence or amount of one or more biomarker for diagnosing or characterising stage I or II pancreatic cancer as defined above and measuring the presence or amount of one or more biomarker for diagnosing or characterising stage I-IV pancreatic cancer as defined above. Alternatively or additionally, one or more of the stage 'I or II' biomarkers is different from one or more of the 'stage I-IV biomarkers'. Alternatively or additionally, all of the stage 'I or II' biomarkers is different from one or more of the 'stage I-IV biomarkers'.

Alternatively or additionally, the pancreatic cancer-associated disease state is pancreatic cancer (i.e., the method is for diagnosing the presence of pancreatic cancer).

Alternatively or additionally, step (b) comprises or consists of measuring the presence and/or amount of 1 or more biomarker selected from the group consisting of CHP-1, MAPKK 2, UBP7, PRD14, STAT1, AGAP-2, PGAM5, LUM, PTPRO and USP07, for example, at least 2, 3, 4, 5, 6, 7, 8, 9 or 10 of these biomarkers.

Alternatively or additionally, step (b) comprises or consists of measuring the presence and/or amount of 1 or more biomarker selected from the group consisting of Apo-A1, BTK, C1q, C5, CDK-2, IgM, IL-11, IL-12, IL-6, JAK3, MAPK8, MCP-1, MUC-1, Properdin, VEGF, C3, ICAM-1, IL-13, ATP-5B, C4, Her2/ErB-2, IL-7, IL-3, IL-8, GM-CSF, IL-9, LDL and ORP3, for example, at least 2, 3, 4, 5, 6, 7, 8, 9, 10, 11, 12, 13, 14, 15, 16, 17, 18, 19, 20, 21, 22, 23, 24, 25, 26, 27, 28 or 29 of these biomarkers.

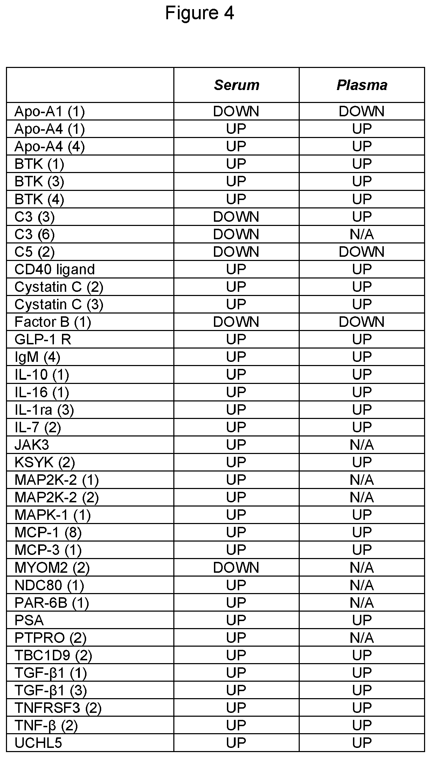

Alternatively or additionally, step (b) comprises measuring one or more of the biomarkers listed in Figure 4, for example, at least 2, 3, 4, 5, 6, 7, 8, 9, 10, 11, 12, 13, 14, 15, 16, 17, 18, 19, 20, 21, 22, 23, 24, 25, 26, 27, 28, 29, 30 or 31 of the biomarkers listed in Figure 4.

Alternatively or additionally, step (b) comprises measuring the presence and/or amount of all of the biomarkers listed in Table A.

Alternatively or additionally the method further comprises or consists of the steps of:
(c) providing one or more control sample from:
   (i) an individual not afflicted with pancreatic cancer; and/or
   (ii) an individual afflicted with pancreatic cancer, wherein the sample was of a different stage to that of that the test sample;
(d) determining a biomarker signature of the one or more control sample by measuring the presence and/or amount in the control sample of the one or more biomarkers measured in step (b);
wherein the pancreatic cancer-associated disease state is identified in the event that the presence and/or amount in the test sample of the one or more biomarkers measured in step (b) is different from the presence and/or amount in the control sample of the one or more biomarkers measured in step (d).

By "is different to the presence and/or amount in a control sample" we mean or include the presence and/or amount of the one or more biomarker in the test sample differs from that of the one or more control sample (or to predefined reference values representing the same). Preferably the presence and/or amount in the test sample differs from the presence or amount in the one or more control sample (or mean of the control samples) by at least ±5%, for example, at least ±6%, ±7%, ±8%, ±9%, ±10%, ±11%, ±12%, ±13%, ±14%, ±15%, ±16%, ±17%, ±18%, ±19%, ±20%, ±21%, ±22%, ±23%, ±24%, ±25%, ±26%, ±27%, ±28%, ±29%, ±30%, ±31%, ±32%, ±33%, ±34%, ±35%, ±36%, ±37%, ±38%, ±39%, ±40%, ±41%, ±42%, ±43%, ±44%, ±45%, ±41%, ±42%, ±43%, ±44%, ±55%, ±60%, ±65%, ±66%, ±67%, ±68%, ±69%, ±70%, ±71%, ±72%, ±73%, ±74%, ±75%, ±76%, ±77%, ±78%, ±79%, ±80%, ±81%, ±82%, ±83%, ±84%, ±85%, ±86%, ±87%, ±88%, ±89%, ±90%, ±91%, ±92%, ±93%, ±94%, ±95%, ±96%, ±97%, ±98%, ±99%, ±100%, ±125%, ±150%, ±175%, ±200%, ±225%, ±250%, ±275%, ±300%, ±350%, ±400%, ±500% or at least ±1000% of the one or more control sample (e.g., the negative control sample).

Alternatively or additionally, the presence or amount in the test sample differs from the mean presence or amount in the control samples by at least >1 standard deviation from the mean presence or amount in the control samples, for example, ≥1.5, ≥2, ≥3, ≥4, ≥5, ≥6, ≥7, ≥8, ≥9, ≥10, ≥11, ≥12, ≥13, ≥14 or ≥15 standard deviations from the from the mean presence or amount in the control samples. Any suitable means may be used for determining standard deviation (e.g., direct, sum of square, Welford's), however, in one embodiment, standard deviation is determined using the direct method (i.e., the square root of [the sum the squares of the samples minus the mean, divided by the number of samples]).

Alternatively or additionally, by "is different to the presence and/or amount in a control sample" we mean or include that the presence or amount in the test sample does not correlate with the amount in the control sample in a statistically significant manner. By "does not correlate with the amount in the control sample in a statistically significant manner" we mean or include that the presence or amount in the test sample correlates with that of the control sample with a p-value of >0.001, for example, >0.002, >0.003, >0.004, >0.005, >0.01, >0.02, >0.03, >0.04 >0.05, >0.06, >0.07, >0.08, >0.09 or >0.1. Any suitable means for determining p-value known to the skilled person can be used, including z-test, *t*-test, Student's *t*-test*,* f-test, Mann-Whitney *U* test, Wilcoxon signed-rank test and Pearson's chi-squared test.

Alternatively or additionally the method further comprises or consists of the steps of:
providing one or more control sample from;
   (e) an individual afflicted with pancreatic cancer (i.e., a positive control); and/or
   (f) an individual afflicted with pancreatic cancer, wherein the sample was of the same stage to that of that the test sample;
   determining a biomarker signature of the control sample by measuring the presence and/or amount in the control sample of the one or more biomarkers measured in step (b);
wherein the pancreatic cancer-associated disease state is identified in the event that the presence and/or amount in the test sample of the one or more biomarkers measured in step (b) corresponds to the presence and/or amount in the control sample of the one or more biomarkers measured in step (f).

By "corresponds to the presence and/or amount in a control sample" we mean or include the presence and/or amount is identical to that of a positive control sample; or closer to that of one or more positive control sample than to one or more negative control sample (or to predefined reference values representing the same). Preferably the presence and/or amount is within ±40% of that of the one or more control sample (or mean of the control samples), for example, within ±39%, ±38%, ±37%, ±36%, ±35%, ±34%, ±33%, ±32%, ±31%, ±30%, ±29%, ±28%, ±27%, ±26%, ±25%, ±24%, ±23%, ±22%, ±21%, ±20%, ±19%, ±18%, ±17%, ±16%, ±15%, ±14%, ±13%, ±12%, ±11%, ±10%, ±9%, ±8%, ±7%, ±6%, ±5%, ±4%, ±3%, ±2%, ±1%, ±0.05% or within 0% of the one or more control sample (e.g., the positive control sample).

Alternatively or additionally, the difference in the presence or amount in the test sample is ≤5 standard deviation from the mean presence or amount in the control samples, for example, ≤4.5, ≤4, ≤3.5, ≤3, ≤2.5, ≤2, ≤1.5, ≤1.4, ≤1.3, ≤1.2, ≤1.1, ≤1, ≤0.9, ≤0.8, ≤0.7, ≤0.6, ≤0.5, ≤0.4, ≤0.3, ≤0.2, ≤0.1 or 0 standard deviations from the from the mean presence or amount in the control samples, provided that the standard deviation ranges for differing and corresponding biomarker expressions do not overlap (e.g., abut, but no not overlap).

Alternatively or additionally, by "corresponds to the presence and/or amount in a control sample" we mean or include that the presence or amount in the test sample correlates with the amount in the control sample in a statistically significant manner. By "correlates with the amount in the control sample in a statistically significant manner" we mean or include that the presence or amount in the test sample correlates with the that of the control sample with a p-value of ≤0.05, for example, ≤0.04, ≤0.03, ≤0.02, ≤0.01, ≤0.005, ≤0.004, ≤0.003, ≤0.002, ≤0.001, ≤0.0005 or ≤0.0001.

Differential expression (up-regulation or down regulation) of biomarkers, or lack thereof, can be determined by any suitable means known to a skilled person. Differential expression is determined to a p value of a least less than 0.05 (p = < 0.05), for example, at least <0.04, <0.03, <0.02, <0.01, <0.009, <0.005, <0.001, <0.0001, <0.00001 or at least <0.000001. Alternatively or additionally, differential expression is determined using a support vector machine (SVM). Alternatively or additionally, the SVM is an SVM as described below. Most preferably, the SVM is, or is derived from, the SVM described in Table 5, below.

It will be appreciated by persons skilled in the art that differential expression may relate to a single biomarker or to multiple biomarkers considered in combination (i.e. as a biomarker signature). Thus, a p value may be associated with a single biomarker or with a group of biomarkers. Indeed, proteins having a differential expression p value of greater than 0.05 when considered individually may nevertheless still be useful as biomarkers in accordance with the invention when their expression levels are considered in combination with one or more other biomarkers.

As exemplified in the accompanying examples, the expression of certain proteins in a tissue, blood, serum or plasma test sample may be indicative of pancreatic cancer in an individual. For example, the relative expression of certain serum proteins in a single test sample may be indicative of the presence of pancreatic cancer in an individual.

In an alternative or additional embodiment the presence and/or amount in the test sample of the one or more biomarkers measured in step (b) are compared against predetermined reference values representative of the measurements in steps (d) and/or (f).

Alternatively or additionally, the individual from which the one or more control sample was obtained was not, at the time the sample was obtained, afflicted with a non-cancerous pancreatic disease or condition, for example acute pancreatitis, chronic pancreatitis and autoimmune pancreatitis, Intraductal Papillary Mucinous Neoplasia (IPMN) of the Pancreas.

Alternatively or additionally, the individual from which the one or more control sample was obtained was not, at the time the sample was obtained, afflicted with any disease or condition of the pancreas.

Alternatively or additionally, the individual not afflicted with pancreatic cancer was not, at the time the sample was obtained, afflicted with any disease or condition.

Alternatively or additionally, the individual not afflicted with pancreatic cancer is a healthy individual.

Alternatively or additionally, the one or more individual afflicted with pancreatic cancer is afflicted with a pancreatic cancer selected from the group consisting of adenocarcinoma (e.g., pancreatic ductal adenocarcinoma or tubular papillary pancreatic adenocarcinoma), pancreatic sarcoma, malignant serous cystadenoma, adenosquamous carcinoma, signet ring cell carcinoma, hepatoid carcinoma, colloid carcinoma, undifferentiated carcinoma, and undifferentiated carcinomas with osteoclast-like giant cells.

Alternatively or additionally, the pancreatic cancer is pancreatic adenocarcinoma (e.g., pancreatic ductal adenocarcinoma).

Alternatively or additionally, the one or more individual afflicted with pancreatic cancer is *not* afflicted with one or more pancreatic cancer selected from the group consisting of adenocarcinoma (e.g., pancreatic ductal adenocarcinoma or tubular papillary pancreatic adenocarcinoma), pancreatic sarcoma, malignant serous cystadenoma, adenosquamous carcinoma, signet ring cell carcinoma, hepatoid carcinoma, colloid carcinoma, undifferentiated carcinoma, and undifferentiated carcinomas with osteoclast-like giant cells.

Alternatively or additionally, the method is repeated.

Alternatively or additionally, the method is repeated and wherein, in step (a), the sample to be tested is taken at different time to the previous method repetition.

Alternatively or additionally, the method is repeated using a test sample taken at a different time period to the previous test sample(s) used.

Alternatively or additionally, the method is repeated using a test sample taken between 1 day to 104 weeks to the previous test sample(s) used, for example, between 1 week to 100 weeks, 1 week to 90 weeks, 1 week to 80 weeks, 1 week to 70 weeks, 1 week to 60 weeks, 1 week to 50 weeks, 1 week to 40 weeks, 1 week to 30 weeks, 1 week to 20 weeks, 1 week to 10 weeks, 1 week to 9 weeks, 1 week to 8 weeks, 1 week to 7 weeks, 1 week to 6 weeks, 1 week to 5 weeks, 1 week to 4 weeks, 1 week to 3 weeks, or 1 week to 2 weeks.

Alternatively or additionally, the method is repeated using a test sample taken every period from the group consisting of: 1 day, 2 days, 3 day, 4 days, 5 days, 6 days, 7 days, 10 days, 2 weeks, 3 weeks, 4 weeks, 5 weeks, 6 weeks, 7 weeks, 8 weeks, 9 weeks, 10 weeks, 15 weeks, 20 weeks, 25 weeks, 30 weeks, 35 weeks, 40 weeks, 45 weeks, 50 weeks, 55 weeks, 60 weeks, 65 weeks, 70 weeks, 75 weeks, 80 weeks, 85 weeks, 90 weeks, 95 weeks, 100 weeks, 104, weeks, 105 weeks, 110 weeks, 115 weeks, 120 weeks, 125 weeks and 130 weeks.

Alternatively or additionally, the method is repeated at least once, for example, 2 times, 3 times, 4 times, 5 times, 6 times, 7 times, 8 times, 9 times, 10 times, 11 times, 12 times, 13 times, 14 times, 15 times, 16 times, 17 times, 18 times, 19 times, 20 times, 21 times, 22 times, 23, 24 times or 25 times.

Alternatively or additionally, the method is repeated continuously.

Alternatively or additionally, the method is repeated until pancreatic cancer is diagnosed and/or staged in the individual using the methods of the present invention and/or conventional clinical methods.

Alternatively or additionally, each repetition uses test sample taken from the same individual.

Alternatively or additionally, step (b) comprises measuring the expression of the protein or polypeptide of the one or more biomarker(s).

Alternatively or additionally, step (b), (d) and/or step (f) is performed using one or more first binding agent capable of binding to a biomarker listed in Table A. It will be appreciated by persons skilled in the art that the first binding agent may comprise or consist of a single species with specificity for one of the protein biomarkers or a plurality of different species, each with specificity for a different protein biomarker.

Suitable binding agents (also referred to as binding molecules) can be selected from a library, based on their ability to bind a given motif, as discussed below.

At least one type of the binding agents, and more typically all of the types, may comprise or consist of an antibody or antigen-binding fragment of the same, or a variant thereof.

Methods for the production and use of antibodies are well known in the art, for example see Antibodies: A Laboratory Manual, 1988, Harlow & Lane, Cold Spring Harbor Press, ISBN-13: 978-0879693145, Using Antibodies: A Laboratory Manual, 1998, Harlow & Lane, Cold Spring Harbor Press, ISBN-13: 978-0879695446 and Making and Using Antibodies: A Practical Handbook, 2006, Howard & Kaser, CRC Press, ISBN-13: 978-0849335280 (the disclosures of which are incorporated herein by reference).

Thus, a fragment may contain one or more of the variable heavy (V_{H}) or variable light (V_{L}) domains. For example, the term antibody fragment includes Fab-like molecules (Better et al (1988) Science 240, 1041); Fv molecules (Skerra et al (1988) Science 240, 1038); single-chain Fv (ScFv) molecules where the V_{H} and V_{L} partner domains are linked via a flexible oligopeptide (Bird et al (1988) Science 242, 423; Huston et al (1988) Proc. Natl. Acad. Sci. USA 85, 5879) and single domain antibodies (dAbs) comprising isolated V domains (Ward et al (1989) Nature 341, 544).

The term "antibody variant" includes any synthetic antibodies, recombinant antibodies or antibody hybrids, such as but not limited to, a single-chain antibody molecule produced by phage-display of immunoglobulin light and/or heavy chain variable and/or constant regions, or other immunointeractive molecule capable of binding to an antigen in an immunoassay format that is known to those skilled in the art.

A general review of the techniques involved in the synthesis of antibody fragments which retain their specific binding sites is to be found in Winter & Milstein (1991) Nature 349, 293-299.

Molecular libraries such as antibody libraries (Clackson et al, 1991, Nature 352, 624-628; Marks et al, 1991, J Mol Biol 222(3): 581-97), peptide libraries (Smith, 1985, Science 228(4705): 1315-7), expressed cDNA libraries (Santi et al (2000) J Mol Biol 296(2): 497-508), libraries on other scaffolds than the antibody framework such as affibodies (Gunneriusson et al, 1999, Appl Environ Microbiol 65(9): 4134-40) or libraries based on aptamers (Kenan et al, 1999, Methods Mol Biol 118, 217-31) may be used as a source from which binding molecules that are specific for a given motif are selected for use in the methods of the invention.

The molecular libraries may be expressed *in vivo* in prokaryotic (Clackson *et al,* 1991, op. *cit*.; Marks *et al,* 1991, *op. cit*.) or eukaryotic cells (Kieke et al, 1999, Proc Natl Acad Sci USA, 96(10):5651-6) or may be expressed *in vitro* without involvement of cells (Hanes & Pluckthun, 1997, Proc Natl Acad Sci USA 94(10):4937-42; He & Taussig, 1997, Nucleic Acids Res 25(24):5132-4; Nemoto et al, 1997, FEBS Lett, 414(2):405-8).

In cases when protein based libraries are used often the genes encoding the libraries of potential binding molecules are packaged in viruses and the potential binding molecule is displayed at the surface of the virus (Clackson *et al,* 1991, *op. cit*.; Marks *et al,* 1991, op. *cit;* Smith, 1985, *op. cit*.).

The most commonly used such system today is filamentous bacteriophage displaying antibody fragments at their surfaces, the antibody fragments being expressed as a fusion to the minor coat protein of the bacteriophage (Clackson *et al,* 1991, *op. cit*.; Marks *et al,* 1991, *op. cit*). However, also other systems for display using other viruses (EP 39578), bacteria (Gunneriusson *et al,* 1999, *op. cit*.; Daugherty et al, 1998, Protein Eng 11(9):825-32; Daugherty et al, 1999, Protein Eng 12(7):613-21), and yeast (Shusta et al, 1999, J Mol Biol 292(5):949-56) have been used.

In addition, display systems have been developed utilising linkage of the polypeptide product to its encoding mRNA in so called ribosome display systems (Hanes & Pluckthun, 1997, *op. cit*.; He & Taussig, 1997, *op. cit*.; Nemoto *et al,* 1997, *op. cit*.), or alternatively linkage of the polypeptide product to the encoding DNA (see US Patent No. 5,856,090 and WO 98/37186).

When potential binding molecules are selected from libraries one or a few selector peptides having defined motifs are usually employed. Amino acid residues that provide structure, decreasing flexibility in the peptide or charged, polar or hydrophobic side chains allowing interaction with the binding molecule may be used in the design of motifs for selector peptides.

For example:
(i) Proline may stabilise a peptide structure as its side chain is bound both to the alpha carbon as well as the nitrogen;
(ii) Phenylalanine, tyrosine and tryptophan have aromatic side chains and are highly hydrophobic, whereas leucine and isoleucine have aliphatic side chains and are also hydrophobic;
(iii) Lysine, arginine and histidine have basic side chains and will be positively charged at neutral pH, whereas aspartate and glutamate have acidic side chains and will be negatively charged at neutral pH;
(iv) Asparagine and glutamine are neutral at neutral pH but contain a amide group which may participate in hydrogen bonds;
(v) Serine, threonine and tyrosine side chains contain hydroxyl groups, which may participate in hydrogen bonds.

Typically, selection of binding agents may involve the use of array technologies and systems to analyse binding to spots corresponding to types of binding molecules.

In an alternative or additional embodiment, the first binding agent(s) is/are immobilised on a surface (e.g. on a multiwell plate or array).

The variable heavy (V_{H}) and variable light (V_{L}) domains of the antibody are involved in antigen recognition, a fact first recognised by early protease digestion experiments. Further confirmation was found by "humanisation" of rodent antibodies. Variable domains of rodent origin may be fused to constant domains of human origin such that the resultant antibody retains the antigenic specificity of the rodent parented antibody (Morrison et al (1984) Proc. Natl. Acad. Sci. USA 81, 6851-6855).

That antigenic specificity is conferred by variable domains and is independent of the constant domains is known from experiments involving the bacterial expression of antibody fragments, all containing one or more variable domains. These molecules include Fab-like molecules (Better et al (1988) Science 240, 1041); Fv molecules (Skerra et al (1988) Science 240, 1038); single-chain Fv (ScFv) molecules where the V_{H} and V_{L} partner domains are linked via a flexible oligopeptide (Bird et al (1988) Science 242, 423; Huston et al (1988) Proc. Natl. Acad. Sci. USA 85, 5879) and single domain antibodies (dAbs) comprising isolated V domains (Ward et al (1989) Nature 341, 544). A general review of the techniques involved in the synthesis of antibody fragments which retain their specific binding sites is to be found in Winter & Milstein (1991) Nature 349, 293-299.

By "ScFv molecules" we mean molecules wherein the V_{H} and V_{L} partner domains are linked via a flexible oligopeptide.

The advantages of using antibody fragments, rather than whole antibodies, are several-fold. The smaller size of the fragments may lead to improved pharmacological properties, such as better penetration of solid tissue. Effector functions of whole antibodies, such as complement binding, are removed. Fab, Fv, ScFv and dAb antibody fragments can all be expressed in and secreted from E. *coli,* thus allowing the facile production of large amounts of the said fragments.

Whole antibodies, and F(ab')₂ fragments are "bivalent". By "bivalent" we mean that the said antibodies and F(ab')₂ fragments have two antigen combining sites. In contrast, Fab, Fv, ScFv and dAb fragments are monovalent, having only one antigen combining sites.

The antibodies may be monoclonal or polyclonal. Suitable monoclonal antibodies may be prepared by known techniques, for example those disclosed in "Monoclonal Antibodies: A manual of techniques", H Zola (CRC Press, 1988) and in "Monoclonal Hybridoma Antibodies: Techniques and applications", J G R Hurrell (CRC Press, 1982), both of which are incorporated herein by reference.

Alternatively or additionally, the first binding agent comprises or consists of an antibody or an antigen-binding fragment thereof.

Alternatively or additionally, the antibody or antigen-binding fragment thereof is a recombinant antibody or antigen-binding fragment thereof.

Alternatively or additionally, the antibody or antigen-binding fragment thereof is selected from the group consisting of: scFv; Fab; a binding domain of an immunoglobulin molecule.

Alternatively or additionally, antibody or antigen-binding fragment is capable of competing for binding to a biomarker specified in Table A with an antibody for that biomarker defined in Table 8.

By "capable of competing" for binding to a biomarker specified in Table A with an antibody molecule as defined herein (or a variant, fusion or derivative of said antibody or antigen-binding fragment, or a fusion of a said variant or derivative thereof, which retains the binding specificity for the required biomarker) we mean or include that the tested antibody or antigen-binding fragment is capable of inhibiting or otherwise interfering, at least in part, with the binding of an antibody molecule as defined herein.

For example, the antibody or antigen-binding fragment may be capable of inhibiting the binding of an antibody molecule defined herein by at least 10%, for example at least 20%, 30%, 40%, 50%, 60%, 70%, 80%, 90%, 35% or even by 100%.

Competitive binding may be determined by methods well known to those skilled in the art, such as ELISA (as described herein) and/or SPR (as described in the accompanying Examples).

Alternatively or additionally, the antibody or antigen-binding fragment is an antibody defined in Table 8 or an antigen-binding fragment thereof, or a variant thereof.

Alternatively or additionally, the antibody the antibody or antigen-binding fragment comprises a VH and VL domain specified in Table 8, or a variant thereof.

By 'variants' of the antibody or antigen-binding fragment of the invention we include insertions, deletions and substitutions, either conservative or non-conservative. In particular we include variants of the sequence of the antibody or antigen-binding fragment where such variations do not substantially alter the activity of the antibody or antigen-binding fragment. In particular, we include variants of the antibody or antigen-binding fragment where such changes do not substantially alter the binding specificity for the respective biomarker specified in Table 8.

The polypeptide variant may have an amino acid sequence which has at least 70% identity with one or more of the amino acid sequences of the antibody or antigen-binding fragment of the invention as defined herein - for example, at least 75%, at least 80%, at least 90%, at least 95%, at least 96%, at least 97%, at least 98%, or at least 99% identity with one or more of the amino acid sequences of the antibody or antigen-binding fragment of the invention as defined herein.

The percent sequence identity between two polypeptides may be determined using suitable computer programs, for example the GAP program of the University of Wisconsin Genetic Computing Group and it will be appreciated that percent identity is calculated in relation to polypeptides whose sequences have been aligned optimally.

The alignment may alternatively be carried out using the Clustal W program (as described in Thompson et al., 1994, Nucl. Acid Res. 22:4673-4680, which is incorporated herein by reference).

The parameters used may be as follows:
- Fast pair-wise alignment parameters: K-tuple(word) size; 1, window size; 5, gap penalty; 3, number of top diagonals; 5. Scoring method: x percent.
- Multiple alignment parameters: gap open penalty; 10, gap extension penalty; 0.05.
- Scoring matrix: BLOSUM.

Alternatively, the BESTFIT program may be used to determine local sequence alignments.

The antibodies may share CDRs (e.g., 1, 2, 3, 4 ,5 or 6) CDRs with one or more of the antibodies defined in Table 8.

CDRs can be defined using any suitable method known in the art. Commonly used methods include Paratome (Kunik, Ashkenazi and Ofran, 2012, 'Paratome: an online tool for systematic identification of antigen-binding regions in antibodies based on sequence or structure' Nucl. Acids Res., 40:W521-W524; http://www.ofranlab.org/paratome/), Kabat (Wu and Kabat, 1970, 'An analysis of the sequences of the variable regions of Bence Jones proteins and myeloma light chains and their implications for antibody complementarity.' J. Exp. Med., 132:211-250), Chothia (Chothia and Lesk, 1987 'Canonical structures for the hypervariable regions of immunoglobulins' J. Mol. Biol., 196:901-917; Chothia et al., 1989 'Conformations of immunoglobulin hypervariable regions' Nature, 342:877-883) and IMGT (Lefranc et al., 2003 'IMGT unique numbering for immunoglobulin and T cell receptor variable domains and Ig superfamily V-like domains. Dev. Comp. Immunol., 27:55-77; Lefranc et al., 2005 'IMGT unique numbering for immunoglobulin and T cell receptor constant domains and Ig superfamily C-like domains' Dev. Comp. Immunol., 29:185-203; http://www.imgt.org). For example, the method used may be the IMGT method.

Alternatively or additionally, the first binding agent is immobilised on a surface (e.g., on a multiwell plate or array).

Alternatively or additionally, the one or more biomarkers in the test sample are labelled with a detectable moiety.

Alternatively or additionally, the one or more biomarkers in the control sample(s) are labelled with a detectable moiety.

By a "detectable moiety" we include the meaning that the moiety is one which may be detected and the relative amount and/or location of the moiety (for example, the location on an array) determined.

Suitable detectable moieties are well known in the art.

Thus, the detectable moiety may be a fluorescent and/or luminescent and/or chemiluminescent moiety which, when exposed to specific conditions, may be detected. For example, a fluorescent moiety may need to be exposed to radiation (*i.e.* light) at a specific wavelength and intensity to cause excitation of the fluorescent moiety, thereby enabling it to emit detectable fluorescence at a specific wavelength that may be detected.

Alternatively, the detectable moiety may be an enzyme which is capable of converting a (preferably undetectable) substrate into a detectable product that can be visualised and/or detected. Examples of suitable enzymes are discussed in more detail below in relation to, for example, ELISA assays.

Alternatively, the detectable moiety may be a radioactive atom which is useful in imaging. Suitable radioactive atoms include ^{99m}Tc and ¹²³I for scintigraphic studies. Other readily detectable moieties include, for example, spin labels for magnetic resonance imaging (MRI) such as ¹²³I again, ¹³¹I, ¹¹¹In, ¹⁹F, ¹³C, ¹⁵N, ¹⁷O, gadolinium, manganese or iron. Clearly, the agent to be detected (such as, for example, the one or more biomarkers in the test sample and/or control sample described herein and/or an antibody molecule for use in detecting a selected protein) must have sufficient of the appropriate atomic isotopes in order for the detectable moiety to be readily detectable.

The radio- or other labels may be incorporated into the agents of the invention (i.e. the proteins present in the samples of the methods of the invention and/or the binding agents of the invention) in known ways. For example, if the binding moiety is a polypeptide it may be biosynthesised or may be synthesised by chemical amino acid synthesis using suitable amino acid precursors involving, for example, fluorine-19 in place of hydrogen. Labels such as ^{99m}Tc, ¹²³I, ¹⁸⁶Rh, ¹⁸⁸Rh and ¹¹¹In can, for example, be attached *via* cysteine residues in the binding moiety. Yttrium-90 can be attached via a lysine residue. The IODOGEN method (Fraker et al (1978) Biochem. Biophys. Res. Comm. 80, 49-57) can be used to incorporate ¹²³I. Reference ("Monoclonal Antibodies in Immunoscintigraphy", J-F Chatal, CRC Press, 1989) describes other methods in detail. Methods for conjugating other detectable moieties (such as enzymatic, fluorescent, luminescent, chemiluminescent or radioactive moieties) to proteins are well known in the art.

Preferably, the one or more biomarkers in the control sample(s) are labelled with a detectable moiety. The detectable moiety may be selected from the group consisting of: a fluorescent moiety; a luminescent moiety; a chemiluminescent moiety; a radioactive moiety; an enzymatic moiety. However, it is preferred that the detectable moiety is biotin.

Alternatively or additionally, the detectable moiety is selected from the group consisting of: a fluorescent moiety; a luminescent moiety; a chemiluminescent moiety; a radioactive moiety; an enzymatic moiety.

Alternatively or additionally, the detectable moiety is biotin.

Alternatively or additionally, step (b), (d) and/or step (f) is performed using an assay comprising a second binding agent capable of binding to the one or more biomarkers, the second binding agent comprising a detectable moiety.

Alternatively or additionally, the second binding agent comprises or consists of an antibody or an antigen-binding fragment thereof.

Alternatively or additionally, the antibody or antigen-binding fragment thereof is a recombinant antibody or antigen-binding fragment thereof.

Alternatively or additionally, the antibody or antigen-binding fragment thereof is selected from the group consisting of: scFv; Fab; a binding domain of an immunoglobulin molecule.

Alternatively or additionally, the detectable moiety is selected from the group consisting of: a fluorescent moiety; a luminescent moiety; a chemiluminescent moiety; a radioactive moiety; an enzymatic moiety.

Alternatively or additionally, the detectable moiety is fluorescent moiety (for example an Alexa Fluor dye, e.g. Alexa647).

Alternatively or additionally, the detection method comprises or consists of an ELISA (Enzyme Linked Immunosorbent Assay).

Preferred assays for detecting serum or plasma proteins include enzyme linked immunosorbent assays (ELISA), radioimmunoassay (RIA), immunoradiometric assays (IRMA) and immunoenzymatic assays (IEMA), including sandwich assays using monoclonal and/or polyclonal antibodies. Exemplary sandwich assays are described by David et al in US Patent Nos. 4,376,110 and 4,486,530, hereby incorporated by reference. Antibody staining of cells on slides may be used in methods well known in cytology laboratory diagnostic tests, as well known to those skilled in the art.

Typically, the assay is an ELISA (Enzyme Linked Immunosorbent Assay) which typically involves the use of enzymes giving a coloured reaction product, usually in solid phase assays. Enzymes such as horseradish peroxidase and phosphatase have been widely employed. A way of amplifying the phosphatase reaction is to use NADP as a substrate to generate NAD which now acts as a coenzyme for a second enzyme system. Pyrophosphatase from *Escherichia coli* provides a good conjugate because the enzyme is not present in tissues, is stable and gives a good reaction colour. Chemi-luminescent systems based on enzymes such as luciferase can also be used.

ELISA methods are well known in the art, for example see The ELISA Guidebook (Methods in Molecular Biology), 2000, Crowther, Humana Press, ISBN-13: 978-0896037281 (the disclosures of which are incorporated by reference).

Conjugation with the vitamin biotin is frequently used since this can readily be detected by its reaction with enzyme-linked avidin or streptavidin to which it binds with great specificity and affinity.

Alternatively or additionally, step (b), (d) and/or step (f) is performed using an array.

Arrays *per se* are well known in the art. Typically they are formed of a linear or two-dimensional structure having spaced apart (*i.e.* discrete) regions ("spots"), each having a finite area, formed on the surface of a solid support. An array can also be a bead structure where each bead can be identified by a molecular code or colour code or identified in a continuous flow. Analysis can also be performed sequentially where the sample is passed over a series of spots each adsorbing the class of molecules from the solution. The solid support is typically glass or a polymer, the most commonly used polymers being cellulose, polyacrylamide, nylon, polystyrene, polyvinyl chloride or polypropylene. The solid supports may be in the form of tubes, beads, discs, silicon chips, microplates, polyvinylidene difluoride (PVDF) membrane, nitrocellulose membrane, nylon membrane, other porous membrane, non-porous membrane (e.g. plastic, polymer, perspex, silicon, amongst others), a plurality of polymeric pins, or a plurality of microtitre wells, or any other surface suitable for immobilising proteins, polynucleotides and other suitable molecules and/or conducting an immunoassay. The binding processes are well known in the art and generally consist of cross-linking covalently binding or physically adsorbing a protein molecule, polynucleotide or the like to the solid support. By using well-known techniques, such as contact or non-contact printing, masking or photolithography, the location of each spot can be defined. For reviews see Jenkins, R.E., Pennington, S.R. (2001, Proteomics, 2,13-29) and Lal et al (2002, Drug Discov Today 15;7(18 Suppl):S143-9).

Typically the array is a microarray. By "microarray" we include the meaning of an array of regions having a density of discrete regions of at least about 100/cm², and preferably at least about 1000/cm². The regions in a microarray have typical dimensions, e.g., diameters, in the range of between about 10-250 µm, and are separated from other regions in the array by about the same distance. The array may also be a macroarray or a nanoarray.

Once suitable binding molecules (discussed above) have been identified and isolated, the skilled person can manufacture an array using methods well known in the art of molecular biology.

Alternatively or additionally, the array is a bead-based array.

Alternatively or additionally, the array is a surface-based array.

Alternatively or additionally, the array is selected from the group consisting of: macroarray; microarray; nanoarray.

Alternatively or additionally, the method comprises:
(i) labelling biomarkers present in the sample with biotin;
(ii) contacting the biotin-labelled proteins with an array comprising a plurality of scFv immobilised at discrete locations on its surface, the scFv having specificity for one or more of the proteins in Table A;
(iii) contacting the immobilised scFv with a streptavidin conjugate comprising a fluorescent dye; and
(iv) detecting the presence of the dye at discrete locations on the array surface
wherein the expression of the dye on the array surface is indicative of the expression of a biomarker from Table III in the sample.

Alternatively or additionally, step (b), (d) and/or (f) comprises measuring the expression of a nucleic acid molecule encoding the one or more biomarkers.

Alternatively or additionally, the nucleic acid molecule is a cDNA molecule or an mRNA molecule.

Alternatively or additionally, the nucleic acid molecule is an mRNA molecule.

Alternatively or additionally, measuring the expression of the one or more biomarker(s) in step (b), (d) and/or (f) is performed using a method selected from the group consisting of Southern hybridisation, Northern hybridisation, polymerase chain reaction (PCR), reverse transcriptase PCR (RT-PCR), quantitative real-time PCR (qRT-PCR), nanoarray, microarray, macroarray, autoradiography and *in situ* hybridisation.

Alternatively or additionally, measuring the expression of the one or more biomarker(s) in step (b) is determined using a DNA microarray.

Alternatively or additionally, measuring the expression of the one or more biomarker(s) in step (b), (d) and/or (f) is performed using one or more binding moieties, each individually capable of binding selectively to a nucleic acid molecule encoding one of the biomarkers identified in Table A.

Alternatively or additionally, the one or more binding moieties each comprise or consist of a nucleic acid molecule.

Alternatively or additionally, the one or more binding moieties each comprise or consist of DNA, RNA, PNA, LNA, GNA, TNA or PMO.

Alternatively or additionally, the one or more binding moieties each comprise or consist of DNA.

Alternatively or additionally, the one or more binding moieties are 5 to 100 nucleotides in length.

Alternatively or additionally, the one or more nucleic acid molecules are 15 to 35 nucleotides in length.

Alternatively or additionally, the binding moiety comprises a detectable moiety.

Alternatively or additionally, the detectable moiety is selected from the group consisting of: a fluorescent moiety; a luminescent moiety; a chemiluminescent moiety; a radioactive moiety (for example, a radioactive atom); or an enzymatic moiety.

Alternatively or additionally, the detectable moiety comprises or consists of a radioactive atom.

Alternatively or additionally, the radioactive atom is selected from the group consisting of technetium-99m, iodine-123, iodine-125, iodine-131, indium-111, fluorine-19, carbon-13, nitrogen-15, oxygen-17, phosphorus-32, sulphur-35, deuterium, tritium, rhenium-186, rhenium-188 and yttrium-90.

Alternatively or additionally, the detectable moiety of the binding moiety is a fluorescent moiety.

Alternatively or additionally, the sample provided in step (a), (c) and/or (e) is selected from the group consisting of unfractionated blood, plasma, serum, tissue fluid, pancreatic tissue, milk, bile and urine.

Alternatively or additionally, the sample provided in step (a), (c) and/or (e) is selected from the group consisting of unfractionated blood, plasma and serum.

Alternatively or additionally, the sample provided in step (a), (c) and/or (e) is serum.

Alternatively or additionally, the predicative accuracy of the method, as determined by an ROC AUC value, is at least 0.50, for example at least 0.55, 0.60, 0.65, 0.70, 0.75, 0.80, 0.85, 0.90, 0.95, 0.96, 0.97, 0.98 or at least 0.99.

Alternatively or additionally, the predicative accuracy of the method, as determined by an ROC AUC value, is at least 0.70.

Typically, diagnosis is performed using a support vector machine (SVM), such as those available from http://cran.r-project.org/web/packages/e1071/index.html (e.g. e1071 1.5-24). However, any other suitable means may also be used.

Support vector machines (SVMs) are a set of related supervised learning methods used for classification and regression. Given a set of training examples, each marked as belonging to one of two categories, an SVM training algorithm builds a model that predicts whether a new example falls into one category or the other. Intuitively, an SVM model is a representation of the examples as points in space, mapped so that the examples of the separate categories are divided by a clear gap that is as wide as possible. New examples are then mapped into that same space and predicted to belong to a category based on which side of the gap they fall on.

More formally, a support vector machine constructs a hyperplane or set of hyperplanes in a high or infinite dimensional space, which can be used for classification, regression or other tasks. Intuitively, a good separation is achieved by the hyperplane that has the largest distance to the nearest training datapoints of any class (so-called functional margin), since in general the larger the margin the lower the generalization error of the classifier. For more information on SVMs, see for example, Burges, 1998, Data Mining and Knowledge Discovery, 2:121-167.

In an alternative or additional embodiment of the invention, the SVM is 'trained' prior to performing the methods of the invention using biomarker profiles from individuals with known disease status (for example, individuals known to have pancreatic cancer, individuals known to have acute inflammatory pancreatitis, individuals known to have chronic pancreatitis or individuals known to be healthy). By running such training samples, the SVM is able to learn what biomarker profiles are associated with pancreatic cancer. Once the training process is complete, the SVM is then able whether or not the biomarker sample tested is from an individual with pancreatic cancer.

However, this training procedure can be by-passed by pre-programming the SVM with the necessary training parameters. For example, diagnoses can be performed according to the known SVM parameters using the SVM algorithm detailed in Table 5, based on the measurement of any or all of the biomarkers listed in Table A.

It will be appreciated by skilled persons that suitable SVM parameters can be determined for any combination of the biomarkers listed in Table A by training an SVM machine with the appropriate selection of data (i.e. biomarker measurements from individuals with known pancreatic cancer status). Alternatively, the data of the Examples and figures may be used to determine a particular pancreatic cancer-associated disease state according to any other suitable statistical method known in the art.

Preferably, the method of the invention has an accuracy of at least 60%, for example 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% accuracy.

Preferably, the method of the invention has a sensitivity of at least 60%, for example 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% sensitivity.

Preferably, the method of the invention has a specificity of at least 60%, for example 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% specificity.

By "accuracy" we mean the proportion of correct outcomes of a method, by "sensitivity" we mean the proportion of all PaC positive sample that are correctly classified as positives, and by "specificity" we mean the proportion of all PaC negative samples that are correctly classified as negatives.

Signal intensities may be quantified using any suitable means known to the skilled person. Alternatively or additionally, signal intensities are quantified using the ScanArray Express software version 4.0. Signal intensity data may be normalised (i.e., to adjust technical variation). Normalisation may be performed using any suitable method known to the skilled person. Alternatively or additionally, data is normalised using the empirical Bayes algorithm ComBat (Johnson *et al*., 2007).

Alternatively or additionally, the individual(s) being tested is genotypically Lewis a-b-.

The individual(s) tested may be of any ethnicity. Alternatively or additionally, the individual(s) tested are Caucasian and/or Chinese (e.g., Han ethnicity).

Alternatively or additionally, the sample(s) provided in step (a), (c) and/or (e) are provided before treatment of the pancreatic cancer (e.g., resection, chemotherapy, radiotherapy).

Alternatively or additionally, the individual(s) being tested suffers from one or more condition selected from the group consisting of chronic pancreatitis, hereditary pancreatic ductal adenocarcinoma and Peutz-Jeghers syndrome.

In an alternative or additional embodiment the pancreatic cancer is selected from the group consisting of adenocarcinoma, adenosquamous carcinoma, signet ring cell carcinoma, hepatoid carcinoma, colloid carcinoma, undifferentiated carcinoma, and undifferentiated carcinomas with osteoclast-like giant cells. Preferably, the pancreatic cancer is a pancreatic adenocarcinoma. More preferably, the pancreatic cancer is pancreatic ductal adenocarcinoma, also known as exocrine pancreatic cancer.

Alternatively or additionally, the method comprises the active step of determining the presence or absence of the/a pancreatic cancer-associated disease state of the invention.

Alternatively or additionally, in the event that the individual is diagnosed with pancreatic cancer, the method comprises the step of:
(g) providing the individual with pancreatic cancer therapy.

Thus, a related aspect of the invention provides a method of treatment of an individual with a pancreatic cancer comprising the following steps:
(a) diagnosing an individual as having a pancreatic cancer using a method according to the first aspect of the invention; and
(b) treating the individual so diagnosed with a pancreatic cancer therapy (for example, see Thota et al., 2014, Oncology 28(1):70-4, the disclosures of which are incorporated herein by reference).

Alternatively or additionally, the pancreatic cancer therapy is selected from the group consisting of surgery, chemotherapy, immunotherapy, chemoimmunotherapy and thermochemotherapy.

In an alternative or additional embodiment the pancreatic cancer therapy is selected from the group consisting of surgery, chemotherapy, immunotherapy, chemoimmunotherapy and thermochemotherapy (e.g., AC chemotherapy; Capecitabine and docetaxel chemotherapy (Taxotere ®); CMF chemotherapy; Cyclophosphamide; EC chemotherapy; ECF chemotherapy; E-CMF chemotherapy (Epi-CMF); Eribulin (Halaven®); FEC chemotherapy; FEC-T chemotherapy; Fluorouracil (5FU); GemCarbo chemotherapy; Gemcitabine (Gemzar ®); Gemcitabine and cisplatin chemotherapy (GemCis or GemCisplat); GemTaxol chemotherapy; Idarubicin (Zavedos ®); Liposomal doxorubicin (DaunoXome ®); Mitomycin (Mitomycin C Kyowa ®); Mitoxantrone; MM chemotherapy; MMM chemotherapy; Paclitaxel (Taxol ®); TAC chemotherapy; Taxotere and cyclophosphamide (TC) chemotherapy; Vinblastine (Velbe ®); Vincristine (Oncovin ®); Vindesine (Eldisine ®); and Vinorelbine (Navelbine ®)).

Accordingly, the present invention comprises an antineoplastic agent for use in treating pancreatic cancer wherein the dosage regime is determined based on the results of the method of the first aspect of the invention.

The present invention comprises the use of an antineoplastic agent in treating pancreatic cancer wherein the dosage regime is determined based on the results of the method of the first aspect of the invention.

The present invention comprises the use of an antineoplastic agent in the manufacture of a medicament for treating pancreatic cancer wherein the dosage regime is determined based on the results of the method of the first aspect of the invention.

The present invention comprises a method of treating pancreatic cancer comprising providing a sufficient amount of an antineoplastic agent wherein the amount of antineoplastic agent sufficient to treat the pancreatic cancer is determined based on the results of the method of the first aspect of the invention.

In one embodiment, the antineoplastic agent comprises or consists of an alkylating agent (ATC code L01a), an antimetabolite (ATC code L01b), a plant alkaloid or other natural product (ATC code L01c), a cytotoxic antibiotic or a related substance (ATC code L01d), or another antineoplastic agents (ATC code L01x).

Hence, in one embodiment the antineoplastic agent comprises or consists of an alkylating agent selected from the group consisting of a nitrogen mustard analogue (for example cyclophosphamide, chlorambucil, melphalan, chlormethine, ifosfamide, trofosfamide, prednimustine or bendamustine) an alkyl sulfonate (for example busulfan, treosulfan, or mannosulfan) an ethylene imine (for example thiotepa, triaziquone or carboquone) a nitrosourea (for example carmustine, lomustine, semustine, streptozocin, fotemustine, nimustine or ranimustine) an epoxides (for example etoglucid) or another alkylating agent (ATC code L01ax, for example mitobronitol, pipobroman, temozolomide or dacarbazine).

In a another embodiment the antineoplastic agent comprises or consists of an antimetabolite selected from the group consisting of a folic acid analogue (for example methotrexate, raltitrexed, pemetrexed or pralatrexate), a purine analogue (for example mercaptopurine, tioguanine, cladribine, fludarabine, clofarabine or nelarabine) or a pyrimidine analogue (for example cytarabine, fluorouracil (5-FU), tegafur, carmofur, gemcitabine, capecitabine, azacitidine or decitabine).

In a still further embodiment the antineoplastic agent comprises or consists of a plant alkaloid or other natural product selected from the group consisting of a vinca alkaloid or a vinca alkaloid analogue (for example vinblastine, vincristine, vindesine, vinorelbine or vinflunine), a podophyllotoxin derivative (for example etoposide or teniposide) a colchicine derivative (for example demecolcine), a taxane (for example paclitaxel, docetaxel or paclitaxel poliglumex) or another plant alkaloids or natural product (ATC code L01cx, for example trabectedin).

In one embodiment the antineoplastic agent comprises or consists of a cytotoxic antibiotic or related substance selected from the group consisting of an actinomycine (for example dactinomycin), an anthracycline or related substance (for example doxorubicin, daunorubicin, epirubicin, aclarubicin, zorubicin, idarubicin, mitoxantrone, pirarubicin, valrubicin, amrubicin or pixantrone) or another (ATC code L01dc, for example bleomycin, plicamycin, mitomycin or ixabepilone).

In a further embodiment the antineoplastic agent comprises or consists of another antineoplastic agent selected from the group consisting of a platinum compound (for example cisplatin, carboplatin, oxaliplatin, satraplatin or polyplatillen) a methylhydrazine (for example procarbazine) a monoclonal antibody (for example edrecolomab, rituximab, trastuzumab, alemtuzumab, gemtuzumab, cetuximab, bevacizumab, panitumumab, catumaxomab or ofatumumab) a sensitizer used in photodynamic/radiation therapy (for example porfimer sodium, methyl aminolevulinate, aminolevulinic acid, temoporfin or efaproxiral) or a protein kinase inhibitor (for example imatinib, gefitinib, erlotinib, sunitinib, sorafenib, dasatinib, lapatinib, nilotinib, temsirolimus, everolimus, pazopanib, vandetanib, afatinib, masitinib or toceranib).

In a still further embodiment the antineoplastic agent comprises or consists of another neoplastic agent selected from the group consisting of amsacrine, asparaginase, altretamine, hydroxycarbamide, lonidamine, pentostatin, miltefosine, masoprocol, estramustine, tretinoin, mitoguazone, topotecan, tiazofurine, irinotecan (camptosar), alitretinoin, mitotane, pegaspargase, bexarotene, arsenic trioxide, denileukin diftitox, bortezomib, celecoxib, anagrelide, oblimersen, sitimagene ceradenovec, vorinostat, romidepsin, omacetaxine mepesuccinate, eribulin or folinic acid.

In one embodiment the antineoplastic agent comprises or consists of a combination of one or more antineoplastic agent, for example, one or more antineoplastic agent defined herein. One example of a combination therapy used in the treatment of pancreatic cancer is FOLFIRINOX which is made up of the following four drugs:
- FOL - folinic acid (leucovorin);
- F - fluorouracil (5-FU);
- IRIN - irinotecan (Camptosar); and
- OX - oxaliplatin (Eloxatin).

Accordingly, a second aspect of the invention provides an array for diagnosing or determining a pancreatic cancer-associated disease state in an individual comprising one or more binding agent as defined in the first aspect of the invention.

Alternatively or additionally, the one or more binding agents is capable of binding to all of the proteins defined in Table A.

A third aspect of the invention provides use of one or more biomarkers selected from the group defined in Table A as a biomarker for determining a pancreatic cancer associated disease states in an individual.

Alternatively or additionally, all of the proteins defined in Table A is used as a marker for determining a pancreatic cancer associated disease state in an individual.

A fourth aspect of the invention provides the use of one or more binding moiety as defined in the first aspect of the invention for determining a pancreatic cancer associated disease state in an individual.

Alternatively or additionally, biomarkers for all of the proteins defined in Table A are used.

A fifth aspect of the invention provides a kit for diagnosing or determining a pancreatic cancer-associated disease state in an individual comprising:
(a) one or more binding agent as defined in the first aspect of the invention or an array according to the second aspect of the invention;
(b) instructions for performing the method as defined above (e.g., in the first aspect of the invention).

A sixth aspect of the invention provides a method of treating pancreatic cancer in an individual comprising the steps of:
(a) determining a pancreatic cancer associated disease state according to the method defined in any the first aspect of the invention; and
(b) providing the individual with pancreatic cancer therapy.

Alternatively or additionally, the pancreatic cancer therapy is selected from the group consisting of surgery (e.g., resection), chemotherapy, immunotherapy, chemoimmunotherapy and thermochemotherapy.

An eighth aspect of the invention provides a computer program for operating the methods the invention, for example, for interpreting the expression data of step (c) (and subsequent expression measurement steps) and thereby diagnosing or determining a pancreatic cancer-associated disease state. The computer program may be a programmed SVM. The computer program may be recorded on a suitable computer-readable carrier known to persons skilled in the art. Suitable computer-readable-carriers may include compact discs (including CD-ROMs, DVDs, Blue Rays and the like), floppy discs, flash memory drives, ROM or hard disc drives. The computer program may be installed on a computer suitable for executing the computer program.

Preferred, non-limiting examples which embody certain aspects of the invention will now be described, with reference to the following tables and figures:

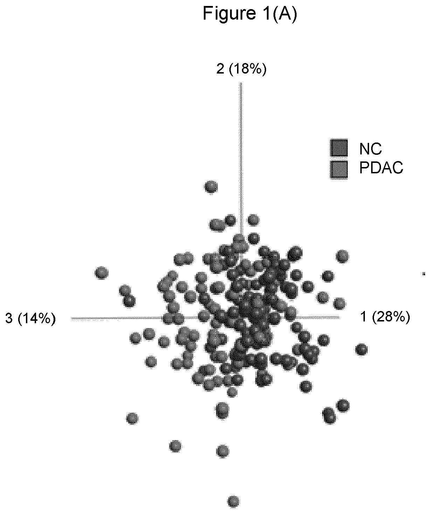

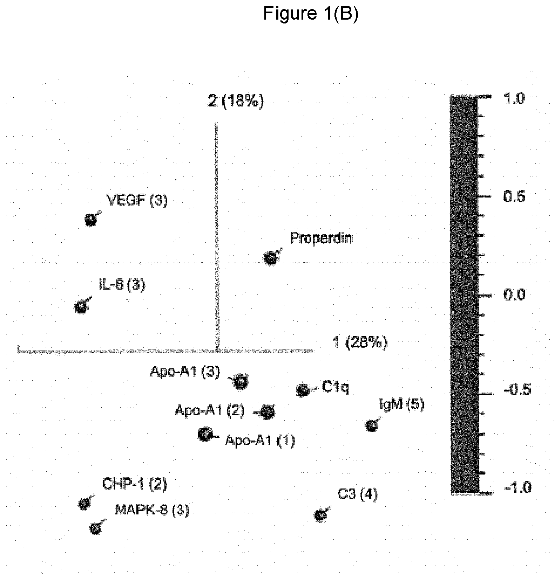

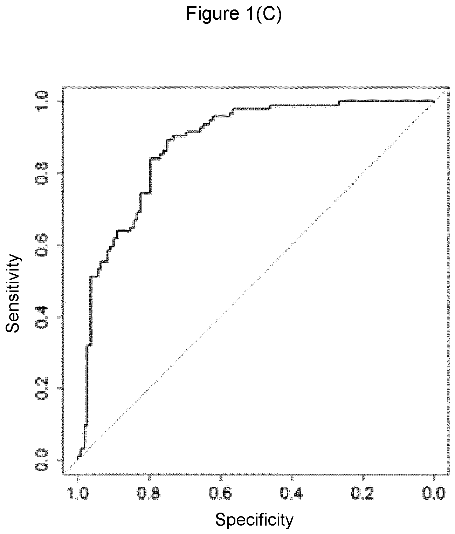

**Figure 1****.** *Discrimination of pancreatic cancer (PDAC) vs. normal controls (NC).*
   (A) Principal component analysis (PCA) of PDAC (grey) and NC (black). The data was filtered to q<0.1 using ANOVA; (B) Relative protein levels corresponding to the 11 antibodies that remained after filtering, in a PCA plot synchronized to the one in A). Grey = up-regulated levels, Black =down-regulated levels in PDAC vs. NC; (C) ROC-curve with AUC of 0.88 from support vector machine analysis (SVM) with leave-one-out (LOO) cross-validation of PDAC vs. NC based on unfiltered data (using unfiltered data from all antibodies).

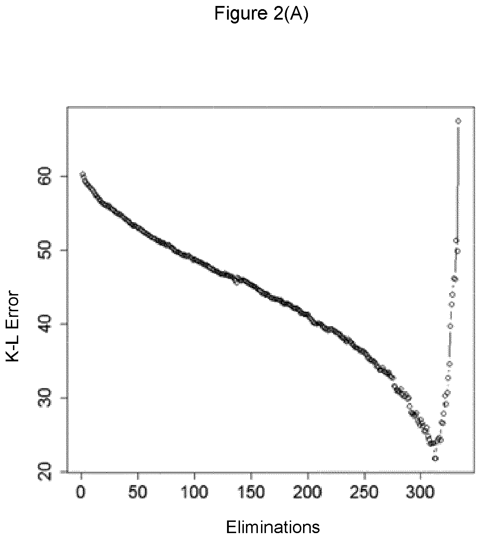

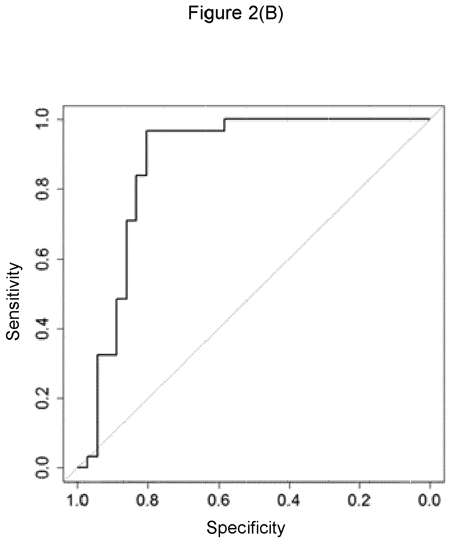

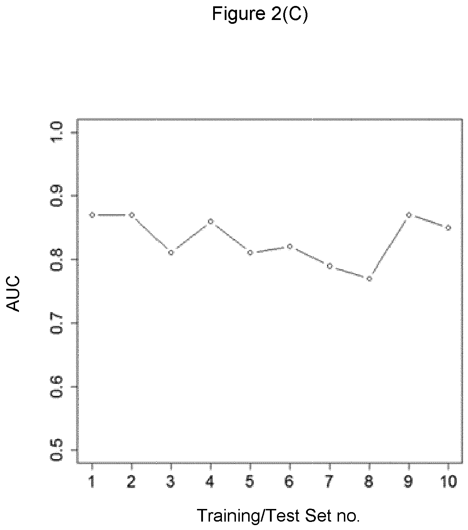

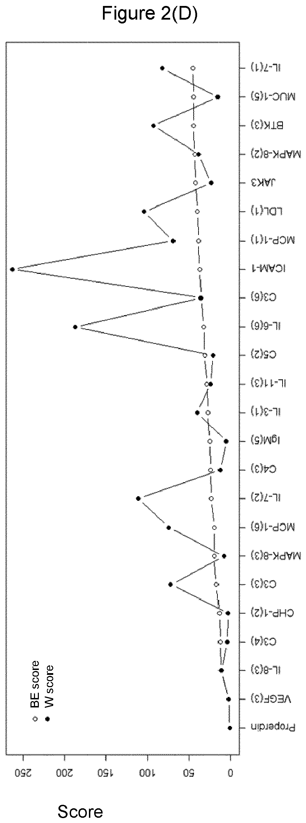

**Figure 2****.** *Identification of plasma protein signatures for PDAC.*
   A training set and a test set was generated by randomized selection of 2/3 of samples from each group (PDAC and NC) to the training set, and the remaining 1/3 of samples to the test set. The training set was used to define a condensed signature for discriminating PDAC from NC. (A) Filtering of variables was conducted by a SVM-based stepwise backward elimination of the antibodies in the training set. In each iterative step, the Kullback-Liebler (K-L) error of the classification was determined and plotted. The antibodies that remained in the elimination process when the classification error reached its minimum value were used as a unique signature for constructing a new model in the training set; (B) ROC-curve resulting from the signature model from the training set, "frozen" and directly applied onto the previously unseen test set samples; (C) The procedure was repeated to a total of 10 times, in 10 different sets of randomly created training and test sets. The area under the ROC-curve (AUC values) generated by the frozen biomarker signature models in each corresponding test set were plotted; (D) The antibody score derived from the overall ranking in the backward elimination (BE) process (open circles) was compared to the score based on the T-test (W) test ranking (filled circles).
**Figure 3****.** *Discrimination of PDAC stages vs. controls.*
   A) AUC-values from SVM analysis using unfiltered data (all antibodies), comparing NC to patients grouped according to their PDAC stage. B) Antibodies with Wilcoxon p<0.05 in one or more PDAC stages vs NC. "UP" = up-regulated, "DOWN" = down-regulated in PDAC vs NC, white = no significant difference.
**Figure 4****.** *Differentiation of primary tumor location and comparison to a previous study in serum (Gerdtsson, 2015).*
   Serum from 156 PDAC patients collected at 5 different sites in Spain for the PANKRAS II [Parker *et al*., 2011, Porta *et al*., 1999] study (Gerdtsson et al., 2016, Mol Oncol.10(8):1305-16).
   "UP" = up-regulated, "DOWN" = down-regulated in Head vs Body/Tail tumors, N/A = antibody not included in study.

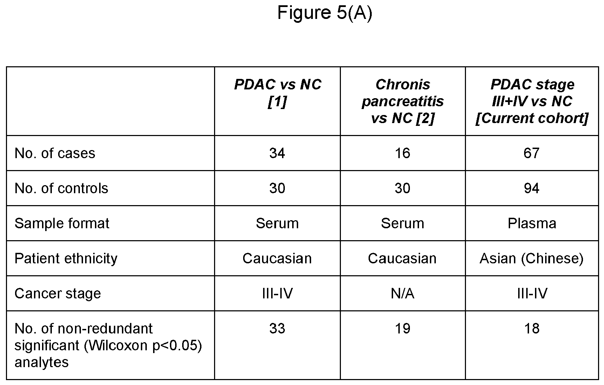

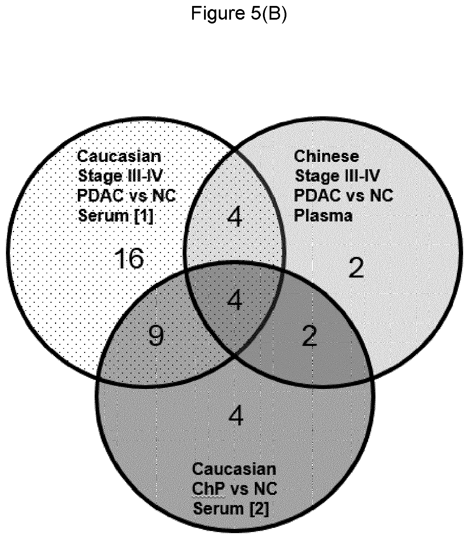

**Figure 5****.** *Comparison to previous cohorts.*
   A) Details of the three studies being compared. B) Venn diagram derived from overlapping markers analyzed in a (i) Caucasian cohort of Stage III-IV PDAC vs. controls , (ii) Chronic pancreatitis patients vs. controls, and the current (iii) Chinese cohort of Stage PDAC vs. controls. For comparative reasons, only the stage III+IV samples were included in the current cohort. Numbers denotes significantly (Wilcoxon p<0.05) differentially expressed markers. [1] Wingren C, Sandstrom A, Segersvard R, Carlsson A, Andersson R, Lohr M, Borrebaeck CA. Identification of serum biomarker signatures associated with pancreatic cancer. Cancer Res. 2012;72: 2481-90; [2] Serum proteome profiling of pancreatitis using recombinant antibody microarrays reveals disease-associated biomarker signatures. Proteomics Clin Appl. 2012;6: 486-96.

### EXAMPLE

### Abstract

In this study, a recombinant antibody microarray platform was used to analyse 213 plasma samples from pancreatic cancer patients and normal control individuals. The cohort was stratified according to disease stage, i.e. resectable disease (stage I/II), locally advanced (stage III) and metastatic disease (stage IV). SVM analysis showed that all PDAC stages could be discriminated from controls. This is the first time patients with stage I/II PDAC tumours could be discriminated from controls with high accuracy based on a plasma protein signature, which indicates a possibility for early diagnosis and an increased rate of surgically resectable tumors.

### Introduction

Pancreatic ductal adenocarcinoma (PDAC) is one of the deadliest cancers with a 5-year survival rate of 3-4%. A key driver behind this poor prognosis is the current inability to diagnose patients at an early stage. Data supports that it takes more than five years from tumor initiation until the acquisition of metastatic ability (Yachida et al., 2010), which clearly demonstrates a window of opportunity for early detection if accurate markers were available. At the time of diagnosis, patients have often developed late-stage disease, and only approximately 15% of the patients have resectable tumors (Conlon et al., 1996; Sohn et al., 2000). The 5-year survival of these patients, displaying large resected tumors, is only 10-20% (Conlon et al., 1996; Sohn et al., 2000). However, the 5-year survival increases to 30-60% if tumors ≤20 mm (Stage I-II) can be resected (Furukawa et al., 1996; Shimizu et al., 2005). The late diagnosis (Stage III-IV) is due to unspecific clinical symptoms in combination with the lack of markers for early diagnosis, something which is addressed in this study. Interestingly, studies suggest that pancreatic tumors could be resectable as early as six months prior to clinical diagnosis at an asymptomatic stage (Gangi et al., 2004; Pelaez-Luna et al., 2007).

The so far most evaluated marker for PDAC, CA19-9, suffers from poor specificity, with elevated levels in several other indications, as well as a complete absence in patients that are genotypically Lewis a-b- (5% of the population). Consequently, the use of CA19-9 for pancreatic cancer screening is not recommended (Locker et al., 2006). Today, no other single biomarker has been shown to accurately diagnose PDAC, although recent discovery studies have demonstrated that both exosomes and nucleosomes contain information associated with pancreatic cancer (Bauden et al., 2015; Melo et al., 2015). However, the field of cancer diagnostics is today moving towards panels of markers, since this yields increased sensitivity and specificity (Brand et al., 2011; Bunger et al., 2011).

Inflammation seems to be a critical component of tumor progression (Coussens and Werb, 2002) and the immunoregulatory plasma proteome may consequently be a source of potential cancer biomarkers.

Previous studies have also demonstrated that an increased number (n=20-25) of immunoregulatory proteins will yield highly disease-specific signatures, reflecting a systemic response to disease (Carlsson et al., 2011b; Ingvarsson et al., 2008; Wingren et al., 2012). However, analysis of the immunoregulatory proteome is associated with several challenges, such as, (i) plasma proteins display a vast dynamic concentration range; (ii) cancer markers are more likely to be found among the most low-abundant proteins (Haab et al., 2005; Surinova et al., 2011); (iii) disease-associated changes in plasma levels of low-abundant markers is expected to be small, requiring a significant number of samples for adequate statistics (Alonzo et al., 2002).

To meet these challenges, we have in the present study analyzed 213 plasma samples from Chinese patients with pancreatic cancer Stage I-IV and normal controls, using a sensitive antibody microarray platform. The aim was to identify stage-associated PDAC markers by comparing control samples to stage I-IV and the results support the concept that the information content in a simple blood sample is enough to find even the earlier disease stages. Consequently, enables early diagnosis of PDAC, particularly for the benefit of patients at high risk, such as chronic pancreatitis, hereditary PDAC, and Peutz-Jeghers syndrome patients.

### Material and Methods

### Plasma Samples

This retrospective study was approved by the Ethics Committee of Tianjin Medical University Cancer Institute and Hospital (TMUCIH). After informed consent, blood was collected at TMUCIH, plasma was isolated and stored at -80°C. A total of 213 plasma samples, collected from Jan-01 2012 to Dec-13 2013, were used (Table 1). The enrolled PDAC patients (n=118) were all Chinese Han ethnicity and treated at TMUCIH. None of the patients had received chemotherapy or radiotherapy at the time of blood draw. All PDAC samples were cytology confirmed by experienced pathologists. Patients were diagnosed with pancreatic ductal adenocarcinoma with the following exceptions: Malignant serous cystadenoma (n=1), pancreatic sarcoma (n=2), tubular papillary pancreatic adenocarcinoma (n=1). Five patients were diagnosed with PDAC with liver metastasis. Data on tumor stage and size at diagnosis, and tumor location within the pancreas were based on clinical pathology. Staging was performed according to the American Cancer Society's guidelines (Table 1) and the extent of resection was classified as R0. Normal control (NC) samples (n=95) were collected from healthy inhabitants of Tianjin at their routine physical examination at TMUCIH, and were genetically unrelated to the PDAC patients (Table 1). Sample IDs were recoded and randomized at labelling, and sample annotation and clinical data was blinded to the operator at all downstream experimental procedures. All samples were labelled at one single occasion, using a previously optimized protocol (Wingren et al., 2012).

### Generation of antibody microarrays

The antibody microarrays contained 350 human recombinant scFv antibodies, selected and generated from in-house designed phage display antibody libraries, produced in *E. coli* as previously been described (Pauly et al., 2014) and printed onto slides in 14 arrays/slide and 3 replicate spots/array. All slides used for this study were printed at a single occasion, shipped to TMUCIH in China, and used for analysis within 4 weeks after printing.

### Antibody microarray analysis

Ten slides (140 individual subarrays) were processed per day with randomized sample order. Briefly, arrays were blocked with PBSMT, washed with PBST, and incubated with biotinylated plasma samples for 2h at RT. Unbound proteins were washed off, and bound proteins were detected using 1 µg/mL Alexa Fluor647-Streptavidin (1h at RT). Excess reagent was washed off, and slides were dried and immediately scanned in a LuxScan 10K Microarray scanner (CapitalBio) at 10 µm resolution using the 635 nm and the 532 nm excitation lasers.

### Data acquisition, quality control and pre-processing

Signal intensities were quantified by two trained analysts (ASG and MN), blinded to patient ID and clinical data, using the ScanArray Express software version 4.0 (PerkinElmer Life and Analytical Sciences) with the fixed-circle option. For each microarray a grid was positioned, using the Alexa Fluor555 signals from microarray printing, and used to quantify the Alexa Fluor647 signal corresponding to the relative level of bound protein. Eleven samples (10 PDAC, 1 NC) were not quantified due to poor quality images resulting from of high background and/or low overall signals. For quantified arrays, the spot saturation, mean intensity and signal-to-noise ratio of each spot were evaluated. Fourteen antibodies were excluded because (i) the median signal intensity was below the cut-off limit, defined as the background (average PBS signal) +2 standard deviations (n=8), (ii) saturated signal in the lowest scanner intensity setting in more than 50% of samples (n=1), and (iii) inadequate antibody printing (n=5). Based on the remaining 202 samples and 336 antibodies, a dataset was assembled using the mean spot intensity after local background subtraction. Each data point represented an average of the 3 replicate spots, unless any replicate CV exceeded 15% from the mean value, in which case it was discarded and the average of the 2 remaining replicates was used instead. The average CV of replicates was 7.9% (±4.1%). Applying a cut-off CV of 15%, 79% of data values were calculated from all 3 replicates and the remaining 21% from 2 replicates. Furthermore, patients with jaundice (n=27) were compared to patients without jaundice (n=81), to analyze whether the bilirubin level was a confounding factor in the antibody microarray analysis. Similarly, a gender-adjusted dataset was generated to assess whether gender was a confounding factor. The logged data was normalized, using the empirical Bayes algorithm ComBat (Johnson et al., 2007) for adjusting technical variation, followed by a linear scaling of data from each array to adjust for variations in sample background level. The scaling factor was based on the 20% of antibodies with the lowest standard deviation across all samples and was calculated by dividing the intensity sum of these antibodies on each array with the average sum across all arrays (Carlsson et al., 2008; Ingvarsson et al., 2008).

### Data analysis

The sample and variable distribution was analyzed and visualized, using a principal component analysis based program (Qlucore, Lund, Sweden). ANOVA was applied for an initial filtering of data. The performance of individual markers was evaluated using Wilcoxon or Student's t-test, Benjamini Hochberg procedure for false discovery rate control, and fold changes. Separation of different subgroups was assessed using support vector machine (SVM), applying a linear kernel with the cost of constraints set to 1. Models for discriminating two groups were created, using a leave-one-out cross validation procedure.

To minimize over-interpretation and to demonstrate robustness of the data set, it was randomly divided into training and test sets, and the SVM-based backward elimination algorithm was applied in the training sets. Consequently, biomarker signatures were generated in training sets of the data, consisting of 2/3 of the total samples from each subgroup, and evaluated in a test sets containing the remaining 1/3 of samples. In training sets, filtering was performed using an SVM-based Backward Elimination algorithm, as previously described (Carlsson et al., 2011a) and models based on the resulting antibody signatures were tested in the corresponding test sets. Ten different pairs of training and test sets were used for this purpose, resulting in a consensus list in which each antibody was given an elimination score corresponding to its median order of elimination in the 10 training sets. The performance was assessed using receiver operating characteristics (ROC) curves and reported as area under the curve (AUC) values. The sensitivity and specificity, positive and negative predictive values of each signature in its respective test set were noted for the SVM decision value threshold corresponding to the maximum sum of sensitivity and specificity.

### Results

### Discrimination between cases and controls and Identification of plasma protein signatures associated with pancreatic cancer

Initially, we show that PDAC cases can be discriminated from normal controls, using PCA, q-value filtration and SVM analysis with leave-on-out cross validation. Principal component analysis revealed a moderate separation of PDAC and NC samples (Figure 1A) and differential analysis with q-value cut-off of 0.1 resulted in 11 antibodies displaying significant different expression levels in PDAC vs. NC (Figure 1B). Three of these were targeting Apolipoprotein A1 (Apo-A1) and showed decreased proteins levels in PDAC compared to NC. Properdin, C1q, C3, IgM and IL-8 also showed reduced levels in PDAC, while VEGF, MAPK-8 and CHP-1 were elevated in the cancer group. Furthermore, SVM with leave-one-out cross validation using data from all 336 antibodies demonstrated that PDAC and NC were separated with an AUC-value of 0.88 (p-value = 6.4x10⁻²¹, Figure 1C). Of note, patients with jaundice could not be significantly discriminated from patients without jaundice, demonstrating that hyperbilirubinemia was not a confounding factor (data not shown). A comparison of significant (Wilcoxon p<0.05) markers in the gender-adjusted dataset showed that neither gender was a confounding factor (Table 4).

The high level of differentiation between PDAC and NC using unfiltered data (AUC 0.88) motivated in-depth data filtering for identifying a condensed PDAC-associated protein signature. To avoid over-fitting the model to the data, samples were first separated into training sets for generating antibody signatures models which were then evaluated on separate test sets. In the training sets, antibodies were filtered using SVM-based backward elimination and the Kullback-Leibler (K-L) error in the classification was plotted against the number of eliminated antibodies. Figure 2A illustrates the elimination process in the first training set, in which a distinct minimum of the error was observed after 313 iterations, corresponding to a final 24 antibody signature Based on this signature, an SVM model was constructed in the training set and evaluated in a separate test set, where it generated an AUC-value of 0.87 (Figure 2B). To test the robustness of the data set this elimination procedure was repeated in a total of 10 different, randomly generated pairs of training and test sets, which in term generated 10 signatures identified for optimal separation of cancer vs. controls. The length of signatures ranged from 17-29 antibodies (median 23.5). The AUC-values in the test sets ranged from 0.77-0.87, with an average of 0.83 (Figure 2C). The sensitivity and specificity had average values of 0.77 (ranging 0.56-0.94) and 0.86 (ranging 0.55-0.97), respectively, with the corresponding average positive predictive value of 0.86 (ranging 0.71-0.97) and average negative predictive value of 0.77 (ranging 0.64-0.89).

Each antibody was scored based on the reverse order of elimination, with number 1 being the last antibody to be eliminated, and ranked in order of their median elimination score from the 10 sequential elimination rounds. Table 2 lists the 25 highest ranked antibodies, with their p- and q-values, the p-value ranking, and the fold change for PDAC vs. NC. The top 2 antibodies Properdin and VEGF with median elimination scores of 1 and 2, respectively, were the last eliminated antibodies in 9/10 (Properdin), and the second last eliminated in 8/10 (VEGF) training sets. The top 25 ranked antibodies together represented 20 different specificities.

The backward elimination procedure was designed to identify the optimal combination of antibodies, not taking into consideration one-dimensional separation of data based on individual antibodies, and the consensus signature presented in Table 2 is based solely on the backward elimination ranking. Of note, the top two antibodies were also the two highest ranked on basis of p- and q-values. In fact, the five highest ranked antibodies all displayed highly significant p- and q-values for PDAC vs. NC (p<4.47E-06 and q<5.00E-04). The backward elimination rank (BE-score) and the t-test rank (W-score) for the consensus signature antibodies were plotted together in Figure 2D. The W-score starts to deviate from the BE-score after the top five antibodies, and then lost any correlation. Thus, the five highest ranked antibodies (Properdin, VEGF, IL-8, C3, and CHP-1) make out a highly stable core of the consensus signature, as indicated by both the backward elimination procedure and the univariate differential expression analysis. However, the current data, in consistency with previous datasets analyzed with similar approaches, shows that the signature core needs to be supplemented by orthogonal markers to reach a clinically relevant level of accuracy in terms of sensitivity and particularly, specificity, for discriminating PDAC vs NC.

### Discrimination between stage I-IV of pancreatic cancer

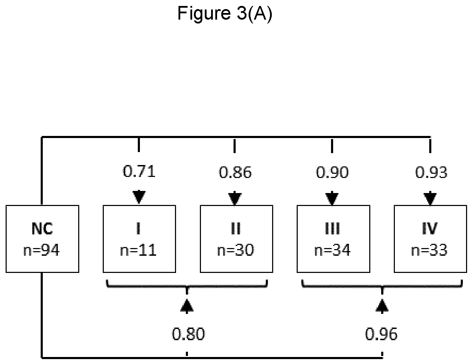

Discrimination between different disease stages is of high interest since this is associated with the ability to diagnose pancreatic cancer in its non-invasive stage. In an attempt to assess the significance of identified markers for early diagnosis, the PDAC samples were stratified according to disease stage. SVM with leave-one-out cross validation showed that all PDAC stages were separated from NC and that classification accuracy increased with disease progression, with AUC-values of 0.71, 0.86, 0.90 and 0.93 for discriminating NC from stage I, II, III, and IV, respectively (Figure 3a). The subgrouping into stages resulted in smaller sample numbers, ranging from n=11 for stage I to n=34 for stage III patients. For increased statistical power, a grouping into early confined disease (stage I/II) and late invasive disease (stage III/IV) was also performed. These groups were discriminated from NC with AUC values of 0.80 (early stage) and 0.96 (late stage).

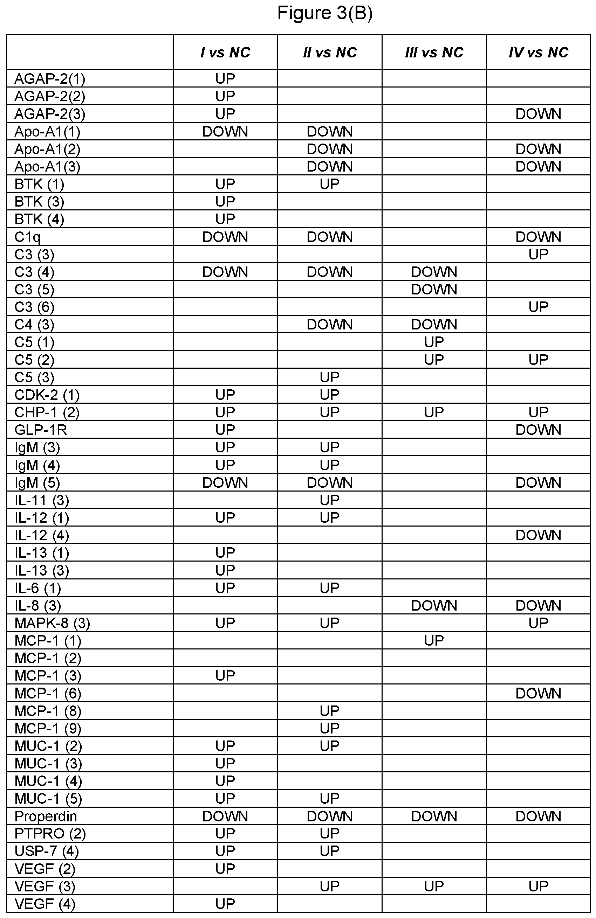

Figure 3b shows all antibodies displaying significant (Wilcoxon p<0.05) differential protein levels in at least one of the stage groups when compared to NC. Among the five described core candidate markers (Properdin, VEGF, IL-8, C3, and CHP-1), Properdin was down-regulated in all stages, CHP-1 was up-regulated in all stages, and IL-8 was down-regulated in Stage III/IV disease only.

It is noteworthy that in our previous study on serum markers in Stage III/IV Caucasian PDAC patients, C5 was ranked as the most prominent marker in a backward elimination filtering analysis, while the current study ranked the same antibody as number 14 (Table 2). However, when stage-specific analysis was performed it was evident that C5 was elevated only in late stage disease, which is coherent with the former study (Wingren et al., 2012). Of note, the stage-specific analysis pointed out several early marker candidate proteins, e.g. elevated levels of BKT, CDK2, MAPK-8, AGAP-2, IL-13, IL-6, PTPRO, USP-7, MUC-1, and reduced levels of Apo-A1 and C1q measurable already at stage I/II disease.

### Markers associated with tumor location

The samples were also grouped by the primary tumor location in the pancreas, and plasma from tumors located in the head of pancreas was compared to those located in the body and/or tail of pancreas. Samples with tumors at other locations (neck=4, neck+body=1, head+tail=1) were excluded from this analysis. Applying a cut-off of Wilcoxon p<0.05, 37 antibodies showed significantly different intensity levels in Head vs. Body/Tail (Figure 4). The AUC for Head (n=63) vs. Body/Tail (n=39) localized tumors was 0.64 (p=5.4e-3). Although the groups were not distinctly separated in the SVM analysis, the 37 significant antibodies overlapped remarkably well with the antibodies identified in an earlier study (Figure 4), despite differences in regards to sample format (serum/plasma), ethnicity (Caucasian/Asian), technical processes (assay protocol and instrumentation) and data processing (normalization procedures) (Gerdtsson, 2015). The only protein not correlating with the former study was C3, which was found to be elevated in plasma but reduced in serum in Head vs. Body/Tail localized tumors. Of note, the proteins that discriminated between Head and Body/Tail localized tumors (Figure 4) were also distinctly different from the protein signatures for PDAC vs. NC (Table 2).

### Correlation between markers derived from Caucasian and Chinese populations

We next assessed the concordance of the plasma protein signature in the Chinese pancreatic cancer subjects with the serum protein signature previously identified in a late stage PDAC Caucasian cohort, analyzed in relation to healthy controls as well as to patients with pancreatitis (Wingren et al., 2012). Of note, the antibody microarray content has expanded significantly since the former study, making correlation measures of the two studies difficult. The former study only contained 36% of the antibodies currently used and did for example not include the core marker CHP-1. However, the other four core signature proteins (Properdin, VEGF, IL-8 and C3) were all part of the previously published serum PDAC signature. In addition to the core panel, there was a clear overlap between the two ethnic cohorts when comparing the 25 highest ranked antibodies in the two studies (Table 2), demonstrating that blood-based proteomics analysis is less affected by the genetic make-up of sample donors (Caucasian/Asian), or sample format (serum/plasma).

In the previous Caucasian cohort it was also shown that PDAC could be differentiated both from controls and pancreatitis (Wingren et al., 2012) . Since our Asian cohort did not include chronic pancreatitis samples, we instead compared significant (Wilcoxon p<0.05) markers present in this and previous studies (Sandstrom et al., 2012; Wingren et al., 2012). Five markers (C3, C5, IL-12, IL-8, Properdin) were commonly expressed in both serum and plasma PDAC samples, as well as in pancreatitis samples. Despite this overlap, the PDAC-associated proteins were notably different compared to chronic pancreatitis. Moreover, the current and previous studies have demonstrated that it is the combination of markers in a multiplexed signature that will deliver the most precise accuracy, regardless of whether a subset of the markers are overlapping with inflammatory or non-related indications.

### Discussion

For the identification of a plasma marker signature two complementary strategies were initially used, (i) univariate differential expression analysis generating a multiple-testing corrected q-value for each antibody in the assay, and (ii) a backward elimination approach designed to pinpoint the optimal combination of markers, contributing with orthogonal information for discriminating cases and controls (Carlsson et al., 2011a). The two strategies resulted in the identification of a robust core signature of the five proteins Properdin, VEGF, IL-8, C3, and CHP-1. Properdin, or Complement Factor P, was previously identified in two independent studies as strongly down-regulated in both serum (Wingren et al., 2012) and plasma (Gerdtsson et al, unpublished observations), but has apart from that, to the best of our knowledge, not previously been associated with PDAC. In contrast, VEGF, associated with the angiogenesis-dependence of tumor growth, is a known upregulated marker in many cancers including PDAC (Itakura et al., 1997). The VEGF antibody, which specificity has been validated by mass spectrometry, has also in earlier studies demonstrated a significantly elevated VEGF level in PDAC (Gerdtsson, 2015; Wingren et al., 2012). Also IL-8 and Complement Factor C3 have previously been shown to be associated with late stage pancreatic cancer (Chen et al., 2013; Shaw et al., 2014; Wingren et al., 2012). In contrast, CHP-1, Calcineurin Homologous Protein-1, has not previously been measured and we believe its association with PDAC is novel to this study. CHP-1 is part of the Ca²⁺-binding family, and is a widely expressed protein localized in multiple subcellular compartments. Apart from being involved in trafficking across the plasma membrane, the functions of this presumably pluripotent protein is largely unknown (Jimenez-Vidal et al., 2010)

The results showed that the core signature needed to be supplemented with additional proteins in order to achieve the highest possible sensitivity and specificity. Here, approximately 23 markers were shown to deliver an optimal discrimination of PDAC vs. NC. In addition to the 5-protein core, several potential markers of interest were identified. Apo-A1 was one of the strongest differentially expressed proteins, as shown by all three Apo-A1-specific antibodies (q-values 0.003-0.02). Its significant down-regulation in plasma from PDAC patients has previously been seen (Honda et al., 2012) (Gerdtsson et al, unpublished observations). Apo-A1 is the major component of high density lipoprotein (HDL) in plasma and decreased levels of HDL are associated with poor cardiovascular health. Thus the reduced Apo-A1 levels observed in this study may reflect the association of PDAC with smoking and obesity. Another strongly down-regulated marker was Complement Factor C1q, in accordance with our previous study (Wingren et al., 2012). It is noteworthy that although both Apo-A1 and C1q were among the top markers based on univariate analysis, they were not included in the consensus signature derived from the backward elimination analysis, which only takes into account the performance of the combined signature and not the individual markers therein. In contrast, MAPK-8 was identified as a high scoring marker by both approaches. MAPK-8, a serine/threonine protein kinase involved in several cellular processes and signaling pathways, has not previously been analyzed in multiparametric assays and its discriminatory power and role in PDAC needs to be confirmed in future studies. Of note, although the sample subgroups were well age matched the PDAC and NC group had a skewed gender distribution. To investigate if the classifier for PDAC vs. NC was affected by the bias in gender, a gender adjusted dataset was created by an additional ComBat normalization step. The result clearly demonstrated that the list of significant antibodies for PDAC vs. NC in the gender adjusted dataset was highly similar to that of the original dataset. Furthermore, an SVM based analysis also demonstrated that male vs female were poorly separated, as compared to PDAC vs NC, again showing that gender was not a confounding factor for the PDAC vs NC classifiers.

To the best of our knowledge, this is the first proteomics study identifying stage-specific PDAC markers in plasma. Since early diagnosis significantly increases the life expectancy of PDAC patients (Furukawa et al., 1996; Shimizu et al., 2005), the defined markers associated with stage I/II are of particular importance when designing a clinically relevant test. Although the discrimination of PDAC vs. NC increased with PDAC stage, we were still able to discriminate stage I/II patients from normal individuals. The results are based on using all data from the microarray analysis, but could be condensed to signatures of high power. Several proteins, including AGAP-2, BTK, CDK-2, IL-13, IL-6, MUC-1, PTPRO, USP-7, were shown to have elevated levels in locally confined early stage cancer specifically. Four of these, AGAP-2, CDK-2, PTPRO, and USP7, have not previously been measured in our microarray assay. CDK-2, or Cyclin-dependent kinase 2, is involved in controlling the cell cycle and the aberrant activation of the CDKs is a well-known hallmark of many cancers, including PDAC (Feldmann et al., 2011). The remaining novel markers have not previously been associated with PDAC specifically Other PDAC markers that have been previously identified (Ingvarsson et al., 2008; Wingren et al., 2012), include MUC-1 which is overexpressed in 90% of PDAC cases (Winter et al., 2012), the cytokines IL-6 (Bellone et al., 2006) and IL-13 (Gabitass et al., 2011), as well as the tyrosine kinase BTK.

The focus of the present study was indeed the identification of stage-specific plasma proteins and the ability to discriminate between resectable pancreatic cancer and disseminated disease. However, differential diagnosis of cancer versus pancreatitis is sometimes difficult and an issue deciphered in a recent study focused in particular on inflammation in pancreas, where we showed that protein signatures could be identified that clearly discriminated between acute, chronic and autoimmune pancreatitis and normal controls (Sandstrom et al., 2012). Importantly, the inflammation associated protein signatures also differed from the Stage I, II, III, IV associated marker signatures in the present study. These findings were further supported by a previous study identifying a signature discriminating between pancreatic cancer patients and a combination of controls, including patients with different inflammatory indications of the pancreas, as well as healthy individuals (Wingren et al., 2012).

The biological diversity of tumors due to localization in pancreas has been previously demonstrated (Ling et al., 2013). Tumors in the body and tail of pancreas are rarer than tumor in the head of pancreas (77% of PDAC) (Lau et al., 2010). Because of differences in e.g. blood supply and lymphatic and venous backflow, there are also differences in the disease presentation with body and tail tumors causing less jaundice, more pain, higher albumin and CEA levels and lower CA19-9 levels (Eyigor et al., 2010; Watanabe et al., 2004). Body and tail tumors are more often detected at a late stage than head tumors and have a higher rate of metastasis. As the biological variances can result in different treatment efficiency (Wu et al., 2007), markers that can discriminate between tumor localization would be of clinical relevance and could aid personalized treatment strategies. However, few differences have been found on a genetic level, with no significant variation in the overall number of mutations, deletions and amplifications, or in K-ras point mutations (Ling et al., 2013). Here, 37 antibodies identified markers that showed on differential protein expression levels between head and body/tail tumors, and this expression pattern correlated remarkably well with a previous study. Consequently, these results are encouraging for a future development of a blood protein biomarker signature discriminating body/tail and head tumors at an early disease stage.

Ethnic genetic diversity is well described and is, in addition to environmental factors, coupled to e.g. the incidence and progression of cancer in different parts of the world (Gupta et al., 2014; Rastogi et al., 2004). On the contrary, we here show that PDAC patients of Asian and Caucasian origin express similar disease-associated protein signatures. While biological heterogeneity is indeed a hurdle in the search for genetic biomarkers (Gupta et al., 2014), our findings indicate that proteomic biomarkers may be more robust and transferrable between different ethnicities, due to less diversity on a whole protein level as compared to genetic mutations.

In summary, we have demonstrated that resectable pancreatic cancer (stage I/II) as well as locally advanced (stage III) and distant metastatic (stage IV) disease could for the first time be accurately discriminated, a prerequisite for a test focusing on early diagnosis of pancreatic cancer. Furthermore, we provide information that plasma protein markers associated with different tumor locations in the pancreas could be identified.

### References

Alonzo, T.A., Pepe, M.S., Moskowitz, C.S., 2002. Sample size calculations for comparative studies of medical tests for detecting presence of disease. Stat Med. 21, 835-852.
Bauden, M., Pamart, D., Ansari, D., Herzog, M., Eccleston, M., Micallef, J., Andersson, B., Andersson, R., 2015. Circulating nucleosomes as epigenetic biomarkers in pancreatic cancer. Clin Epigenet 7, 106.
Bellone, G., Smirne, C., Mauri, F.A., Tonel, E., Carbone, A., Buffolino, A., Dughera, L., Robecchi, A., Pirisi, M., Emanuelli, G., 2006. Cytokine expression profile in human pancreatic carcinoma cells and in surgical specimens: implications for survival. Cancer Immunol Immunother. 55, 684-698.
Brand, R.E., Nolen, B.M., Zeh, H.J., Allen, P.J., Eloubeidi, M.A., Goldberg, M., Elton, E., Arnoletti, J.P., Christein, J.D., Vickers, S.M., Langmead, C.J., Landsittel, D.P., Whitcomb, D.C., Grizzle, W.E., Lokshin, A.E., 2011. Serum biomarker panels for the detection of pancreatic cancer. Clin Cancer Res. 17, 805-816.
Bunger, S., Laubert, T., Roblick, U.J., Habermann, J.K., 2011. Serum biomarkers for improved diagnostic of pancreatic cancer: a current overview. J Cancer Res Clin Oncol. 137, 375-389.
Carlsson, A., Wingren, C., Ingvarsson, J., Ellmark, P., Baldertorp, B., Ferno, M., Olsson, H., Borrebaeck, C.A., 2008. Serum proteome profiling of metastatic breast cancer using recombinant antibody microarrays. Eur J Cancer 44, 472-480.
Carlsson, A., Wingren, C., Kristensson, M., Rose, C., Ferno, M., Olsson, H., Jernstrom, H., Ek, S., Gustavsson, E., Ingvar, C., Ohlsson, M., Peterson, C., Borrebaeck, C.A., 2011a. Molecular serum portraits in patients with primary breast cancer predict the development of distant metastases. Proc Natl Acad Sci U. S. A. 108, 14252-14257.
Carlsson, A., Wuttge, D.M., Ingvarsson, J., Bengtsson, A.A., Sturfelt, G., Borrebaeck, C.A., Wingren, C., 2011b. Serum protein profiling of systemic lupus erythematosus and systemic sclerosis using recombinant antibody microarrays. Mol Cell Proteomics. 10, M110 005033.
Chen, J., Wu, W., Zhen, C., Zhou, H., Yang, R., Chen, L., Hu, L., 2013. Expression and clinical significance of complement C3, complement C4b1 and apolipoprotein E in pancreatic cancer. Oncol Lett. 6, 43-48.
Conlon, K.C., Klimstra, D.S., Brennan, M.F., 1996. Long-term survival after curative resection for pancreatic ductal adenocarcinoma. Clinicopathologic analysis of 5-year survivors. Ann Surg. 223, 273-279.
Coussens, L.M., Werb, Z., 2002. Inflammation and cancer. Nature 420, 860-867.
Eyigor, C., Karaca, B., Kuzeyli-Yildirim, Y., Uslu, R., Uyar, M., Coker, A., 2010. Does the tumor localization in advanced pancreatic cancer have an influence on the management of symptoms and pain? J B.U.ON. 15, 543-546.
Feldmann, G., Mishra, A., Bisht, S., Karikari, C., Garrido-Laguna, I., Rasheed, Z., Ottenhof, N.A., Dadon, T., Alvarez, H., Fendrich, V., Rajeshkumar, N.V., Matsui, W., Brossart, P., Hidalgo, M., Bannerji, R., Maitra, A., Nelkin, B.D., 2011. Cyclin-dependent kinase inhibitor Dinaciclib (SCH727965) inhibits pancreatic cancer growth and progression in murine xenograft models. Cancer Biol Ther. 12, 598-609.
Furukawa, H., Okada, S., Saisho, H., Ariyama, J., Karasawa, E., Nakaizumi, A., Nakazawa, S., Murakami, K., Kakizoe, T., 1996. Clinicopathologic features of small pancreatic adenocarcinoma. A collective study. Cancer 78, 986-990.
Gabitass, R.F., Annels, N.E., Stocken, D.D., Pandha, H.A., Middleton, G.W., 2011. Elevated myeloid-derived suppressor cells in pancreatic, esophageal and gastric cancer are an independent prognostic factor and are associated with significant elevation of the Th2 cytokine interleukin-13. Cancer Immunol Immunother. 60, 1419-1430.
Gangi, S., Fletcher, J.G., Nathan, M.A., Christensen, J.A., Harmsen, W.S., Crownhart, B.S., Chari, S.T., 2004. Time interval between abnormalities seen on CT and the clinical diagnosis of pancreatic cancer: retrospective review of CT scans obtained before diagnosis. Am J Roentgenol. 182, 897-903.
Gerdtsson, A.S.M., N.; Säll, A.; Real, F. X.; Porta, M.; Skoog, P.; Persson, H.; Wingren, C.; Borrebaeck, C. A. K. , 2015. A Multicenter Trial Defining a Serum Protein Signature Associated with Pancreatic Ductal Adenocarcinoma. Int J Proteomics 2015.
Gupta, S., Venkatesh, A., Ray, S., Srivastava, S., 2014. Challenges and prospects for biomarker research: a current perspective from the developing world. Biochim Biophys Acta 1844, 899-908.
Haab, B.B., Geierstanger, B.H., Michailidis, G., Vitzthum, F., Forrester, S., Okon, R., Saviranta, P., Brinker, A., Sorette, M., Perlee, L., Suresh, S., Drwal, G., Adkins, J.N., Omenn, G.S., 2005. Immunoassay and antibody microarray analysis of the HUPO Plasma Proteome Project reference specimens: systematic variation between sample types and calibration of mass spectrometry data. Proteomics 5, 3278-3291.
Honda, K., Okusaka, T., Felix, K., Nakamori, S., Sata, N., Nagai, H., loka, T., Tsuchida, A., Shimahara, T., Shimahara, M., Yasunami, Y., Kuwabara, H., Sakuma, T., Otsuka, Y., Ota, N., Shitashige, M., Kosuge, T., Buchler, M.W., Yamada, T., 2012. Altered plasma apolipoprotein modifications in patients with pancreatic cancer: protein characterization and multi-institutional validation. PLoS One 7, e46908.
Hou, J., Xu, J., Jiang, R., Wang, Y., Chen, C., Deng, L., Huang, X., Wang, X., Sun, B., 2013. Estrogen-sensitive PTPRO expression represses hepatocellular carcinoma progression by control of STAT3. Hepatol. 57, 678-688.
Huang, Y.T., Li, F.F., Ke, C., Li, Z., Li, Z.T., Zou, X.F., Zheng, X.X., Chen, Y.P., Zhang, H., 2013. PTPRO promoter methylation is predictive of poorer outcome for HER2-positive breast cancer: indication for personalized therapy. J Transl Med. 11, 245.
Ingvarsson, J., Wingren, C., Carlsson, A., Ellmark, P., Wahren, B., Engstrom, G., Harmenberg, U., Krogh, M., Peterson, C., Borrebaeck, C.A., 2008. Detection of pancreatic cancer using antibody microarray-based serum protein profiling. Proteomics 8, 2211-2219.
Itakura, J., Ishiwata, T., Friess, H., Fujii, H., Matsumoto, Y., Buchler, M.W., Korc, M., 1997. Enhanced expression of vascular endothelial growth factor in human pancreatic cancer correlates with local disease progression. Clin Cancer Res. 3, 1309-1316.
Jimenez-Vidal, M., Srivastava, J., Putney, L.K., Barber, D.L., 2010. Nuclear-localized calcineurin homologous protein CHP1 interacts with upstream binding factor and inhibits ribosomal RNA synthesis. J Biol Chem. 285, 36260-36266.
Jin, Q., Kong, B., Yang, X., Cui, B., Wei, Y., Yang, Q., 2007. Overexpression of CHP2 enhances tumor cell growth, invasion and metastasis in ovarian cancer. In vivo 21, 593-598.
Johnson, W.E., Li, C., Rabinovic, A., 2007. Adjusting batch effects in microarray expression data using empirical Bayes methods. Biostatistics 8, 118-127.
Lau, M.K., Davila, J.A., Shaib, Y.H., 2010. Incidence and survival of pancreatic head and body and tail cancers: a population-based study in the United States. Pancreas 39, 458-462.
Ling, Q., Xu, X., Zheng, S.S., Kalthoff, H., 2013. The diversity between pancreatic head and body/tail cancers: clinical parameters and in vitro models. Hepatobil Pancreat Dis Intl. 12, 480-487.
Locker, G.Y., Hamilton, S., Harris, J., Jessup, J.M., Kemeny, N., Macdonald, J.S., Somerfield, M.R., Hayes, D.F., Bast, R.C., Jr., 2006. ASCO 2006 update of recommendations for the use of tumor markers in gastrointestinal cancer. J Clin Oncol. 24, 5313-5327.
Melo, S.A., Luecke, L.B., Kahlert, C., Fernandez, A.F., Gammon, S.T., Kaye, J., LeBleu, V.S., Mittendorf, E.A., Weitz, J., Rahbari, N., Reissfelder, C., Pilarsky, C., Fraga, M.F., Piwnica-Worms, D., Kalluri, R., 2015. Glypican-1 identifies cancer exosomes and detects early pancreatic cancer. Nature 523, 177-182.
Motiwala, T., Kutay, H., Ghoshal, K., Bai, S., Seimiya, H., Tsuruo, T., Suster, S., Morrison, C., Jacob, S.T., 2004. Protein tyrosine phosphatase receptor-type O (PTPRO) exhibits characteristics of a candidate tumor suppressor in human lung cancer. Proc Natl Acad Sci U. S. A. 101, 13844-13849.
Pannala, R., Basu, A., Petersen, G.M., Chari, S.T., 2009. New-onset diabetes: a potential clue to the early diagnosis of pancreatic cancer. Lancet Oncol. 10, 88-95.
Pauly, F., Smedby, K.E., Jerkeman, M., Hjalgrim, H., Ohlsson, M., Rosenquist, R., Borrebaeck, C.A., Wingren, C., 2014. Identification of B-cell lymphoma subsets by plasma protein profiling using recombinant antibody microarrays. Leukemia Res. 38, 682-690.
Pelaez-Luna, M., Takahashi, N., Fletcher, J.G., Chari, S.T., 2007. Resectability of presymptomatic pancreatic cancer and its relationship to onset of diabetes: a retrospective review of CT scans and fasting glucose values prior to diagnosis. Am J Gastroenterol. 102, 2157-2163.
Rastogi, T., Hildesheim, A., Sinha, R., 2004. Opportunities for cancer epidemiology in developing countries. Nature Rev Cancer 4, 909-917.
Sandstrom, A., Andersson, R., Segersvard, R., Lohr, M., Borrebaeck, C.A., Wingren, C., 2012. Serum proteome profiling of pancreatitis using recombinant antibody microarrays reveals disease-associated biomarker signatures. Proteomics Clin Appl. 6, 486-496.
Shaw, V.E., Lane, B., Jenkinson, C., Cox, T., Greenhalf, W., Halloran, C.M., Tang, J., Sutton, R., Neoptolemos, J.P., Costello, E., 2014. Serum cytokine biomarker panels for discriminating pancreatic cancer from benign pancreatic disease. Mol Cancer 13, 114.
Shimizu, Y., Yasui, K., Matsueda, K., Yanagisawa, A., Yamao, K., 2005. Small carcinoma of the pancreas is curable: new computed tomography finding, pathological study and postoperative results from a single institute. J Gastroenterol Hepatol. 20, 1591-1594.
Sohn, T.A., Yeo, C.J., Cameron, J.L., Koniaris, L., Kaushal, S., Abrams, R.A., Sauter, P.K., Coleman, J., Hruban, R.H., Lillemoe, K.D., 2000. Resected adenocarcinoma of the pancreas-616 patients: results, outcomes, and prognostic indicators. J Gastrointest Surg. 567-579.
Song, M.S., Salmena, L., Carracedo, A., Egia, A., Lo-Coco, F., Teruya-Feldstein, J., Pandolfi, P.P., 2008. The deubiquitinylation and localization of PTEN are regulated by a HAUSP-PML network. Nature 455, 813-817.
Steinhauer, C., Wingren, C., Hager, A.C., Borrebaeck, C.A., 2002. Single framework recombinant antibody fragments designed for protein chip applications. BioTechniques Suppl. 38-45.
Stoevesandt, O., Taussig, M.J., 2012. European and international collaboration in affinity proteomics. N biotechnol. 29, 511-514.
Surinova, S., Schiess, R., Huttenhain, R., Cerciello, F., Wollscheid, B., Aebersold, R., 2011. On the development of plasma protein biomarkers. J Proteome Res. 10, 5-16.
Watanabe, I., Sasaki, S., Konishi, M., Nakagohri, T., Inoue, K., Oda, T., Kinoshita, T., 2004. Onset symptoms and tumor locations as prognostic factors of pancreatic cancer. Pancreas 28, 160-165.
Wingren, C., Borrebaeck, C.A., 2008. Antibody microarray analysis of directly labelled complex proteomes. Curr Opin Biotechnol. 19, 55-61.
Wingren, C., Sandstrom, A., Segersvard, R., Carlsson, A., Andersson, R., Lohr, M., Borrebaeck, C.A., 2012. Identification of serum biomarker signatures associated with pancreatic cancer. Cancer Res. 72, 2481-2490.
Wingren, C., Steinhauer, C., Ingvarsson, J., Persson, E., Larsson, K., Borrebaeck, C.A., 2005. Microarrays based on affinity-tagged single-chain Fv antibodies: sensitive detection of analyte in complex proteomes. Proteomics 5, 1281-1291.
Winter, J.M., Tang, L.H., Klimstra, D.S., Brennan, M.F., Brody, J.R., Rocha, F.G., Jia, X., Qin, L.X., D'Angelica, M.I., DeMatteo, R.P., Fong, Y., Jarnagin, W.R., O'Reilly, E.M., Allen, P.J., 2012. A novel survival-based tissue microarray of pancreatic cancer validates MUC1 and mesothelin as biomarkers. PLoS One 7, e40157.
Wu, T.C., Shao, Y.F., Shan, Y., Wu, J.X., Zhao, P., 2007. [Surgical effect of malignant tumor of body and tail of the pancreas: compare with pancreatic head cancer]. Zhonghua wai ke za zhi [Chinese journal of surgery] 45, 30-33.
Xia, C., Ma, W., Stafford, L.J., Liu, C., Gong, L., Martin, J.F., Liu, M., 2003. GGAPs, a new family of bifunctional GTP-binding and GTPase-activating proteins. Mol Cell Biol. 23, 2476-2488.
Yachida, S., Jones, S., Bozic, I., Antal, T., Leary, R., Fu, B., Kamiyama, M., Hruban, R.H., Eshleman, J.R., Nowak, M.A., Velculescu, V.E., Kinzler, K.W., Vogelstein, B., lacobuzio-Donahue, C.A., 2010. Distant metastasis occurs late during the genetic evolution of pancreatic cancer. Nature 467, 1114-1117.
Zhao, G.Y., Lin, Z.W., Lu, C.L., Gu, J., Yuan, Y.F., Xu, F.K., Liu, R.H., Ge, D., Ding, J.Y., 2014. USP7 overexpression predicts a poor prognosis in lung squamous cell carcinoma and large cell carcinoma. Tumour Biol. 36, 1721-1729.

| **TABLE A** | | | | | | |
|---|---|---|---|---|---|---|
| **(I) - Preferred 1/2/1+2** | | | | | | |
| **#** | **Short name** | **Accession #** | **1-4 vs HC** | **1 vs HC** | **2 vs HC** | **1+2 vs HC** |
| 1 | R-PTP-O | Q16827 | | ↑ | ↑ | ↑ |
| 2 | UBP7 | Q93009 | ↑ | ↑ | ↑ | ↑ |
| 3 | CHP-1 | Q99653 | ↑ | ↑ | ↑ | ↑ |
| 4 | MAPKK 2 | P36507 | ↓ | ↓ | ↓ | ↓ |
| 5 | MAPKK 6 | P52564 | | ↑ | ↑ | ↑ |

| **(II) - Optional 1/2/1+2** | | | | | | |
|---|---|---|---|---|---|---|
| **#** | **Short name** | **Accession #** | **1-4 vs HC** | **1 vs HC** | **2 vs HC** | **1+2 vs HC** |
| 6 | Apo-A1 | P02647 | ↓ | ↓ | ↓ | ↓ |
| 7 | BTK | Q06187 | ↑ | ↑↓ | ↑ | ↑ |
| 8 | C1q | P02745/6/7 | ↓ | ↓ | ↓ | ↓ |
| 9 | C3 | P01024 | ↓ | ↓ | ↓ | ↓ |
| 10 | C5 | P01031 | ↑ | ↑↓ | ↑ | ↑ |
| 11 | CDK-2 | P24941 | ↑ | ↑ | ↑ | ↑ |
| 12 | IgM | P01871 | ↓ | ↓ | ↓ | ↓ |
| 13 | IL-11 | P20809 | ↑ | ↑ | ↑ | ↑ |
| 14 | IL-12 | P29459/60 | ↑ | ↑ | ↑ | ↑ |
| 15 | IL-6 | P05231 | ↑↓ | ↑ | ↑ | ↑ |
| 16 | JAK3 | P52333 | ↑ | ↑ | ↑ | ↑ |
| 17 | MAPK8 | P45983 | ↑ | ↑ | ↑ | ↑ |
| 18 | MCP-1 | P13500 | ↑↓ | ↑ | ↑ | ↑ |
| 19 | MUC-1 | P15941 | ↑ | ↑ | ↑ | ↑ |
| 20 | Properdin | P27918 | ↓ | ↓ | ↓ | ↓ |
| 21 | VEGF | P15692 | ↑ | ↑ | ↑ | ↑ |

| **(III) - Preferred 1/1+2** | | | | | | |
|---|---|---|---|---|---|---|
| **#** | **Short name** | **Accession #** | **1-4 vs HC** | **1 vs HC** | **2 vs HC** | **1+2 vs HC** |
| 22 | PRD14 | Q9GZV8 | ↑ | ↑ | | ↑ |
| 23 | GRIP-2 | Q9C0E4 | | ↑ | | ↑ |
| 24 | MAPK9 | P45984 | | ↑ | | ↑ |
| 25 | PKB gamma (AKT-3) | Q9Y243 | | ↑ | | ↑ |
| 26 | R-PTP-eta | Q12913 | | ↑↓ | | ↑ |
| 27 | TopBP1 | Q92547 | | ↑ | | ↑↓ |

| **(IV) - Optional 1/1+2** | | | | | | |
|---|---|---|---|---|---|---|
| **#** | **Short name** | **Accession #** | **1-4 vs HC** | **1 vs HC** | **2 vs HC** | **1+2 vs HC** |
| | | | | | | |
| 28 | CD40L | P29965 | | ↑ | | ↑ |
| 29 | EGFR | P00533 | | ↑ | | ↑ |
| 30 | HADH2 | Q6IBS9 | | ↑ | | ↑ |
| 31 | ICAM-1 | P05362 | ↑↓ | ↑↓ | | ↑↓ |
| 32 | IL-13 | P35225 | ↑↓ | ↑ | | ↑ |
| 33 | IL-18 | Q14116 | | ↑ | | ↑ |
| 34 | MYOM2 | P54296 | | ↓ | | ↓ |
| 35 | Osteopontin | P10451 | | ↑ | | ↑ |
| 36 | P85A | P27986 | | ↑↓ | | ↑↓ |
| 37 | RANTES | P13501 | | ↑ | | ↑ |
| 38 | TGF-b1 | P01137 | | ↑ | | ↑ |
| 39 | IL-4 | P05112 | | ↑ | | ↑ |

| **(V) - Preferred 2/1+2** | | | | | | |
|---|---|---|---|---|---|---|
| **#** | **Short name** | **Accession #** | **1-4 vs HC** | **1 vs HC** | **2 vs HC** | **1+2 vs HC** |
| 40 | CIMS (13) | SSAYSR* | | | ↑ | ↑ |
| 41 | GNAI3 | P08754 | | | ↑ | ↑ |
| 42 | HsMAD2 | Q13257 | | | ↑ | ↑ |
| 43 | hSpindly | Q96EA4 | | | ↑ | ↑ |
| 44 | R-PTP-kappa | Q15262 | | | ↑↓ | ↑↓ |
| 45 | STAT1 | P42224 | ↑↓ | | ↑ | ↑ |

| **(VI) - Optional 2/1+2** | | | | | | |
|---|---|---|---|---|---|---|
| **#** | **Short name** | **Accession #** | **1-4 vs HC** | **1 vs HC** | **2 vs HC** | **1+2 vs HC** |
| 46 | ATP-5B | P06576 | ↑ | | ↑ | ↑ |
| 47 | C4 | POCOL4/5 | ↓ | | ↓ | ↓ |
| 48 | CHX10 | P58304 | | | ↑ | ↑ |
| 49 | Factor B | P00751 | | | ↓ | ↓ |
| 50 | Her2/ErbB-2 | P04626 | ↑ | | ↑ | ↑ |
| 51 | IL-1b | P01584 | | | ↑↓ | ↑↓ |
| 52 | IL-7 | P13232 | ↑↓ | | ↑ | ↑ |
| 53 | IL-10 | P22301 | | | ↑ | ↑ |
| 54 | Lewis X | NA | | | ↑ | ↑ |
| 55 | MCP-3 | P80098 | | | ↑ | ↑ |
| 56 | Sialyl Lewis x | NA | | | ↑ | ↑ |
| 57 | TBC1D9 | Q6ZT07 | | | ↑ | ↑ |
| 58 | TNFRSF3 | P36941 | | | ↑ | ↑ |

| **(VII) - Preferred 1** | | | | | | |
|---|---|---|---|---|---|---|
| **#** | **Short name** | **Accession #** | **1-4 vs HC** | **1 vs HC** | **2 vs HC** | **1+2 vs HC** |
| 59 | AGAP-2 | Q99490 | ↑↓ | ↑ | | |

| **(VIII)** - **Optional 1** | | | | | | |
|---|---|---|---|---|---|---|
| **#** | **Short name** | **Accession #** | **1-4 vs HC** | **1 vs HC** | **2 vs HC** | **1+2 vs HC** |
| 60 | C1-INH | P05155 | | ↑ | | |
| 61 | C1s | P09871 | | ↑↓ | | |
| 62 | GLP-1 R | P43220 | ↑↓ | ↑ | | |
| 63 | IL-2 | P60568 | | ↑↓ | | |
| 64 | KSYK | P43405 | | ↑ | | |
| 65 | MAPK1 | P28482 | | ↑ | | |

| **(IX) - Preferred 2** | | | | | | |
|---|---|---|---|---|---|---|
| **#** | **Short name** | **Accession #** | **1-4 vs HC** | **1 vs HC** | **2 vs HC** | **1+2 vs HC** |
| 66 | TENS4 | Q8IZW8 | | | ↑ | |

| **(X) - Optional 2** | | | | | | |
|---|---|---|---|---|---|---|
| **#** | **Short name** | **Accession #** | **1-4 vs HC** | **1 vs HC** | **2 vs HC** | **1+2 vs HC** |
| 67 | FASN | Q6PJJ3 | | | ↑↓ | |
| | | | | | | |
| 68 | IL-3 | P08700 | ↑↓ | | ↑↓ | |
| 69 | IL-8 | P10145 | ↓ | | ↑↓ | |
| 70 | STAP2 | Q9UGK3 | | | ↑↓ | |

| **(XI) - Preferred 1+2** | | | | | | |
|---|---|---|---|---|---|---|
| **#** | **Short name** | **Accession #** | **1-4 vs HC** | **1 vs HC** | **2 vs HC** | **1+2 vs HC** |
| 71 | HsHec1 | 014777 | | | | ↑ |
| 72 | PAR-6B | Q9BYG5 | | | | ↑ |
| 73 | PGAM5 | Q96HS1 | ↑ | | | ↑ |

| **(XII)** - **Optional 1+2** | | | | | | |
|---|---|---|---|---|---|---|
| **#** | **Short name** | **Accession #** | **1-4 vs HC** | **1 vs HC** | **2 vs HC** | **1+2 vs HC** |
| 74 | IFN-γ | P01579 | | | | ↑ |
| 75 | IL-16 | Q14005 | | | | ↑ |
| | | | | | | |
| 76 | Sox11A | P35716 | | | | ↑ |
| 77 | TNF-b | P01374 | | | | ↑ |
| 78 | TNFRSF14 | Q92956 | | | | ↑ |
| 79 | UPF3B | Q9BZI7 | | | | ↑ |

| **(XIII) - Preferred 1-4** | | | | | | |
|---|---|---|---|---|---|---|
| **#** | **Short name** | **Accession #** | **1-4 vs HC** | **1 vs HC** | **2 vs HC** | **1+2 vs HC** |
| 80 | LUM | P51884 | ↑↓ | | | |
| 81 | PTPRO | Q16827 | ↑↓ | | | |

| **(XIV) - Optional 1-4** | | | | | | |
|---|---|---|---|---|---|---|
| **#** | **Short name** | **Accession #** | **1-4 vs HC** | **1 vs HC** | **2 vs HC** | **1+2 vs HC** |
| 82 | GM-CSF | P04141 | ↑ | | | |
| 83 | IL-9 | P15248 | ↑↓ | | | |
| 84 | LDL | P04114 | ↑↓ | | | |
| 85 | ORP-3 | Q9H4L5 | ↑↓ | | | |

| | | | | | | |
|---|---|---|---|---|---|---|
| * peptide antibody was selected against, not database accession number. '↑' indicates the biomarker is up-regulated in PC; '↓' indicates the biomarker is down-regulated in PC; '↑↓' indicates the biomarker is dysregulated in PC, but the trend may be up- or down-regulation (i.e., the biomarker is up-regulated in some PC subtype(s), down-regulated in others, and/or non-dysregulated yet others); | | | | | | |

**Table 1. Clinical samples**

| *Group* | *No of samples* | *Gender (M*/*F)* | *Median age (range)* | *Tumor location* | *Stage groupins** |
|---|---|---|---|---|---|
| **PDAC** | **118** | **76/42** | **59 (21-83)** | **Head=69, Body/Tail=39** | |
| Stage I | 11 | 6/5 | 59 (48-71) | Head=6, Body/Tail= | T1/2, N0, M0 |
| Stage II | 33 | 16/17 | 59 (46-83) | Head=27, Body/Tail=6 Head=22, Body/Tail=12, | T1-3, N0/1, M0 |
| Stage III | 38 | 28/10 | 59 (21-75) | Neck/Neck+Body=4 | T4, any N, M0 |
| Stage IV | 36 | 26/10 | 59 (38-75) | Head=14, Body/Tail=20, Neck/Head+Tail=2 | Any T & N, M1 |
| **NC** | **95** | **20/75** | **63 (52-74)** | N/A | |
| Total | 213 | 96/117 | 62 (21-83) | | |

| | | | | | |
|---|---|---|---|---|---|
| * Staging according to the guidelines of the American Cancer Society | | | | | |

**Table 2. Top 25 signature candidate analytes**

| *Elimination rank* | *Name* | *Median elimination score* | *Wilcoxon p-value* | *Benjamini- Hochberg q-value* | *Wilcoxon rank* | *Fold change* | *Previous elimination rank (Wingren et al., 2012)* |
|---|---|---|---|---|---|---|---|
| 1 | Properdin | 1 | 6.18E-15 | 2.08E-12 | 1 | 0.74 | 3 |
| 2 | VEGF (3) | 2 | 1.84E-08 | 3.10E-06 | 2 | 1.25 | 17 |
| 3 | IL-8 (3) | 11.5 | 1.99E-03 | 6.07E-02 | 11 | 0.87 | 114 |
| 4 | C3 (4) | 12.5 | 3.74E-05 | 3.15E-03 | 4 | 0.83 | N/A |
| 5 | CHP-1 (2) | 13 | 4.47E-06 | 5.00E-04 | 3 | 1.16 | N/A |
| 6 | C3 (3) | 17 | 1.31E-01 | 6.01E-01 | 72 | 1.08 | N/A |
| 7 | MAPK-8 (3) | 19.5 | 1.85E-04 | 7.76E-03 | 8 | 1.22 | N/A |
| 8 | MCP-1 (6) | 19.5 | 1.32E-01 | 6.01E-01 | 74 | 0.96 | N/A |
| 9 | IL-7 (2) | 23 | 2.53E-01 | 7.60E-01 | 111 | 1.03 | 21 |
| 10 | C4 (3) | 23.5 | 2.96E-03 | 8.29E-02 | 12 | 0.89 | N/A |
| 11 | IgM (5) | 24.5 | 5.61E-05 | 3.77E-03 | 5 | 0.92 | N/A |
| 12 | IL-3 (1) | 27 | 7.36E-02 | 5.72E-01 | 40 | 1.06 | 18 |
| 13 | IL-11 (3) | 28 | 2.17E-02 | 3.03E-01 | 24 | 1.08 | 35 |
| 14 | C5 (2) | 31 | 1.81E-02 | 2.84E-01 | 21 | 1.09 | 1 |
| 15 | IL-6 (6) | 32 | 4.95E-01 | 8.88E-01 | 187 | 0.97 | N/A |
| 16 | C3 (6) | 35 | 6.06E-02 | 5.66E-01 | 36 | 1.09 | N/A |
| 17 | ICAM-1 | 37 | 7.36E-01 | 9.37E-01 | 263 | 1.01 | N/A |
| 18 | MCP-1 (1) | 38.5 | 1.22E-01 | 5.89E-01 | 69 | 1.04 | 15 |
| 19 | LDL (1) | 39.5 | 2.33E-01 | 7.52E-01 | 104 | 1.02 | N/A |
| 20 | JAK3 | 42 | 1.96E-02 | 2.86E-01 | 23 | 1.05 | 34 |
| 21 | MAPK-8 (2) | 43 | 6.79E-02 | 5.72E-01 | 38 | 1.07 | N/A |
| 22 | MUC-1 (5) | 44.5 | 1.02E-02 | 2.29E-01 | 15 | 1.09 | 53 |
| 23 | BTK (3) | 44.5 | 2.06E-01 | 7.26E-01 | 93 | 1.03 | N/A |
| 24 | IL-7 (1) | 45 | 1.61E-01 | 6.61E-01 | 82 | 0.97 | 88 |
| 25 | LUM | 46.5 | 1.04E-01 | 5.89E-01 | 55 | 1.05 | N/A |

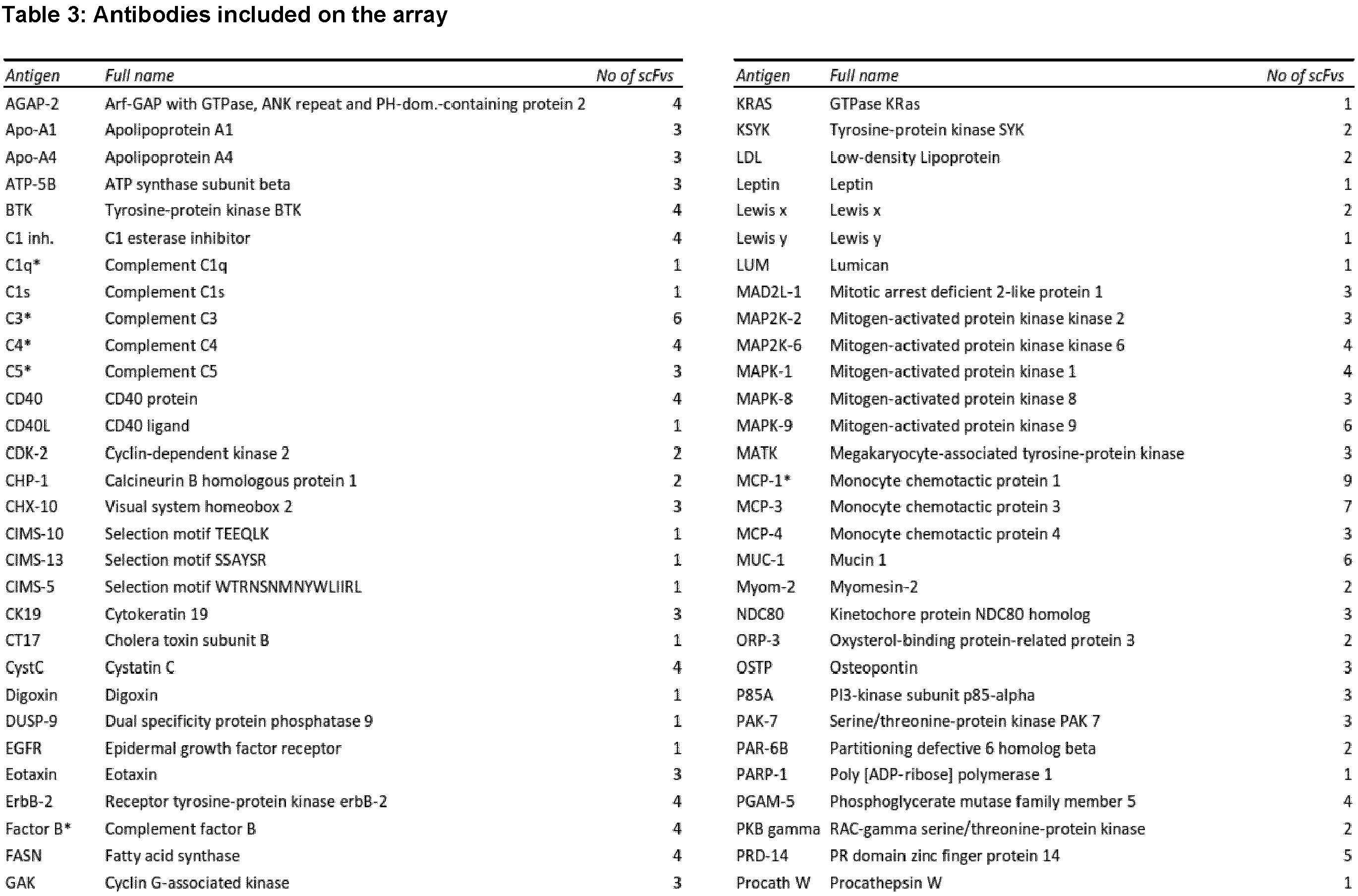

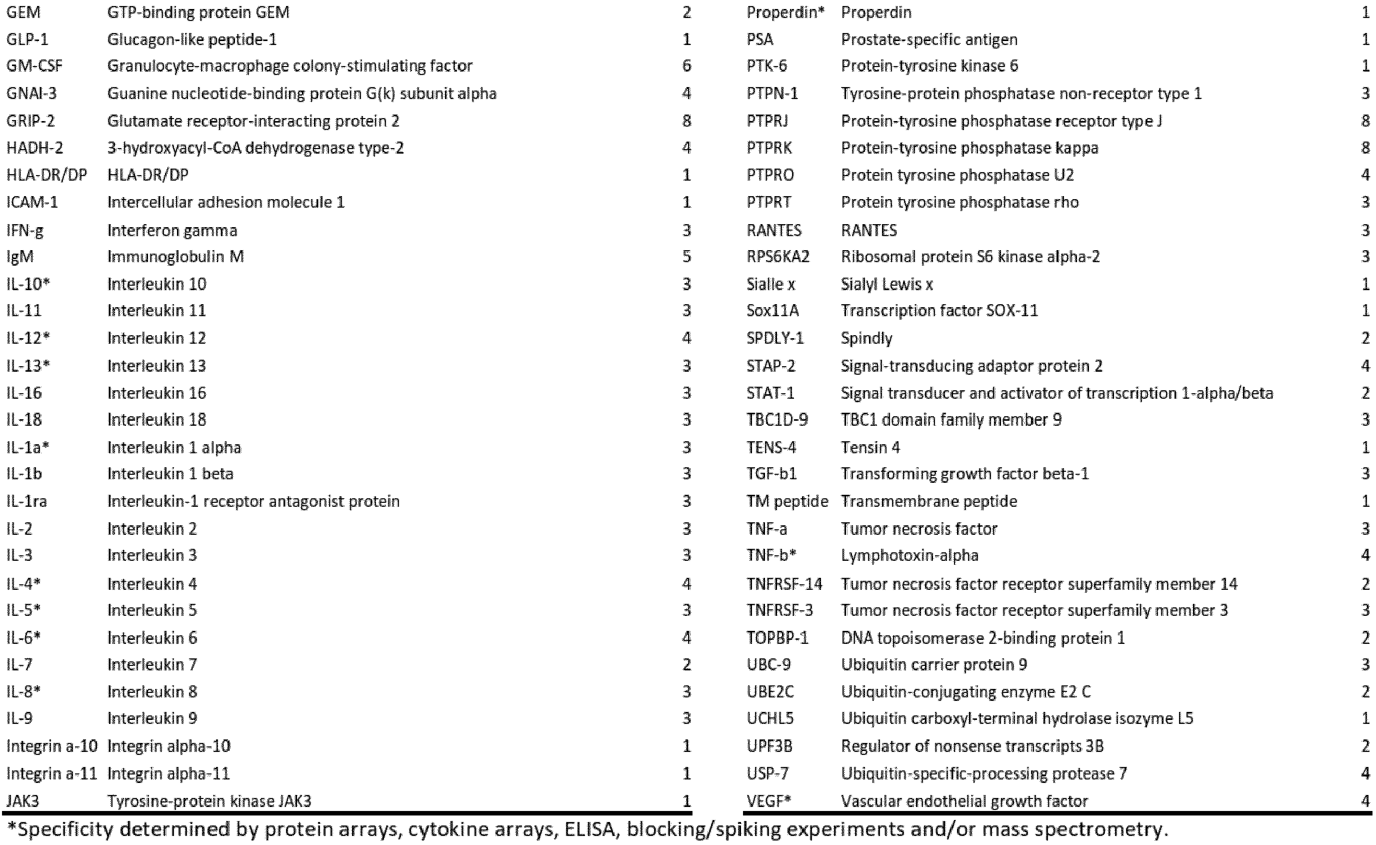

**Table 4: comparison of significant (Wilcoxon p<0.05) markers**

| ***cancer vs non-cancer all data*** | | | | ***cancer vs non-cancer gender-adjusted data*** | | | |
|---|---|---|---|---|---|---|---|
| PDAC (108) vs NC (94) | ROC area: | 0.88 | | PDAC (108) vs NC (94) | ROC area: | 0.89 | |

| Name | Fold change | Wilcoxon p-value | BH q-value | Name | Fold change | Wilcoxon p-value | BH q-value |
|---|---|---|---|---|---|---|---|
| Properdin | 0.741 | 6.18E-15 | 2.08E-12 | Properdin | 0.753 | 5.17E-14 | 1.74E-11 |
| VEGF | 1.252 | 1.84E-08 | 3.10E-06 | VEGF | 1.267 | 4.36E-09 | 7.33E-07 |
| CBPP22 | 1.164 | 4.47E-06 | 0.00050 | IgM | 0.912 | 3.30E-06 | 0.00030 |
| C3 | 0.830 | 3.74E-05 | 0,00315 | C3 | 0.815 | 3.62E-06 | 0.00030 |
| IgM | 0.924 | 5.61E-05 | 0.00377 | CBPP22 | 1.154 | 1.16E-05 | 0.00078 |
| C1q | 0.889 | 0.000129 | 0.00644 | C1q | 0.881 | 3.99E-05 | 0.00223 |
| MK08 | 1.218 | 0,000185 | 0.00776 | ApoA1 | 0.899 | 9.03E-05 | 0.00434 |
| ApoA1 | 0.885 | 0.000432 | 0.01615 | MK08 | 1.219 | 0.000142 | 0,00531 |
| **IL-8** | 0.874 | 0.001986 | 0.06066 | BTK | 1.083 | 0.000278 | 0.00933 |
| C4 | 0.888 | 0.002962 | 0.08293 | UBP7 | 1.101 | 0.000398 | 0.01178 |
| Her2 | 1.058 | 0.006326 | 0.16351 | Her2 | 1.078 | 0.000465 | 0.01202 |
| MAP2K2 | 0.939 | 0.007056 | 0.16935 | MUC1 | 1.110 | 0.000659 | 0.01582 |
| MUC1 | 1.086 | 0.010208 | 0.22866 | PRD14 | 1.074 | 0.000993 | 0,02223 |
| UBP7 | 1.078 | 0.011248 | 0.23620 | JAK3 | 1.074 | 0.001081 | 0.02271 |
| C5 | 1.059 | 0.012983 | 0.25660 | **MCP-3** | 1.083 | 0.001672 | 0.03305 |
| **CDK2** | 1.072 | 0.014557 | 0.27173 | **TNFRSF3** | 1.060 | 0.001922 | 0.03447 |
| **ATP5B** | 1.060 | 0.015457 | 0.27334 | MAP2K6 | 1.079 | 0.002002 | 0.03447 |
| PGAM5 | 1071 | 0.016622 | 0,27926 | PGAM5 | 1.093 | 0.002051 | 0,03447 |
| **MAP2K6** | 1.109 | 0.018576 | 0.28371 | **MAPK9** | 1.100 | 0.002189 | 0.03502 |
| JAK3 | 1.053 | 0.019563 | 0.28579 | **MCP1** | 1.107 | 0.002610 | 0.03863 |
| **IL-11** | 1.079 | 0.021675 | 0.30345 | MAP2K2 | 0.933 | 0.002694 | 0.03863 |
| PRD14 | 1.049 | 0.B31840 | 0.41147 | **STAT1** | 1.073 | 0.002759 | 0.03863 |
| **IL-12** | 1.069 | 0.039907 | 9 47888 | C4 | 0.888 | 0.003080 | 0.04139 |
| BTK | 1.050 | 0.048259 | 0.54050 | C5 | 1.071 | 0.00.3203 | 0.04139 |
| **CSF2** | 1.039 | 0048259 | 0.54050 | **PTPRJ** | 1.080 | 0.003330 | 0.04144 |

**Table 5**

| |
|---|
| ```
 apriori ← read.delim("apri.txt",header=FALSE)
 apriori ← as.character(apriori[[1]])
 aprioriBoolean ← is.element(rownames(data), apriori)
 filnamn←"Stages I+II.txt"
 rawfile ← read.delim(filnamn)
 samplenames ← as.character(rawfile[,1])
 groups ← rawfile[,2]
 data ← t(rawfile[,-c(1:2)])
 ProteinNames ← read.delim(filnamn,header=FALSE)
 ProteinNames ← as.character(as.matrix(ProteinNames)[1,])
 ProteinNames ← ProteinNames[-(1:2)]
 rownames(data) ← ProteinNames
 colnames(data) ← samplenames
 library(MASS)
 library(gplots)
 library(e1071)
``` |
| ```
 source("NaiveBayesian")
 redgreen ← function(n)
    {
      c(
         hsv(h=0/6, v=c(rep(seq(1,0.3,length=5), c(13,10, 8, 6, 4)), 0)
 ),hsv(h=2/6, v=c(0 , rep(seq(0.3,1,length=5) , c(3, 5, 7, 9, 11)))
 )
        )
    }
    pal ← rev(redgreen(100));
 svmLOOvalues ← function(data , fac) {
 n1 ← sum(fac==levels(fac)[1])
 n2 ← sum(fac==levels(fac)[2])
 nsamples ← n1+n2
 ngenes ← nrow(data)
 SampleInformation ← paste(levels(fac)[1]," ",nl," , ",levels(fac)[2],"
 ",n2,sep="")
 res ← numeric(nsamples)
 sign ← numeric(nsamples)
 for (i in 1:nsamples) {
   svmtrain ← svm(t(data[,-i]), fac[-i], kernel="linear")
  pred ← predict(svmtrain , t(data[,i]), decision.values=TRUE)
   res[i] ← as.numeric(attributes(pred)$decision.values)
   facnames ← colnames(attributes(pred)$decision.values)[1]
  if (facnames ==
  paste(levels(fac)[1],"/",levels(fac)[2],sep="")){sign[i] ← 1}
  if (facnames ==
  paste(levels(fac)[2],"/",levels(fac)[1],sep="")){sign[i] ← -1}
 }
 if (length(unique(sign)) >1) {print("error") }
 res ← sign * res
 names ← colnames(data , do.NULL=FALSE)
 orden ← order(res , decreasing=TRUE)
 Samples ← data.frame(names[orden],res[orden],fac[orden])
 ROCdata ← myROC(res,fac)
 SenSpe ← SensitivitySpecificity(res,fac)
 return(list(SampleInformation=SampleInformation,ROCarea=ROCdata[1],p.val
 ue=ROCdata[2],SenSpe ← SenSpe,samples=Samples))
 }
 wilcoxtest ← function(prot,subsetl,subset2){
 res ← wilcox.test(prot[subsetl],prot[subset2])
 res$p.value
 }
 # Definierar foldchange
 foldchange ← function(prot,subsetl,subset2){
  2^(mean(prot[subsetl]) - mean(prot[subset2]))
 }
 # Definierar q-värdesberäkningen
 BenjaminiHochberg ← function(pvalues){
    # This function takes a vector of p-values as input and outputs
    # their q-values. No reordering of the values is performed
    NAindices ← is.na(pvalues)
    Aindices ← !NAindices
``` |
| ```
    Apvalues ← pvalues[Aindices]
    N ← length(Apvalues)
    orderedindices ← order(Apvalues)
    OrdValues ← Apvalues[orderedindices]
    CorrectedValues ← OrdValues * N /(1:N)
    MinValues ← CorrectedValues
    for (i in 1:N) {MinValues[i] ← min(CorrectedValues[i:N])}
    Aqvalues ← numeric(N)
    Aqvalues[orderedindices] ← MinValues
    Qvalues ← pvalues
    Qvalues[Aindices] ← Aqvalues
    return(Qvalues)
 }
 AnalyseraMAA ← function(groupl ,group2){
  outputfiletxt ← paste(groupl," versus ",group2,".txt" ,sep="")
  outputfilepdf ← paste(groupl," versus ",group2,".pdf" ,sep="")
   subsetl ← is.element(groups , strsplit(group1,",") [[1]])
   subset2 ← is.element(groups , strsplit(group2,",") [[1]])
  wilcoxpvalues ← apply(data , 1 , wilcoxtest , subsetl , subset2)
   foldchange ← apply(data , 1 , foldchange , subsetl , subset2)
  QvaluesAll ← BenjaminiHochberg(wilcoxpvalues)
  QvaluesApriori ← numeric(length(wilcoxpvalues))
  QvaluesApriori[!aprioriBoolean] ← NA
  QvaluesApriori[aprioriBoolean] ←
  BenjaminiHochberg(wilcoxpvalues[aprioriBoolean])
  HugeTable ←
 cbind(ProteinNames,foldchange,wilcoxpvalues,QvaluesAll, QvaluesApriori)
  write.table(HugeTable, file=outputfiletxt , quote=FALSE,
 sep="\t",row.names=FALSE)
   color ← rep('black' , length(subsetl))
   color[subsetl] ← 'red'
   color[subset2] ← 'blue'
  pdf(outputfilepdf)
   Sam ← sammon(dist(t(data[,subset1\|subset2])), k=2)
  plot(Sam$points , type="n" , xlab = NA , ylab=NA, main="All proteins"
 , asp=1)
  text(Sam$point , labels = colnames(data[,subset1\|subset2]),
 col=color[subset1\|subset2])
   Sam ← sammon(dist(t(data[aprioriBoolean,subset1\|subset2])), k=2)
  plot(Sam$points , type="n" , xlab = NA , ylab=NA, main="Apriori
  proteins" ,asp=1)
  text(Sam$point , labels = colnames(data[,subset1\|subset2]),
 col=color[subset1\|subset2])
  heatmap.2(data[,subset1\|subset2] , labRow = row.names(data),
  trace="none" , labCol ="" , ColSideColors=
 color[subset1\|subset2],col=pal , na.color= "grey", key=FALSE , symkey
 =FALSE , tracecol = "black" , main ="" , dendrogram= 'both' , scale
 ="row" ,cexRow=0.2)
  heatmap.2(data[apriori,subset1\|subset2] , labRow =
 row.names(data[apriori,subset1\|subset2]), trace="none" , labCol ="" ,
 ColSideColors= color[subset1\|subset2],col=pal , na.color= "grey",
 key=FALSE , symkey =FALSE , tracecol = "black" , main ="" , dendrogram=
 'both' , scale ="row")
   svmfac ←
 factor(rep('rest',ncol(data)),levels=c(group1,group2,'rest'))
   svmfac[subsetl] ← group1
   svmfac[subset2] ← group2
   svmResAll ← svmLOOvalues(data[,subset1\|subset2],
 factor(as.character(svmfac[subset1\|subset2]),levels=c(groupl,group2)))
   svmResApriori ← svmLOOvalues(data[apriori,subset1\|subset2],
``` |
| ```
 factor(as.character(svmfac[subset1\|subset2]),levels=c(group1,group2)))
  ROCplot(svmResAll , sensspecnumber=4)
  ROCplot(svmResApriori , sensspecnumber=4)
  write("" , file=outputfiletxt , append=TRUE)
  write("All proteins" , file=outputfiletxt , append=TRUE)
  write("" , file=outputfiletxt , append=TRUE)
   for (i in 1:5) {write.table(svmResAll[[i]], file=outputfiletxt ,
   append=TRUE, sep="\t" , quote=FALSE)
                     write("" , file=outputfiletxt , append=TRUE)
   }
  write("" , file=outputfiletxt , append=TRUE)
  write("Apriori proteins" , file=outputfiletxt , append=TRUE)
  write("" , file=outputfiletxt , append=TRUE)
   for (i in 1:5) {write.table(svmResApriori [[i]], file=outputfiletxt ,
   append=TRUE, sep="\t" , quote=FALSE)
                     write("" , file=outputfiletxt , append=TRUE)
   }
  dev.off()
 }
 AnalyseraMAA("I+II","non-cancer")
``` |

**Table 6 - ROC-AUCs for (example signature 1)**

| ***Order of elimination*** | ***smallestErrorPerLength*** | ***Antibody*** | ***Uniprot entry ID*** | ***Full antigen name*** | ***Publication name*** | ***Signature length*** | ***ROC-AUC*** |
|---|---|---|---|---|---|---|---|
| 336 | NA | **Prop-3** | P27918 | Properdin | Properdin | 1 | NA |
| 335 | 68.227384 | **VEGF-3** | P15692 | Vascular endothelial growth factor | VEGF (3) | 2 | 0.84 |
| 334 | 56.359648 | **Clq-4** | P02745/6/7 | Complement C1q | C1q | 3 | 0.89 |
| 333 | 52.20471 | **IL-11-45** | P20809 | Interleukin-11 | IL-11 (3) | 4 | 0.89 |
| 332 | 50.36022 | **JAK3** | P52333 | Tyrosine-protein kinase JAK3 | JAK3 | 5 | 0.89 |
| 331 | 47.573241 | **ICAM-1** | P05362 | Intercellular adhesion molecule 1 | ICAM-1 | 6 | 0.92 |
| 330 | 48.842061 | **IL-12-39** | P29459/60 | Interleukin-12 | IL-12 (1) | 7 | 0.91 |
| 329 | 45.823312 | **C-AKT3-1** | Q9Y243 | RAC-gamma serine/threonine-protein kinase | PKB gamma (1) | 8 | 0.92 |
| 328 | 45.489121 | **C5-9** | P01031 | Complement C5 | C5 (2) | 9 | 0.94 |
| 327 | 47.220274 | **C3_016_D12** | P01024 | Complement C3 | C3 (4) | 10 | 0.92 |
| 326 | 39.899609 | **C-P85A-2** | P27986 | Phosphatidylinositol 3-kinase regulatory subunit alpha | P85A (1) | 11 | 0.95 |
| 325 | 36.255768 | **IL-6-58** | P05231 | Interleukin-6 | IL-6 (2) | 12 | 0.96 |
| 324 | 34.457063 | **I-PTPRK-15** | Q15262 | Receptor-type tyrosine-protein phosphatase kappa | R-PTP-kappa (2) | 13 | 0.96 |
| 323 | 28.625926 | **I-MAP2K6-3** | P52564 | Dual specificity mitogen-activated protein kinase kinase 6 | MAPKK 6 (2) | 14 | 0.98 |
| 322 | 28.699206 | **I-TOPB1-1** | Q92547 | DNA topoisomerase 2-binding protein 1 | TopBP1 (1) | 15 | 0.98 |
| 321 | 26.57558 | **I-PGAM5-2** | Q96HS1 | Serine/threonine-protein phosphatase PGAM5, mitochondrial | PGAM5 (2) | 16 | 0.99 |
| 320 | 24.772185 | **I-CBPP22-3** | Q99653 | Calcineurin B homologous protein 1 | CHP1 (2) | 17 | 0.99 |
| 319 | 24.58218 | **P3-16** | P15941 | Mucin-1 | MUC1 (5) | 18 | 0.99 |
| 318 | 22.865319 | **IgM-3** | N/A | N/A | IgM (3) | 19 | 0.99 |

| | | | | | | | |
|---|---|---|---|---|---|---|---|
| Signatures accumulate according to the order shown, e.g., the signature with three analytes comprises properdin, VEGF and C1q. | | | | | | | |

**Table 7 - ROC-AUCs for (example signature 2)**

| ***Priority*** | ***Analyte*** | ***Antibody*** | ***Uniprot entry ID*** | ***Publication name*** | ***Fold change*** | ***Wilcoxon p-value*** | ***Q-value*** | ***Signature length*** | ***ROC-AUC*** |
|---|---|---|---|---|---|---|---|---|---|
| core 1 | R-PTP-O | I-PTPRO-4 | Q16827 | R-PTP-O (2) | 1.070125572 | 0.009704244 | 0.098806849 | 1 | 0.64 |
| core 2 | UBP7 | I-UBP7-10 | Q93009 | UBP7 (1) | 1.11356247 | 0.002987912 | 0.055774355 | 2 | 0.51 |
| 3 | PRD14 | I-PRD14-1 | Q9GZV8 | PRD14 (1) | 1.076408781 | 0.039871393 | 0.155799707 | 3 | 0.41 |
| 4 | GRIP-2 | I-GRIP2-5 | Q9COE4 | GRIP-2 (5) | 1.106919962 | 0.023770038 | 0.137702289 | 4 | 0.59 |
| 5 | CHP-1 | I-CBPP22-3 | Q99653 | CHP1 (2) | 1.161203653 | 0.00065158 | 0.019911196 | 5 | 0.73 |
| 6 | MAPKK 2 | I-MAP2K2-5 | P36507 | MAPKK 2 (2) | 0.904776087 | 0.002285971 | 0.048005396 | 6 | 0.69 |
| 7 | MAPKK 6 | I-MAP2K6-4 | P52564 | MAPKK 6 (3) | 1.157809175 | 0.011922794 | 0.111279406 | 7 | 0.72 |
| 8 | R-PTP-eta | I-PTPRJ-1 | Q12913 | R-PTP-eta (1) | 1.09949869 | 0.017976981 | 0.13018673 | 8 | 0.71 |
| 9 | TopBP1 | I-TOPB1-2 | Q92547 | TopBP1 (2) | 1.071204183 | 0.112701484 | 0.268565239 | 9 | 0.73 |
| 10 | MAPK9 | I-MAPK9-5 | P45984 | MAPK9 (4) | 1.107889922 | 0.007750485 | 0.086805429 | 10 | 0.69 |
| 11 | PKB gamma | C-AKT3-1 | Q9Y243 | PKB gamma (1) | 1.061977425 | 0.048411664 | 0.171224411 | 11 | 0.68 |
| 12 | CIMS (13) | FN17-C08 | N/A | CIMS (13) | 1.10417223 | 0.00563986 | 0.073961513 | 12 | 0.74 |
| 13 | GNAI3 | I-GNAI3-6 | P08754 | GNAl3 (3) | 1.090520613 | 0.011137803 | 0.110067699 | 13 | 0.72 |
| 14 | HsMAD2 | I-MD2L1-7 | Q13257 | HsMAD2 (3) | 1.10784704 | 0.03423597 | 0.152971314 | 14 | 0.72 |
| 15 | hSpindly | I-SPDLY-2 | Q96EA4 | hSpindly (2) | 1.076228973 | 0.006156874 | 0.076618872 | 15 | 0.72 |
| 16 | R-PTP-kappa | I-PTPRK-8 | Q15262 | R-PTP-kappa (7) | 1.074997443 | 0.182672471 | 0.350731144 | 16 | 0.73 |
| 17 | STAT1 | C-STAT1-3 | P42224 | STAT1 (2) | 1.081796782 | 0.015781058 | 0.129327692 | 17 | 0.79 |
| 18 | HsHec1 | I-NDC80-3 | 014777 | HsHec1 (2) | 1.075526742 | 0.040804685 | 0.155799707 | 18 | 0.78 |
| 19 | PAR-6B | I-PARP6B-2 | Q9BYG5 | PAR-6B (1) | 1.099340103 | 0.017976981 | 0.13018673 | 19 | 0.77 |
| 20 | PGAM5 | I-PGAM5-2 | Q96HS1 | PGAM5 (2) | 1.095269092 | 0.03187375 | 0.152971314 | 20 | 0.73 |
| 21 | AGAP-2 | I-CENTG1-1 | Q99490 | AGAP-2 (1) | 1.096662882 | 0.050068011 | 0.175238039 | 21 | 0.71 |
| 22 | TENS4 | C-TENS4-1 | Q8IZW8 | TENS4 | 1.062769906 | 0.051771605 | 0.175709691 | 22 | 0.75 |
| 23 | LUM | LUM_1 | P51884 | LUM | 1.068518726 | 0.072383465 | 0.209662449 | 23 | 0.72 |
| 24 | USP-7 | I-UBP7-10 | Q93009 | UBP7 (1) | 1.11356247 | 0.002987912 | 0.055774355 | 24 | 0.72 |

| | | | | | | | | | |
|---|---|---|---|---|---|---|---|---|---|
| Signatures accumulate according to the order shown, e.g., the signature with three analytes comprises R-PTP-O, UBP7 and PRD14. | | | | | | | | | |

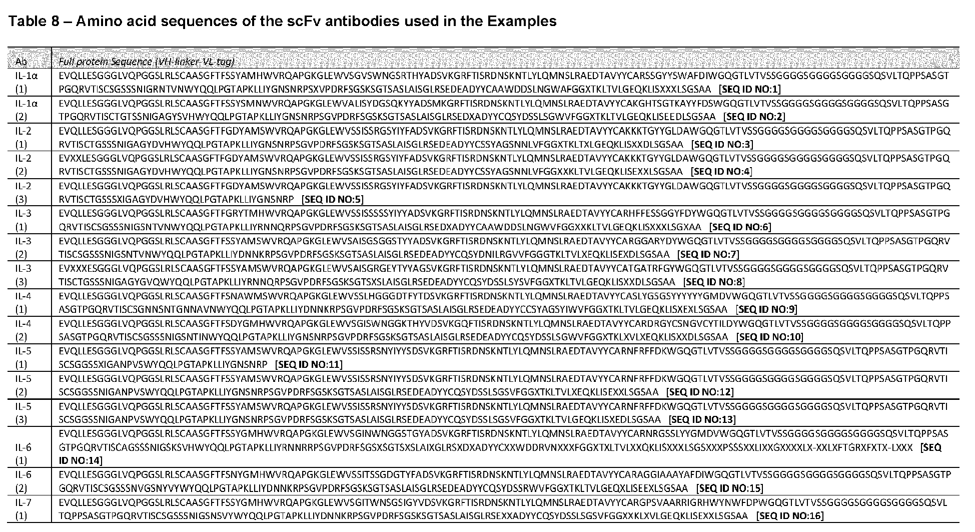

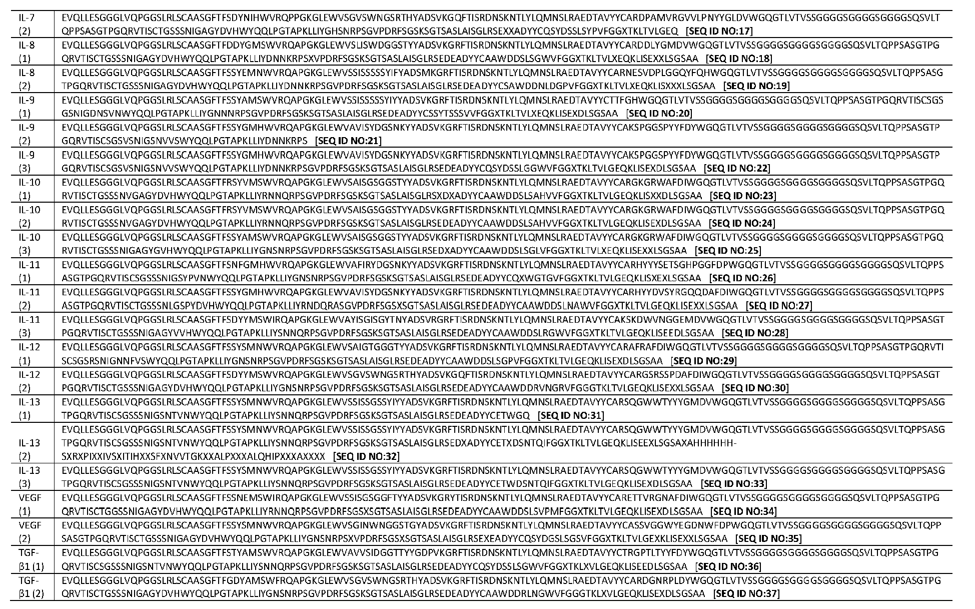

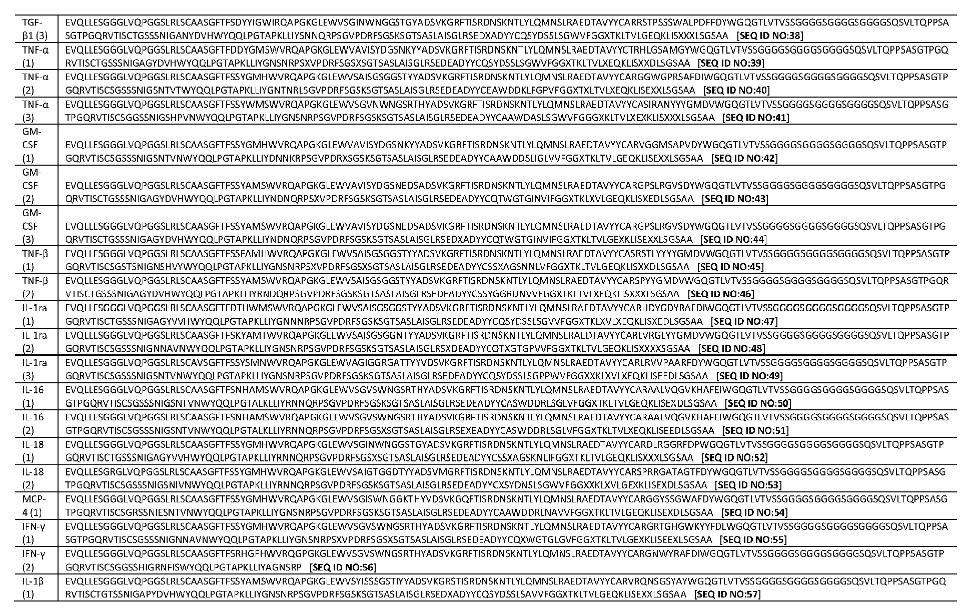

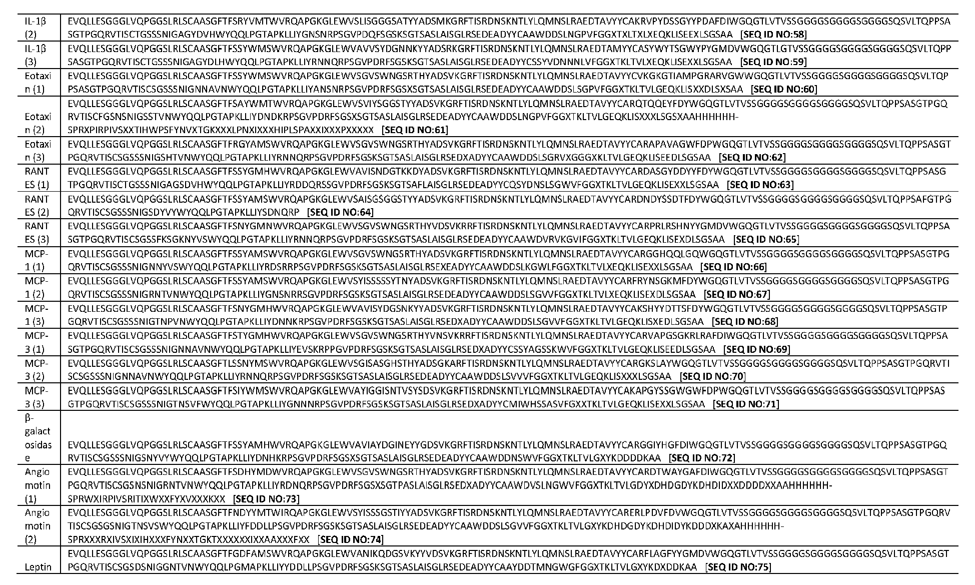

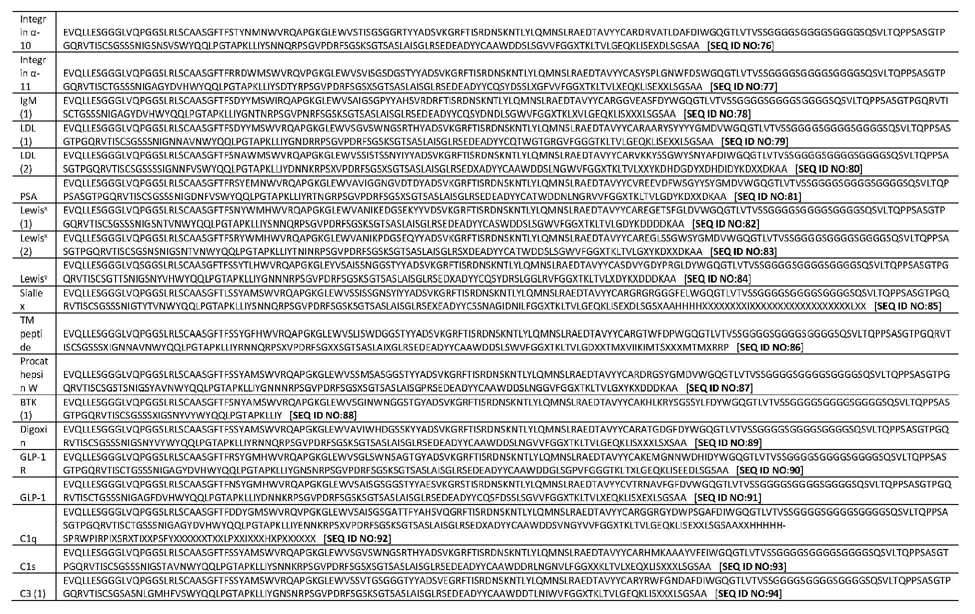

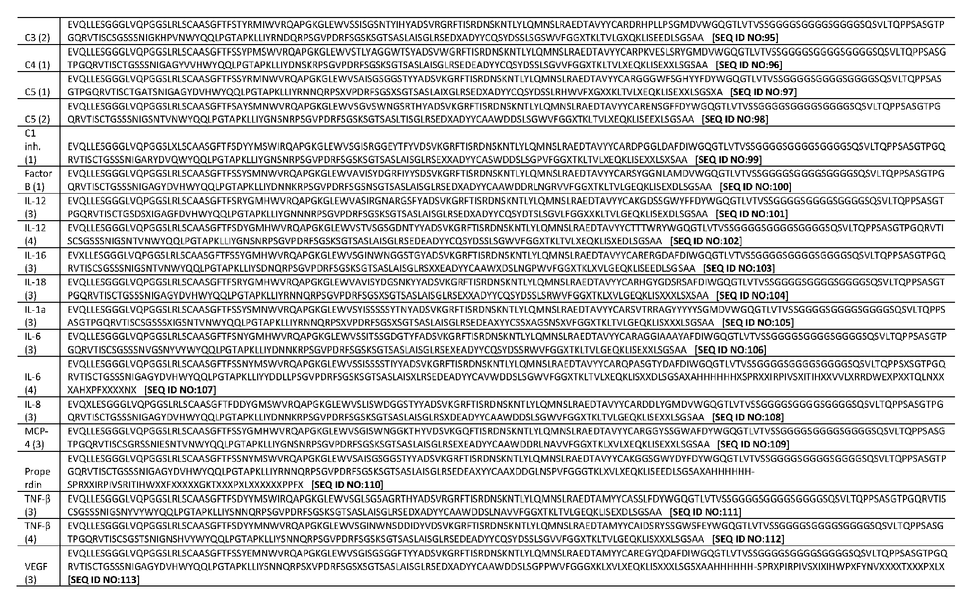

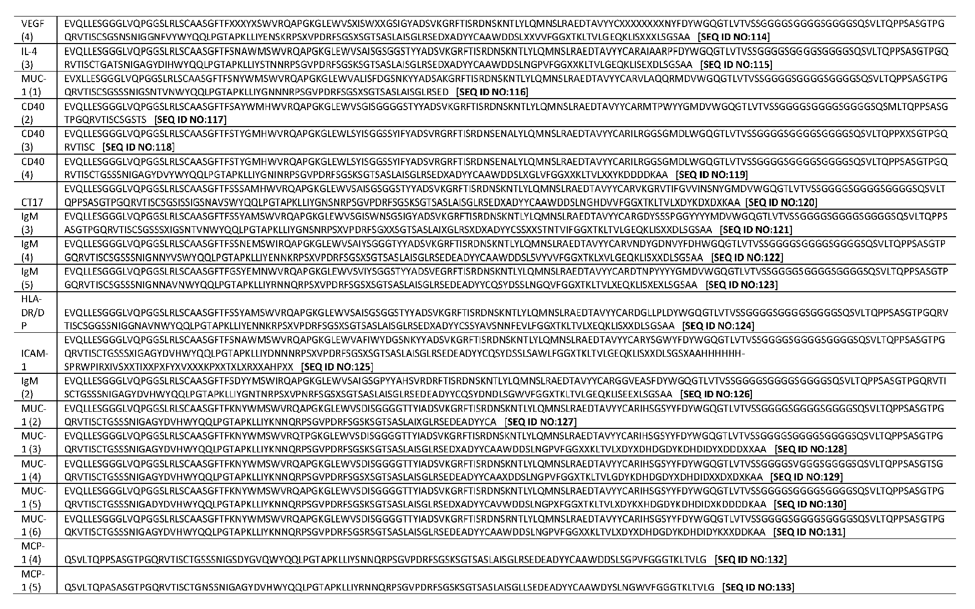

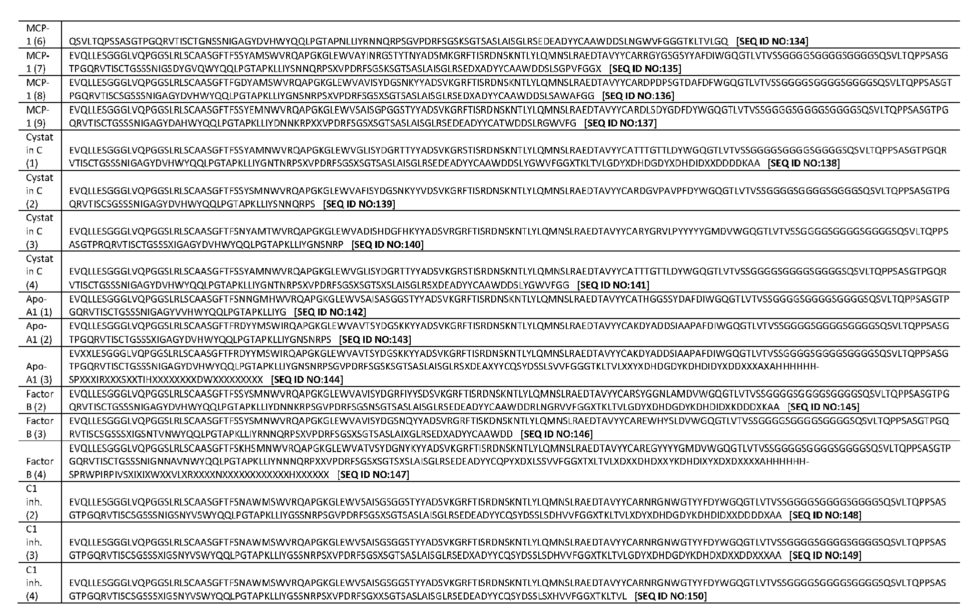

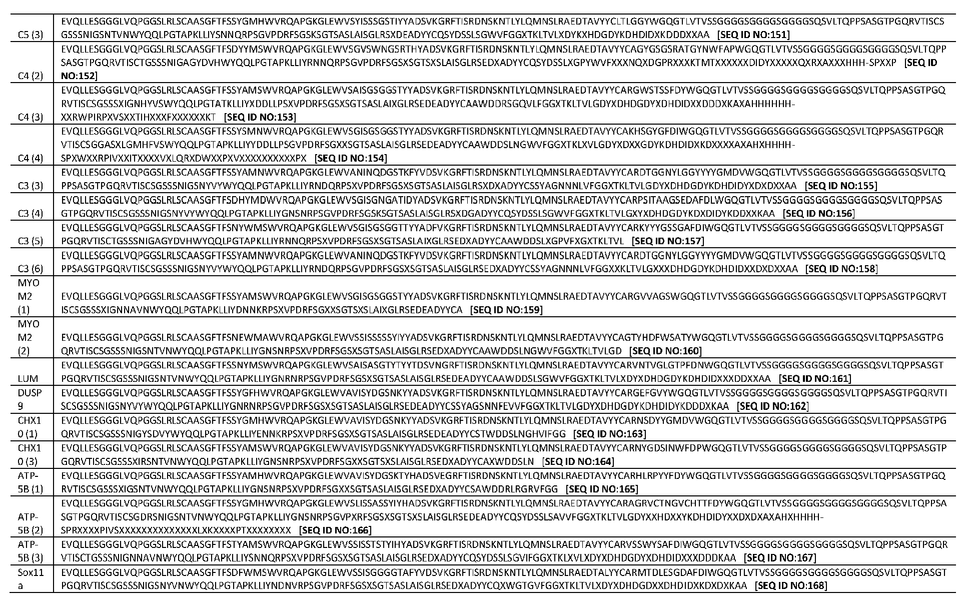

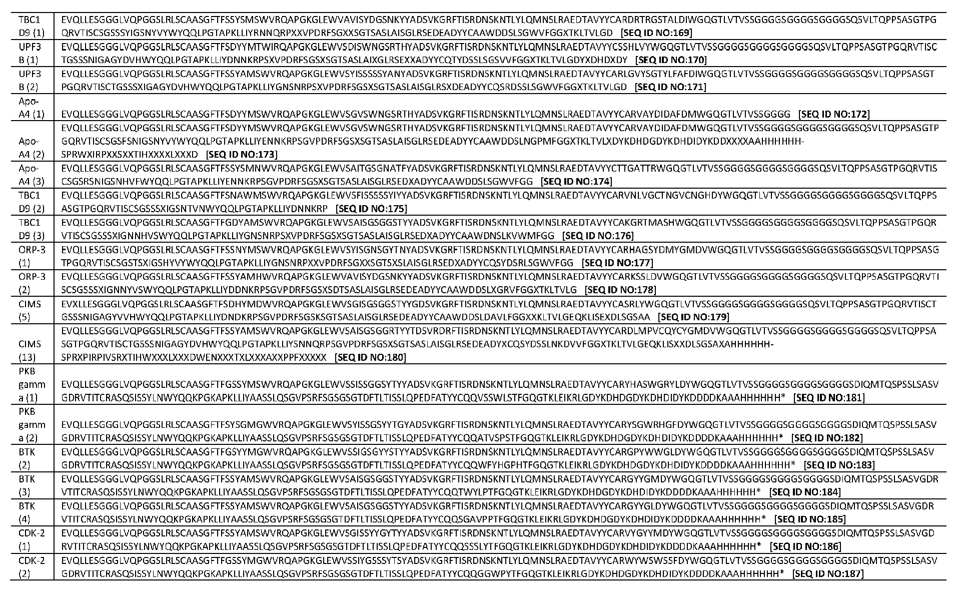

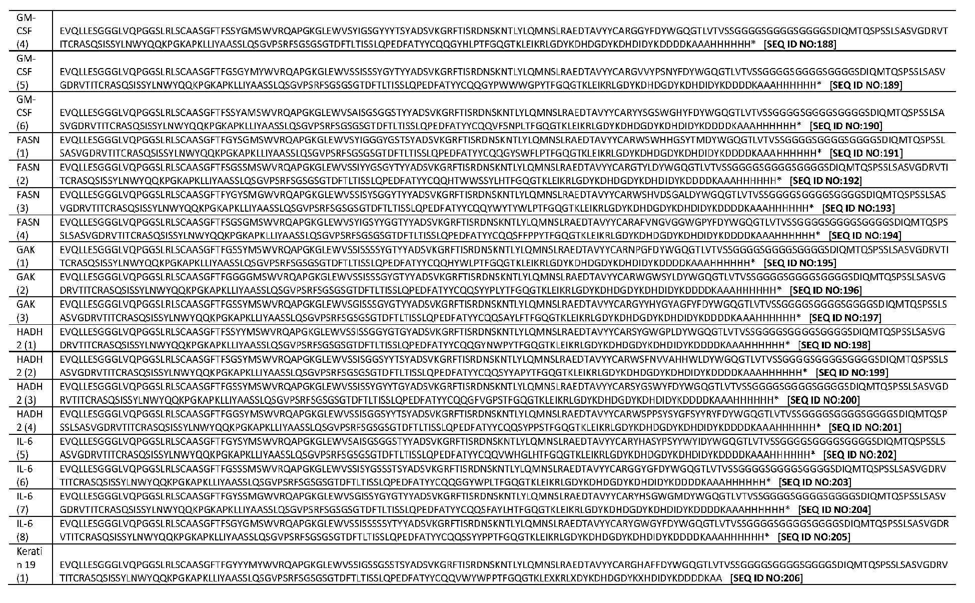

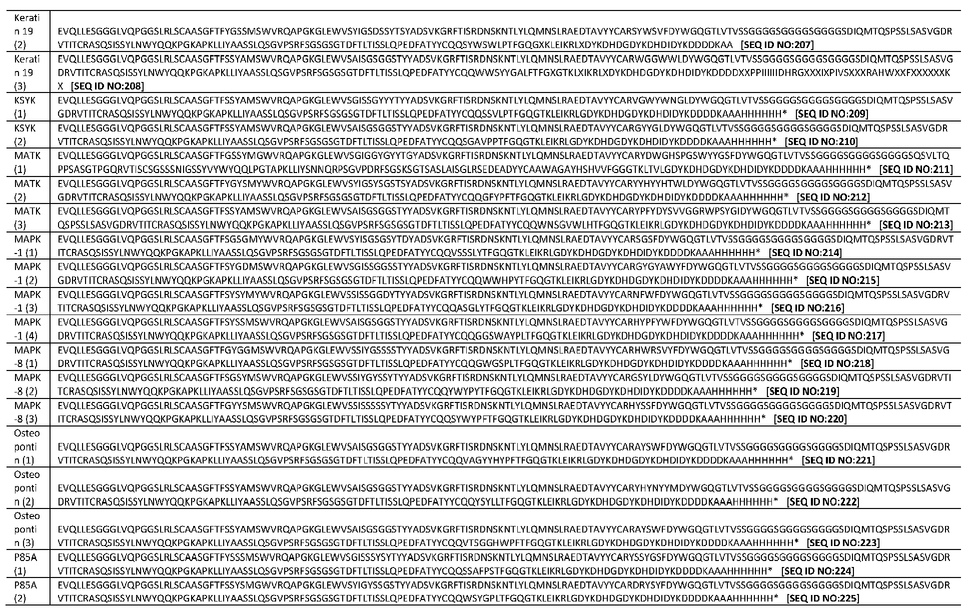

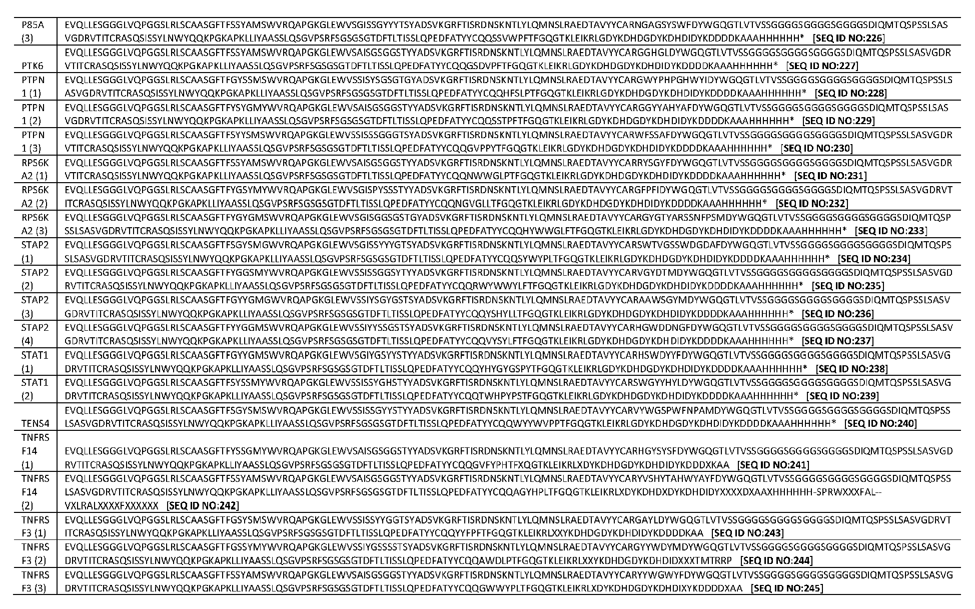

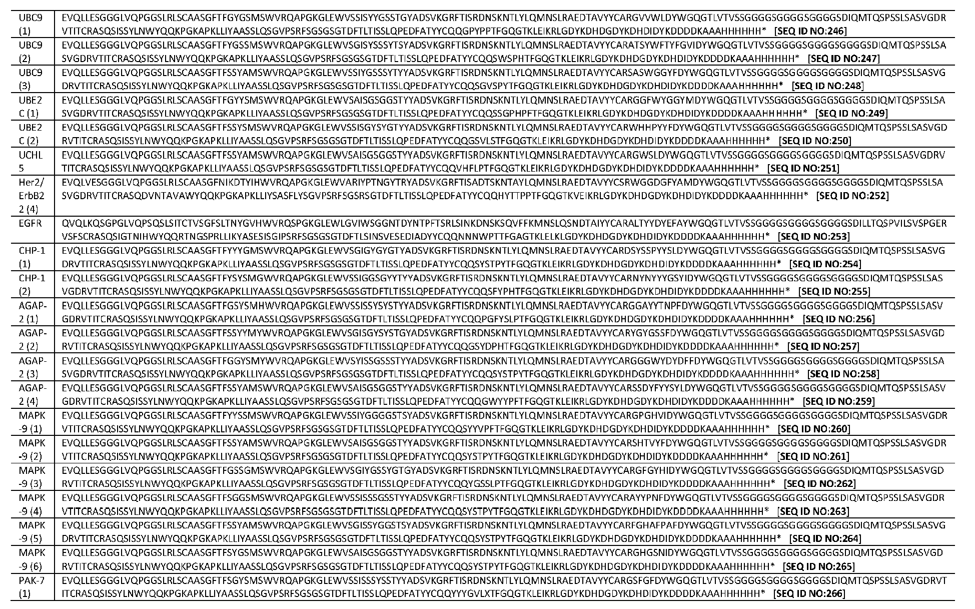

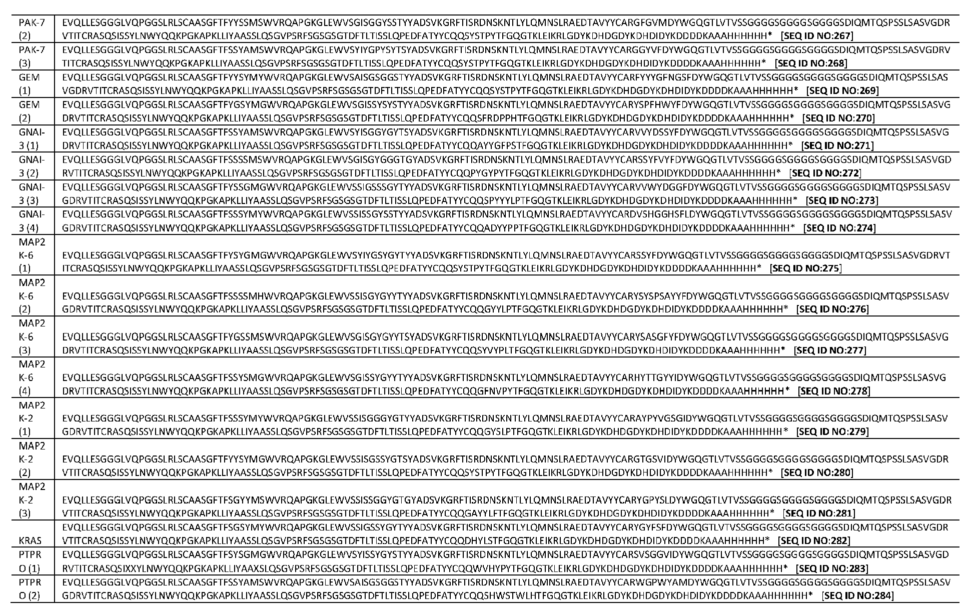

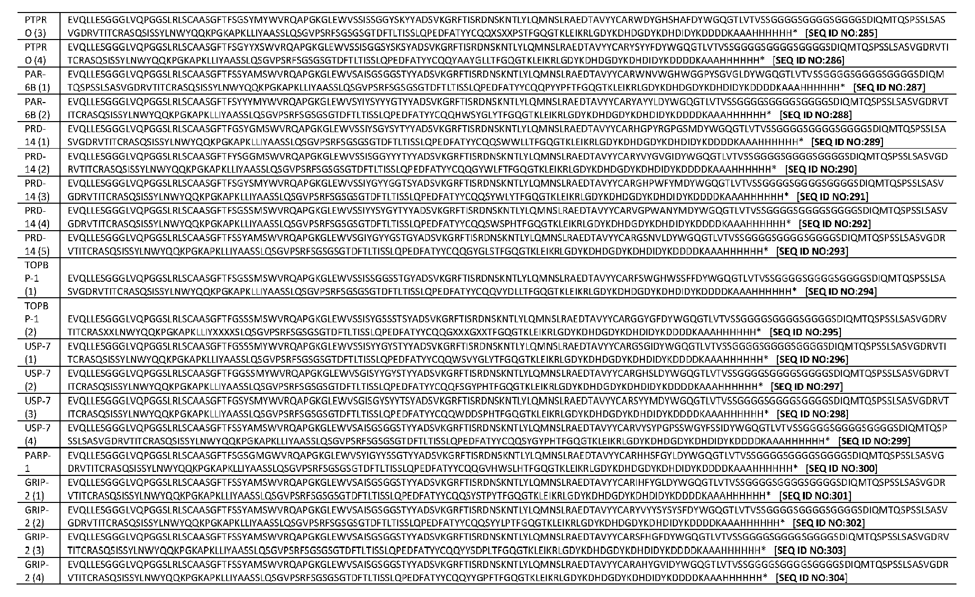

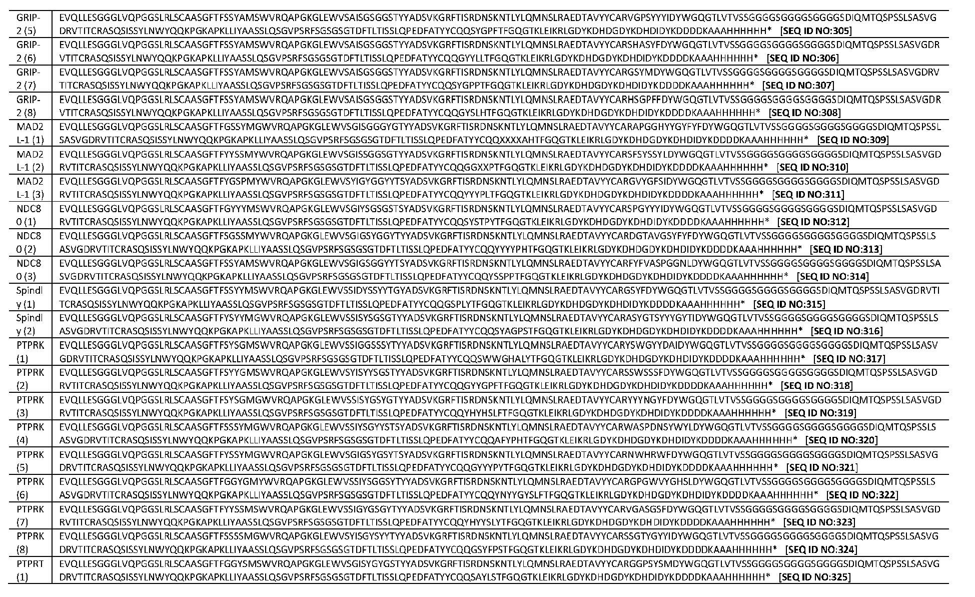

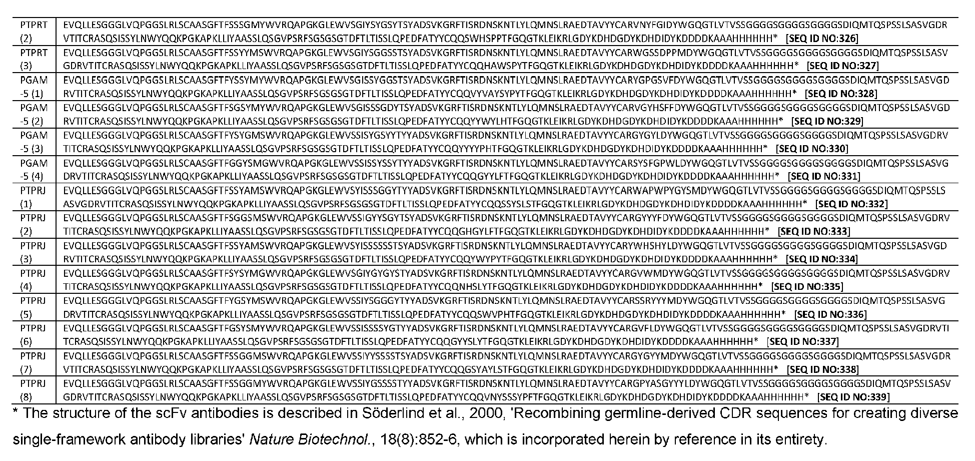

**Embodiments of the invention will now be described with reference to the following numbered paragraphs:**
1. A method for diagnosing or determining a pancreatic cancer-associated disease state comprising or consisting of the steps of:
   (a) providing a sample from an individual to be tested; and
   (b) determining a biomarker signature of the test sample by measuring the presence and/or amount in the test sample of one or more biomarker selected from the group defined in Table A;
   wherein the presence and/or amount in the test sample of the one or more biomarker selected from the group defined in Table A is indicative of the pancreatic cancer-associated disease in the individual.
2. The method according to Paragraph 1 wherein the pancreatic cancer-associated disease state is selected from the group consisting of:
   (i) diagnosis and/or staging of early pancreatic cancer;
   (ii) diagnosis and/or staging of pancreatic cancer; and
3. The method according to any one of Paragraphs 1-2 wherein step (b) comprises or consists of measuring the presence and/or amount of 1 or more biomarker listed in Table A, for example at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84 or 85 of the biomarkers listed in Table A.
4. The method according to any one of the preceding Paragraphs wherein the pancreatic cancer-associated disease state is early pancreatic cancer.
5. The method according to Paragraph 4 wherein the method is for the diagnosis of stage I or stage II pancreatic cancer.
6. The method according to Paragraph 5 wherein step (b) comprises or consists of measuring the presence and/or amount of 1 or more biomarker listed in:
   (i) Table A(I), for example at least 2, 3, 4 or 5 of the biomarkers listed in Table A(I);
   (ii) Table A(III), for example at least 2, 3, 4, 5 or 6 of the biomarkers listed in Table A(III);
   (iii) Table A(V), for example at least 2, 3, 4, 5 or 6 of the biomarkers listed in Table A(V); and
   (iv) Table A(XI), for example at least 2 or 3 of the biomarkers listed in Table A(XI).
7. The method according to Paragraph 5 or 6 wherein step (b) comprises or consists of measuring the presence and/or amount of 1 or more biomarker listed in:
   (i) Table A(II), for example at least 2, 3, 4, 5, 6, 7, 8, 9, 10, 11, 12, 13, 14, 15 or 16 of the biomarkers listed in Table A(II);
   (ii) Table A(IV), for example at least 2, 3, 4, 5, 6, 7, 8, 9, 10, 11 or 12 of the biomarkers listed in Table A(IV);
   (iii) Table A(VI), for example at least 2, 3, 4, 5, 6, 7, 8, 9, 10, 11, 12 or 13 of the biomarkers listed in Table A(VI); and
   (iv) Table A(XII), for example at least 2, 3, 4, 5 or 6 of the biomarkers listed in Table A(XII).
8. The method according to Paragraph 4 wherein the method is for the diagnosis of stage I pancreatic cancer.
9. The method according to Paragraph 8 wherein step (b) comprises or consists of measuring the presence and/or amount of 1 or more biomarker listed in:
   (i) Table A(I), for example at least 2, 3, 4 or 5 of the biomarkers listed in Table A(I);
   (ii) Table A(III), for example at least 2, 3, 4, 5 or 6 of the biomarkers listed in Table A(III);
   (iii) Table A(VII).
10. The method according to Paragraph 8 or 9 wherein step (b) comprises or consists of measuring the presence and/or amount of 1 or more biomarker listed in:
   (i) Table A(II), for example at least 2, 3, 4, 5, 6, 7, 8, 9, 10, 11, 12, 13, 14, 15 or 16 of the biomarkers listed in Table A(II);
   (ii) Table A(IV), for example at least 2, 3, 4, 5, 6, 7, 8, 9, 10, 11 or 12 of the biomarkers listed in Table A(IV); and
   (iii) Table A(VIII), for example at least 2, 3, 4, 5 or 6 of the biomarkers listed in Table A(VIII).
11. The method according to Paragraph 4 wherein the method is for the diagnosis of stage II pancreatic cancer.
12. The method according to Paragraph 11 wherein step (b) comprises or consists of measuring the presence and/or amount of 1 or more biomarker listed in:
   (iv) Table A(I), for example at least 2, 3, 4 or 5 of the biomarkers listed in Table A(I);
   (v) Table A(V), for example at least 2, 3, 4, 5 or 6 of the biomarkers listed in Table A(V);
   (vi) Table A(IX).
13. The method according to Paragraph 11 or 12 wherein step (b) comprises or consists of measuring the presence and/or amount of 1 or more biomarker listed in:
   (iv) Table A(II), for example at least 2, 3, 4, 5, 6, 7, 8, 9, 10, 11, 12, 13, 14, 15 or 16 of the biomarkers listed in Table A(II);
   (v) Table A(VI), for example at least 2, 3, 4, 5, 6, 7, 8, 9, 10, 11, 12 or 13 of the biomarkers listed in Table A(VI); and
   (vi) Table A(X), for example at least 2, 3 or 4 of the biomarkers listed in Table A(X).
14. The method according to any one of the preceding Paragraphs wherein the pancreatic cancer-associated disease state is pancreatic cancer.
15. The method according to Paragraph 14 wherein step (b) comprises or consists of measuring the presence and/or amount of 1 or more biomarker selected from the group consisting of CHP-1, MAPKK 2, UBP7, PRD14, STAT1, AGAP-2, PGAM5, LUM, PTPRO and USP07, for example, at least 2, 3, 4, 5, 6, 7, 8, 9 or 10 of these biomarkers.
16. The method according to Paragraph 14 or 15 wherein step (b) comprises or consists of measuring the presence and/or amount of 1 or more biomarker selected from the group consisting of Apo-A1, BTK, C1q, C5, CDK-2, IgM, IL-11, IL-12, IL-6, JAK3, MAPK8, MCP-1, MUC-1, Properdin, VEGF, C3, ICAM-1, IL-13, ATP-5B, C4, Her2/ErB-2, IL-7, IL-3, IL-8, GM-CSF, IL-9, LDL and ORP3 for example, at least 2, 3, 4, 5, 6, 7, 8, 9, 10, 11, 12, 13, 14, 15, 16, 17, 18, 19, 20, 21, 22, 23, 24, 25, 26, 27, 28 or 29 of these biomarkers.
17. The method according to any one of the preceding Paragraphs wherein step (b) comprises measuring the presence and/or amount of all of the biomarkers listed in Table A.
18. The method according to any one of the preceding Paragraphs further comprising or consisting of the steps of:
   c) providing one or more control sample from:
      i. an individual not afflicted with pancreatic cancer; and/or
      ii. an individual afflicted with pancreatic cancer, wherein the sample was of a different stage to that of that the test sample;
   d) determining a biomarker signature of the one or more control sample by measuring the presence and/or amount in the control sample of the one or more biomarkers measured in step (b);
   wherein the pancreatic cancer-associated disease state is identified in the event that the presence and/or amount in the test sample of the one or more biomarkers measured in step (b) is different from the presence and/or amount in the control sample of the one or more biomarkers measured in step (d).
19. The method according to any one of the preceding Paragraphs further comprising or consisting of the steps of:
   e) providing one or more control sample from;
      i. an individual afflicted with pancreatic cancer (i.e., a positive control); and/or
      ii. an individual afflicted with pancreatic cancer, wherein the sample was of the same stage to that of that the test sample;
   f) determining a biomarker signature of the control sample by measuring the presence and/or amount in the control sample of the one or more biomarkers measured in step (b);
   wherein the pancreatic cancer-associated disease state is identified in the event that the presence and/or amount in the test sample of the one or more biomarkers measured in step (b) corresponds to the presence and/or amount in the control sample of the one or more biomarkers measured in step (f).
20. The method according to any of Paragraphs 18-19, wherein the individual from which the one or more control sample was obtained was not, at the time the sample was obtained, afflicted with a non-cancerous pancreatic disease or condition, for example acute pancreatitis, chronic pancreatitis and autoimmune pancreatitis and Intraductal Papillary Mucinous Neoplasia (IPMN) of the Pancreas.
21. The method according to any of Paragraphs 18-20, wherein the individual from which the one or more control sample was obtained was not, at the time the sample was obtained, afflicted with any disease or condition of the pancreas.
22. The method according to Paragraph 18, wherein the individual not afflicted with pancreatic cancer was not, at the time the sample was obtained, afflicted with any disease or condition.
23. The method according to Paragraph 18 wherein the individual not afflicted with pancreatic cancer is a healthy individual.
24. The method according to any one of Paragraphs 18-23 wherein the one or more individual afflicted with pancreatic cancer is afflicted with a pancreatic cancer selected from the group consisting of adenocarcinoma (e.g., pancreatic ductal adenocarcinoma or tubular papillary pancreatic adenocarcinoma), pancreatic sarcoma, malignant serous cystadenoma, adenosquamous carcinoma, signet ring cell carcinoma, hepatoid carcinoma, colloid carcinoma, undifferentiated carcinoma, and undifferentiated carcinomas with osteoclast-like giant cells.
25. The method according to any one of the preceding Paragraphs wherein the pancreatic cancer is pancreatic adenocarcinoma (e.g., pancreatic ductal adenocarcinoma).
26. The method according to any one of the preceding Paragraphs wherein the method is repeated.
27. The method according to any one of the preceding Paragraphs wherein the method is repeated and wherein, in step (a), the sample to be tested is taken at different time to the previous method repetition.
28. The method according to Paragraph 26 or 27 wherein the method is repeated using a test sample taken at a different time period to the previous test sample(s) used.
29. The method according to Paragraph 27 or 28 wherein the method is repeated using a test sample taken between 1 day to 104 weeks to the previous test sample(s) used, for example, between 1 week to 100 weeks, 1 week to 90 weeks, 1 week to 80 weeks, 1 week to 70 weeks, 1 week to 60 weeks, 1 week to 50 weeks, 1 week to 40 weeks, 1 week to 30 weeks, 1 week to 20 weeks, 1 week to 10 weeks, 1 week to 9 weeks, 1 week to 8 weeks, 1 week to 7 weeks, 1 week to 6 weeks, 1 week to 5 weeks, 1 week to 4 weeks, 1 week to 3 weeks, or 1 week to 2 weeks.
30. The method according to Paragraph 27 or 28 wherein the method is repeated using a test sample taken every period from the group consisting of: 1 day, 2 days, 3 day, 4 days, 5 days, 6 days, 7 days, 10 days, 2 weeks, 3 weeks, 4 weeks, 5 weeks, 6 weeks, 7 weeks, 8 weeks, 9 weeks, 10 weeks, 15 weeks, 20 weeks, 25 weeks, 30 weeks, 35 weeks, 40 weeks, 45 weeks, 50 weeks, 55 weeks, 60 weeks, 65 weeks, 70 weeks, 75 weeks, 80 weeks, 85 weeks, 90 weeks, 95 weeks, 100 weeks, 104, weeks, 105 weeks, 110 weeks, 115 weeks, 120 weeks, 125 weeks and 130 weeks.
31. The method according to any one of Paragraphs 26-30 wherein the method is repeated at least once, for example, 2 times, 3 times, 4 times, 5 times, 6 times, 7 times, 8 times, 9 times, 10 times, 11 times, 12 times, 13 times, 14 times, 15 times, 16 times, 17 times, 18 times, 19 times, 20 times, 21 times, 22 times, 23, 24 times or 25 times.
32. The method according to any one of Paragraphs 26-30 wherein the method is repeated continuously.
33. The method according to any one of Paragraphs 26-30 wherein the method is repeated until pancreatic cancer is diagnosed in the individual using conventional clinical methods.
34. The method according to any one of Paragraphs 26-30 wherein each repetition uses test sample taken from the same individual.
35. The method according to any one of Paragraphs 1 to 34 wherein step (b) comprises measuring the expression of the protein or polypeptide of the one or more biomarker(s)
36. The method according to any one of the preceding Paragraphs wherein step (b), (d) and/or step (f) is performed using one or more first binding agent capable of binding to a biomarker listed in Table A.
37. The method according to Paragraph 36 wherein the first binding agent comprises or consists of an antibody or an antigen-binding fragment thereof.
38. The method according to Paragraph 37 wherein the antibody or antigen-binding fragment thereof is a recombinant antibody or antigen-binding fragment thereof.
39. The method according to Paragraph 37 or 38 wherein the antibody or antigen-binding fragment thereof is selected from the group consisting of: scFv; Fab; a binding domain of an immunoglobulin molecule.
40. The method according to any one of Paragraphs 36 to 39 wherein the first binding agent is immobilised on a surface.
41. The method according to any one of Paragraphs 36 to 40 wherein the one or more biomarkers in the test sample are labelled with a detectable moiety.
42. The method according to any one of Paragraphs 36 to 41 wherein the one or more biomarkers in the control sample(s) are labelled with a detectable moiety.
43. The method according to Paragraph 41 or 42 wherein the detectable moiety is selected from the group consisting of: a fluorescent moiety; a luminescent moiety; a chemiluminescent moiety; a radioactive moiety; an enzymatic moiety.
44. The method according to Paragraph 41 or 42 wherein the detectable moiety is biotin.
45. The method according to any one of Paragraphs 36 to 44 wherein step (b), (d) and/or step (f) is performed using an assay comprising a second binding agent capable of binding to the one or more biomarkers, the second binding agent comprising a detectable moiety.
46. The method according to any one of Paragraph 45 wherein the second binding agent comprises or consists of an antibody or an antigen-binding fragment thereof.
47. The method according to Paragraph 46 wherein the antibody or antigen-binding fragment thereof is a recombinant antibody or antigen-binding fragment thereof.
48. The method according to Paragraph 46 or 47 wherein the antibody or antigen-binding fragment thereof is selected from the group consisting of: scFv; Fab; a binding domain of an immunoglobulin molecule.
49. The method according to any one of Paragraphs 46 to 48 wherein the detectable moiety is selected from the group consisting of: a fluorescent moiety; a luminescent moiety; a chemiluminescent moiety; a radioactive moiety; an enzymatic moiety.
50. The method according to Paragraph 49 wherein the detectable moiety is fluorescent moiety (for example an Alexa Fluor dye, e.g. Alexa647).
51. The method according to any one of the preceding Paragraphs wherein the method comprises or consists of an ELISA (Enzyme Linked Immunosorbent Assay).
52. The method according to any one of the preceding Paragraphs wherein step (b), (d) and/or step (f) is performed using an array.
53. The method according to Paragraph 52 wherein the array is a bead-based array.
54. The method according to Paragraph 53 wherein the array is a surface-based array.
55. The method according to any one of Paragraphs 52 to 54 wherein the array is selected from the group consisting of: macroarray; microarray; nanoarray.
56. The method according to any one of the preceding Paragraphs wherein the method comprises:
   (i) labelling biomarkers present in the sample with biotin;
   (ii) contacting the biotin-labelled proteins with an array comprising a plurality of scFv immobilised at discrete locations on its surface, the scFv having specificity for one or more of the proteins in Table A;
   (iii) contacting the immobilised scFv with a streptavidin conjugate comprising a fluorescent dye; and
   (iv) detecting the presence of the dye at discrete locations on the array surface
   wherein the expression of the dye on the array surface is indicative of the expression of a biomarker from Table III in the sample.
57. The method according to any one of Paragraphs 1 to 34 wherein, step (b), (d) and/or (f) comprises measuring the expression of a nucleic acid molecule encoding the one or more biomarkers.
58. The method according to Paragraph 57, wherein the nucleic acid molecule is a cDNA molecule or an mRNA molecule.
59. The method according to Paragraph 58, wherein the nucleic acid molecule is an mRNA molecule.
60. The method according to Paragraph 57, 58 or 59, wherein measuring the expression of the one or more biomarker(s) in step (b), (d) and/or (f) is performed using a method selected from the group consisting of Southern hybridisation, Northern hybridisation, polymerase chain reaction (PCR), reverse transcriptase PCR (RT-PCR), quantitative real-time PCR (qRT-PCR), nanoarray, microarray, macroarray, autoradiography and *in situ* hybridisation.
61. The method according to any one of Paragraphs 57-60, wherein measuring the expression of the one or more biomarker(s) in step (b) is determined using a DNA microarray.
62. The method according to any one of Paragraphs 57-61, wherein measuring the expression of the one or more biomarker(s) in step (b), (d) and/or (f) is performed using one or more binding moieties, each individually capable of binding selectively to a nucleic acid molecule encoding one of the biomarkers identified in Table A.
63. The method according to Paragraph 62, wherein the one or more binding moieties each comprise or consist of a nucleic acid molecule.
64. The method according to Paragraph 63 wherein, the one or more binding moieties each comprise or consist of DNA, RNA, PNA, LNA, GNA, TNA or PMO.
65. The method according to Paragraph 63 or 64, wherein the one or more binding moieties each comprise or consist of DNA.
66. The method according to any one of Paragraphs 63-65 wherein the one or more binding moieties are 5 to 100 nucleotides in length.
67. The method according to any one of Paragraphs 63-65 wherein the one or more nucleic acid molecules are 15 to 35 nucleotides in length.
68. The method according to any one of Paragraphs 63-67 wherein the binding moiety comprises a detectable moiety.
69. The method according to Paragraph 68 wherein the detectable moiety is selected from the group consisting of: a fluorescent moiety; a luminescent moiety; a chemiluminescent moiety; a radioactive moiety (for example, a radioactive atom); or an enzymatic moiety.
70. The method according to Paragraph 69 wherein the detectable moiety comprises or consists of a radioactive atom.
71. The method according to Paragraph 70 wherein the radioactive atom is selected from the group consisting of technetium-99m, iodine-123, iodine-125, iodine-131, indium-111, fluorine-19, carbon-13, nitrogen-15, oxygen-17, phosphorus-32, sulphur-35, deuterium, tritium, rhenium-186, rhenium-188 and yttrium-90.
72. The method according to Paragraph 69 wherein the detectable moiety of the binding moiety is a fluorescent moiety.
73. The method according to any one of the preceding Paragraphs wherein, the sample provided in step (a), (c) and/or (e) is selected from the group consisting of unfractionated blood, plasma, serum, tissue fluid, pancreatic tissue, milk, bile and urine.
74. The method according to Paragraph 73, wherein the sample provided in step (a), (c) and/or (e) is selected from the group consisting of unfractionated blood, plasma and serum.
75. The method according to Paragraph 73 or 74, wherein the sample provided in step (a), (c) and/or (e) is serum.
76. The method according to any one of the preceding Paragraphs wherein the predicative accuracy of the method, as determined by an ROC AUC value, is at least 0.50, for example at least 0.55, 0.60, 0.65, 0.70, 0.75, 0.80, 0.85, 0.90, 0.95, 0.96, 0.97, 0.98 or at least 0.99.
77. The method according to Paragraph 76 wherein the predicative accuracy of the method, as determined by an ROC AUC value, is at least 0.70.
78. The method according to any one of the preceding Paragraphs wherein, in the event that the individual is diagnosed with pancreatic cancer, the method comprises the step of:
   (g) providing the individual with pancreatic cancer therapy.
79. The method according to Paragraph 78 wherein the pancreatic cancer therapy is selected from the group consisting of surgery, chemotherapy, immunotherapy, chemoimmunotherapy and thermochemotherapy.
80. An array for determining the presence of pancreatic cancer in an individual comprising one or more binding agent as defined in any one of Paragraphs 36-51 and 64-72.
81. The array according to Paragraph 80 wherein the one or more binding agents is capable of binding to all of the proteins defined in Table A.
82. Use of one or more biomarkers selected from the group defined in Table A as a biomarker for determining the presence of pancreatic cancer in an individual.
83. The use according to Paragraph 82 wherein all of the proteins defined in Table A is used as a diagnostic marker for determining the presence of pancreatic cancer in an individual.
84. The use of one or more binding moiety as defined in any one of Paragraphs 36-51 and 64-72 for determining the presence of pancreatic cancer in an individual.
85. The use according to Paragraph 84 wherein biomarkers for all of the proteins defined in Table A are used.
86. A kit for determining the presence of pancreatic cancer comprising:
   A) one or more binding agent as defined in any one of Paragraphs 36-51 and 64-72 or an array according to Paragraphs 80-81;
   B) instructions for performing the method as defined in any one of Paragraphs 1-79.
87. A method of treating pancreatic cancer in an individual comprising the steps of:
   (a) diagnosing pancreatic cancer according to the method defined in any one of Paragraphs 1-79; and
   (b) providing the individual with pancreatic cancer therapy.
88. The method according to Paragraph 87 wherein the pancreatic cancer therapy is selected from the group consisting of surgery (e.g., resection), chemotherapy, immunotherapy, chemoimmunotherapy and thermochemotherapy.
89. A method or use for determining the presence of pancreatic cancer in an individual substantially as described herein.
90. An array or kit for determining the presence of pancreatic cancer in an individual substantially as described herein.

It should be understood that various changes and modifications to the presently preferred embodiments described herein will be apparent to those skilled in the art. Such changes and modifications can be made without departing from the spirit and scope of the present invention and without diminishing its intended advantages.

## Claims

1. A method for diagnosis and/or staging of stage I or stage II pancreatic cancer comprising or consisting of the steps of:
(a) providing a blood, serum or plasma sample obtained from an individual to be tested; and
(b) determining the amount in the test sample of two or more biomarkers selected from the group defined in Table A, wherein the two or more biomarkers include two or more of Factor B, Complement 5 (C5), and Complement 4 (C4);
wherein the amount in the test sample of the two or more biomarkers selected from the group defined in Table A is indicative of the diagnosis and/or stage of stage I or stage II pancreatic cancer in the individual.

2. The method according to Claim 2 wherein step (b) comprises or consists of measuring the amount of Factor B, Complement 5 (C5), and Complement 4 (C4).

3. The method according to Claim 1 or 2 wherein step (b) comprises or consists of measuring the amount of 3 or more biomarkers listed in Table A, for example at least 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84 or 85 of the biomarkers listed in Table A.

4. The method according to any one of the preceding claims wherein step (b) comprises measuring the amount of 1 or more biomarker selected from the group consisting of Calcineurin B homologous protein 1 (CHP-1), Dual specificity mitogen-activated protein kinase kinase 2 (MAPKK 2), PR domain zinc finger protein 14 (PRD14), Signal transducer and activator of transcription 1-alpha/beta (STAT1), Arf-GAP with GTPase, ANK repeat and PH domain-containing protein 2 (AGAP-2), Serine/threonine-protein phosphatase PGAM5, mitochondrial (PGAM5), Lumican (LUM), and Receptor-type tyrosine-protein phosphatase O (PTPRO) and USP07, for example, at least 2, 3, 4, 5, 6, 7, 8, or 9 of these biomarkers.

5. The method according to any one of the preceding claims wherein step (b) comprises measuring the amount of 1 or more biomarker selected from the group consisting of Apolipoprotein A-I (Apo-A1), Tyrosine-protein kinase BTK (BTK), Complement C1q (C1q), Cyclin-dependent kinase 2 (CDK-2), Immunoglobulin heavy constant mu (IgM), Interleukin-11 (IL-11), Interleukin-12 (IL-12), Interleukin-6 (IL-6), Tyrosine-protein kinase JAK3 (JAK3), Mitogen-activated protein kinase 8 (MAPK8), C-C motif chemokine 2 (MCP-1), Mucin-1 (MUC-1), Properdin, Vascular endothelial growth factor A (VEGF), Complement C3 (C3), Intercellular adhesion molecule 1 (ICAM-1), Interleukin-13 (IL-13), ATP synthase subunit beta, mitochondrial (ATP-5B), Receptor tyrosine-protein kinase erbB-2 (Her2/ErB-2), Interleukin-7 (IL-7), Interleukin-3 (IL-3), Interleukin-8 (IL-8), Granulocyte-macrophage colony-stimulating factor (GM-CSF), Interleukin-9 (IL-9), Apolipoprotein B-100 (LDL) and Oxysterol-binding protein-related protein 3 (ORP3) for example, at least 2, 3, 4, 5, 6, 7, 8, 9, 10, 11, 12, 13, 14, 15, 16, 17, 18, 19, 20, 21, 22, 23, 24, 25, or 26 of these biomarkers.

6. The method according to any one of the preceding claims wherein step (b) comprises measuring the amount of all of the biomarkers listed in Table A.

7. The method according to any one of the preceding claims further comprising or consisting of the steps of:
(c) providing one or more control sample obtained from:
i. an individual not afflicted with pancreatic cancer; and/or
ii. an individual afflicted with pancreatic cancer, wherein the sample was of a different stage to that of that the test sample;
(d) determining a biomarker signature of the one or more control sample by measuring the amount in the control sample of the two or more biomarkers measured in step (b);
wherein the diagnosis and/or stage of pancreatic cancer is identified in the event that the amount in the test sample of the two or more biomarkers measured in step (b) is different from the amount in the control sample of the two or more biomarkers measured in step (d).

8. The method according to any one of the preceding claims further comprising or consisting of the steps of:
(e) providing one or more control sample obtained from:
i. an individual afflicted with pancreatic cancer (i.e., a positive control); and/or
ii. an individual afflicted with pancreatic cancer, wherein the sample was of the same stage to that of that the test sample;
(f) determining a biomarker signature of the control sample by measuring the amount in the control sample of the two or more biomarkers measured in step (b);
wherein the diagnosis and/or stage of pancreatic cancer is identified in the event that the amount in the test sample of the two or more biomarkers measured in step (b) corresponds to the amount in the control sample of the two or more biomarkers measured in step (f).

9. The method according to any one of the preceding claims wherein the pancreatic cancer is pancreatic adenocarcinoma (e.g., pancreatic ductal adenocarcinoma).

10. The method according to any one of Claims 1 to 9 wherein step (b) comprises measuring the expression of the protein or polypeptide of the two or more biomarkers.

11. The method according to any one of the preceding claims wherein step (b), (d) and/or step (f) is performed using two or more first binding agents capable of binding to a biomarker listed in Table A.

12. The method according to Claim 11 wherein the first binding agent comprises or consists of an antibody or an antigen-binding fragment thereof; optionally wherein the antibody or antigen-binding fragment thereof is a recombinant antibody or antigen-binding fragment thereof; optionally wherein the antibody or antigen-binding fragment thereof is selected from the group consisting of: scFv; Fab; a binding domain of an immunoglobulin molecule.

13. The method according to Claim 12, wherein the first binding agents comprise at least two of: (i) an antibody or an antigen-binding fragment thereof comprising the sequence of SEQ ID NOs: 100, or 145, or 146, or 147; and (ii) an antibody or an antigen-binding fragment thereof comprising the sequence of SEQ ID NOs: 97, or 98, or 151; and (iii) an antibody or an antigen-binding fragment thereof comprising the sequence of SEQ ID NOs: 96, or 152, or 153, or 154.

14. The method according to any one of the preceding claims wherein step (b), (d) and/or step (f) is performed using an array.

15. An array for determining the presence of pancreatic cancer in an individual comprising two or more binding agents as defined in any one of Claims 11-13; optionally wherein the two or more binding agents are capable of binding to all of the proteins defined in Table A.
